# EUROPEAN PATENT SPECIFICATION

(11) **EP 1 480 972 B1**
(45) Date of publication and mention of the grant of the patent: **11.04.2007**
(21) Application number: 03727261.4
(22) Date of filing: 17.02.2003
(51) Int. Cl.: C07D 401/12

(54) **COMPOUNDS POSSESSING AFFINITY AT 5HT1-TYPE RECEPTORS AND USE THEREOF IN THERAPY OF CNS DISORDERS**
VERBINDUNGEN MIT AFFINITÄT ZU REZEPTOREN DES 5HT-1-TYPS UND DEREN VERWENDUNG IN DER THERAPIE VON ERKRANKUNGEN DES ZNS
COMPOSES PRESENTANT UNE AFFINITE VIS-A-VIS DES RECEPTEURS DU TYPE 5HT1, ET LEUR UTILISATION DANS DES TRAITEMENTS DES TROUBLES DU SNC

(30) Priority: 18.02.2002 GB 0203813
(43) Date of publication of application: 01.12.2004
(73) Proprietor: GLAXO GROUP LIMITED, Greenford, Middlesex UB6 0NN (GB)
(72) Inventor: SMITH, Paul, W., GlaxoSmithKline, Harlow, Essex CM19 5AW (GB); THEWLIS, Kevin, Michael, GlaxoSmithKline, Harlow, Essex CM19 5AW (GB); VONG, Antonio, Kuok, Keong, GlaxoSmithKline, Harlow, Essex CM19 5AW (GB); WARD, Simon, E., GlaxoSmithKline, Harlow, Essex CM19 5AW (GB)
(74) Representative: Giddings, Peter John
(86) International application number: PCT/EP2003/001709
(87) International publication number: WO 2003/068760

(56) References cited:
- WO-A-95/32196
- WO-A-97/28139

## Description

The present invention relates to novel compounds, processes for their preparation, pharmaceutical compositions containing the same and their use as medicaments in the treatment of CNS disorders and other disorders. Related compounds with such activities can be found in WO 95/32 196.

A novel series of compounds has now been found that possess high affinity for 5-HT₁ type receptors. The present invention therefore provides, in a first aspect, a compound of formula (I) or a pharmaceutically acceptable salt thereof: 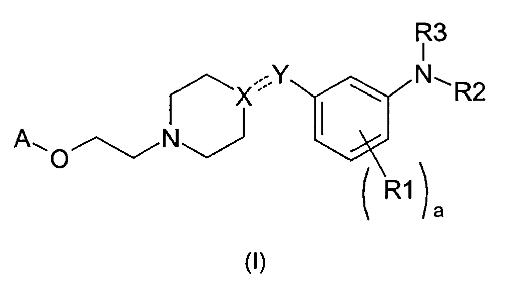 wherein:
A is optionally substituted phenyl, naphthyl, indolyl, quinolinyl, quinazolinyl, indazolyl, isoquinolinyl or benzofuranyl;
X is carbon, Y is CH and 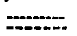 is a double bond; or X is CH, Y is CH₂ or oxygen and 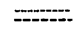 is a single bond; or X is nitrogen, Y is CH₂ and 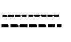 is a single bond;
R1 is halogen, hydroxy, cyano, C₁₋₆alkyl, haloC₁₋₆alkyl or C₁₋₆alkoxy;
a is 0, 1, 2, 3 or 4;
R2 and R3, together with the nitrogen atom to which they are attached, form a nitro group or an optionally substituted 3 to 7 membered heterocyclic group, or R2 and R3 are independently hydrogen, aroyl, C₁₋₆alkyl, C₁₋₆alkanoyl, fluoroC₁₋₆alkanoyl, C₁₋₆alkylsulfonyl, fluoroC₁₋₆alkylsulfonyl, carbamoyl, C₁₋₆alkylcarbamoyl, arylC₁₋₆alkyl or a group CO(CH₂)bNR4R5 wherein b is 1, 2, 3 or 4 and R4 and R5 are independently hydrogen or C₁₋₆alkyl, or R4 and R5, together with the nitrogen atom to they are attached, form part of an optionally substituted 3 to 7 membered heterocyclic group.

The term "halogen" and its abbreviation "halo" refer to fluorine, chlorine, bromine or iodine.

The term "C₁₋₆alkyl" refers to an alkyl group having from one to six carbon atoms, in all isomeric forms, such as methyl, ethyl, propyl, isopropyl, butyl, isobutyl, sec-butyl, tert-butyl, pentyl, neopentyl, sec-pentyl, n-pentyl, isopentyl, tert-pentyl and hexyl.

The term "haloC₁₋₆alkyl" refers to an alkyl group having one or more substitutions by halogen atoms, such as for example CF₃.

The term "C₁₋₆alkoxy" refers to a straight chain or branched chain alkoxy (or "alkyloxy") group having from one to six carbon atoms, such as methoxy, ethoxy, propoxy, isopropoxy, butoxy, isobutoxy, sec-butoxy, tert-butoxy, pentoxy, neopentoxy, sec-pentoxy, n-pentoxy, isopentoxy, tert-pentoxy and hexoxy.

The term "C₁₋₆alkanoyl" refers to an alkanoyl group having from 1 to 6 carbon atoms, such as methanoyl (or "formyl"), ethanoyl (or "acetyl"), propanoyl, isopropanoyl, butanoyl, isobutanoyl, sec-butanoyl, pentanoyl, neopentanoyl, sec-pentanoyl, isopentanoyl, tertpentanoyl and hexanoyl.

The term "fluoroC₁₋₆alkanoyl" refers to a fluorine-substituted C₁₋₆alkanoyl group such as CF₃CO. The term "fluoroC₁₋₆alkylsulfonyl" refers to a fluorine-substituted C₁₋₆alkylsulfonyl group such as CF₃SO₂.

The term "carbamoyl" refers to the group H₂NCO. The term "C₁₋₆alkylcarbamoyl" refers to a group having the formula (C₁₋₆alkyl)HNCO, such as CH₃NHCO.

The term "aryl", whether alone or as part of another group, is intended, unless otherwise stated, to denote an aromatic carbocyclic or heterocyclic group such as phenyl, naphthyl, thienyl, furyl, pyridyl, pyrimidinyl, isoxazolyl or pyrazinyl, optionally substituted by one or more, preferably 1 to 3, halogen, C₁₋₆alkyl, CF₃, cyano, hydroxy, C₁₋₆alkanoyl, or C₁₋₆alkoxy. Where used herein the term naphthyl, whether alone or as part of another group, is intended, unless otherwise stated, to denote both 1-naphthyl and 2-naphthyl groups.

The term "aroyl" refers to the group aryl-CO- wherein "aryl" is as defined above.

The term "oxo" refers to the group "=O".

The term "optionally substituted 3 to 7 membered heterocyclic group" refers to an optionally substituted saturated or non-saturated ring containing at least one nitrogen atom and optionally a further 1 or 2 heteroatoms selected from nitrogen, sulphur or oxygen, the ring consisting of a total of 3 to 7 atoms. Examples of such heterocyclic groups include aziridinyl, azetidinyl, pyrrolidinyl, imidazolidinyl, pyrazolidinyl, oxazolidinyl, isothiazolidinyl, thiazolidinyl, pyrrolyl, pyrrolinyl, pyrazolinyl, imidazolyl, pyrazolyl, thiazolyl, piperidyl, piperazinyl, morpholinyl, thiomorpholinyl, azepinyl and azepanyl. The heterocyclic group may be substituted by one or more, preferably 1 to 3, substituents, which may be the same or different, and which is selected from the following group: halogen, oxo, C₁₋₆alkyl, cyano, CF₃, C₁₋₆alkoxy and C₁₋₆alkanoyl. The optional substituent(s) may be attached to any available carbon, nitrogen or sulphur atom. Substituents in the heterocyclic group may form a bridge structure, to form a group such as for example 2-oxa-5-azabicyclo[2.2.1]heptyl. Such a bicyclic group may be further substituted by one or more, preferably 1 to 3, halogen, oxo, C₁₋₆alkyl, cyano, CF₃, C₁₋₆alkoxy or C₁₋₆alkanoyl.

The term "C₃₋₇cycloalkylC₁₋₆alkoxy" refers to a cycloalkyl group consisting of from 3 to 7 carbon atoms (for example cyclopropane, cyclobutane, cyclopentane, cyclohexane and cycloheptane) attached to an arylC₁₋₆alkoxy group.

When a is two or more, the two or more R1 groups may be the same or different.

A is optionally substituted phenyl, naphthyl, indolyl, quinolinyl, quinazolinyl, indazolyl, isoquinolinyl or benzofuranyl. These groups may be attached to the oxygen atom at any suitable position. These groups may be substituted by 1 to 4 substituents, which may be the same or different, and which are selected from the following group: halogen, hydroxy, cyano, CF₃, C₁₋₆alkyl, C₁₋₆alkoxy, C₁₋₆alkoxyC₁₋₆alkyl, C₃₋₇cycloalkylC₁₋₆alkoxy, C₁₋₆alkanoyl, C₁₋₆alkylsulfonyl, C₁₋₆alkylsulfinyl, C₁₋₆alkylsulfonyloxy, C₁₋₆alkylsulfonylC_{1 -6}alkyl, C_{1 -6}alkylsulfonamido, C_{1 -6}alkylamido, C₁₋₆alkylsulfonamidoC₁₋₆alkyl and C₁₋₆alkylamidoC₁₋₆alkyl. Preferred optional substituents for A are C₁₋₆alkyl, cyano, CF₃, C₁₋₆alkoxy and C₁₋₆alkanoyl.

Preferably A is quinolinyl or quinazolinyl. Most preferably, A is 5-(2-methyl)quinolinyl or 5-(2-methyl)q uinazolinyl.

Preferably Y is CH or CH₂.

Preferably a is 0, 1 or 2.

Preferably R1 is fluoro.

Preferably R2 and R3 are independently hydrogen, C₁₋₆alkyl (particularly methyl, ethyl or propyl), C₁₋₆alkanoyl, C₁₋₆alkylsulfonyl, haloC₁₋₆alkanoyl or C₁₋₆alkylcarbamoyl. More preferably, one of R2 and R3 is hydrogen or C₁₋₆alkyl (particularly methyl, ethyl or propyl) and the other is C₁₋₆alkanoyl, C₁₋₆alkylsulfonyl, fluoroC₁₋₆alkanoyl, or C₁₋₆alkylcarbamoyl, or R2 and R3, together with the nitrogen atom to which they are attached, form a saturated 5 or 6 membered heterocyclic group such as piperazinyl, pyrrolidinyl, imidazolidinyl, isothiazolidinyl, thiazolidinyl, morpholinyl or piperidyl, optionally substituted by 1 or 2 substituent(s) selected from C₁₋₄alkyl and oxo. When R2 and/or R3 is a group CO(CH₂)bNR4R5, b is preferably 1. R4 and R5 may form an optionally substituted 3 to 7 membered heterocyclic group, preferably a saturated 5 or 6 membered heterocylic group, such as pyrrolidinyl or piperidyl.

Preferred compounds of this invention are example compounds E1-E122 (as described below) and pharmaceutically acceptable salts thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. Certain of the compounds of formula (I) may form acid addition salts with one or more equivalents of the acid. The present invention includes within its scope all possible stoichiometric and non-stoichiometric forms.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

Certain compounds of formula (I) are capable of existing in stereoisomeric forms (e.g. geometric (or *"cis-trans")* isomers, diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof. The present invention includes within its scope all such isomers, including mixtures.

In a further aspect, this invention provides a process for the preparation of a compound of formula (I) or a pharmaceutically acceptable salt thereof, which process comprises:
(a) the coupling of a compound of formula (II): 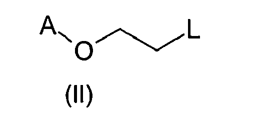 wherein A is as defined for formula (I) and L is a leaving group, and a compound of formula (III): 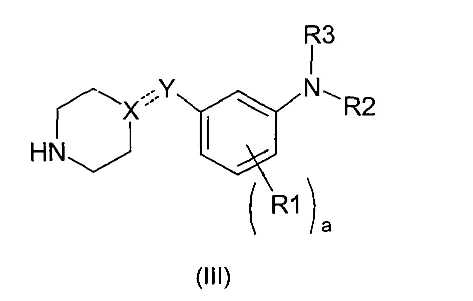 wherein a, R1, R2, R3, X, Y and 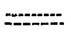 are as defined for formula (I); or
(b) for a compound wherein X is nitrogen, the coupling of a compound of formula (IV): 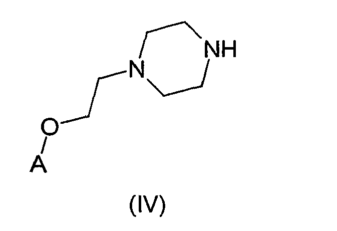 wherein A is as defined for formula (I), and a compound of formula (V): 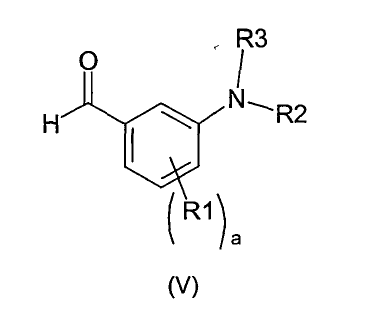 wherein a, R1, R2 and R3 are as defined for formula (I), or
(c) a Buchwald reaction between a compound of formula (VI): 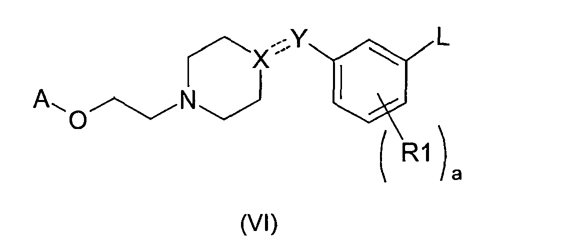 wherein L is a suitable leaving group and a, R1, X, Y and 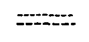 are as defined for formula (I), and a compound of formula (VII): 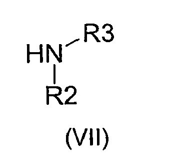 wherein R2 and R3 are as defined for formula (I);
   and thereafter optionally for process (a), (b) or (c):
   - removing any protecting groups and/or
   - converting a compound of formula (I) into another compound of formula (I) and/or
   - forming a pharmaceutically acceptable salt.

For process (a), the reaction of compounds of formulae (II) and (III) is preferably carried out in a suitable solvent such as isopropyl alcohol or *N,N*-dimethylformamide, in the presence of an appropriate base such as *N,N*-diisopropylethylamine or potassium carbonate. A suitable leaving group L is bromine.

For process (b), the reaction of compounds of formulae (IV) and (V) is preferably carried out in an aprotic solvent such as 1,2-dichloroethane, in the presence of an appropriate reducing agent such as sodium triacetoxyborohydride.

Standard conditions for a Buchwald reaction may be used for process (c). Suitable leaving groups are bromine and triflate.

Compounds of formula (I) can be converted into further compounds of formula (I) using standard techniques. For example, and by way of illustration rather than limitation, for compounds of formula (I) wherein ::::: is a double bond can be converted to compounds of formula (I) in which ::::: is a single bond by palladium catalysed hydrogenation in a suitable solvent such as ethanol. Other possible conversion reactions include acylation with an appropriate acylating agent such as acetyl chloride, alkylation using an appropriate alkylating reagent such as methyl iodide, and sulfonylation using a sulfonylating agent such as methanesulfonic anhydride.

Compounds of formulae (II) to (VII) are commercially available, may be prepared according to procedures described herein, by known literature methods, or by analogous procedures thereto.

For example, for compounds of the present invention wherein X is carbon and ::::: is a double bond, compounds of formula (III) wherein X is carbon may be prepared by reacting a compound of formula (VIII): 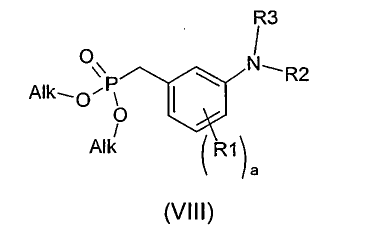 wherein "Alk" refers to an alkyl group, with a compound of formula (IX): 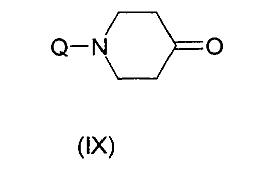 wherein Q is a protecting group such as t-butyloxycarbonyl, in the presence of a base such as sodium hydride, in a solvent such as tetrahydrofuran or *N*,*N*-dimethylformamide. The protecting group Q may be removed thereafter by any suitable means.

Compounds of formula (VIII) may be prepared by treating a compound of formula (X): 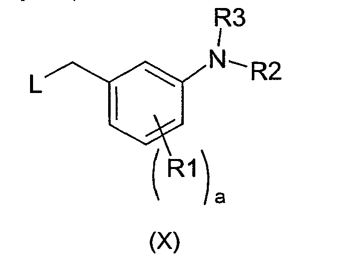 wherein L is a leaving group such as bromine, with a trialkyl phosphite such as triethyl phosphite or trimethyl phosphite, in the absence of solvent or in the presence of a solvent such as toluene.

It will be appreciated by those skilled in the art that it may be necessary to protect certain reactive substituents during some of the above procedures. Standard protection and deprotection techniques, such as those described in Greene T.W. *Protective groups in organic synthesis,* New York, Wiley (1981), can be used. For example, primary amines can be protected as phthalimide, benzyl, t-butyloxycarbonyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art. For example, protecting groups such as t-butyloxycarbonyl may be removed using an acid such as hydrochloric or trifluroroacetic acid in a suitable solvent such as dichloromethane, diethylether, isopropanol or mixtures thereof.

Pharmaceutically acceptable salts may be prepared conventionally by reaction with the appropriate acid or acid derivative.

The affinities of the compounds of this invention for 5-HT_{1A}, 5-HT_{1B} and 5-HT_{1D} receptors can be determined by the radioligand binding assay as described in WO 99/07700. All compounds tested according to the radioligand binding assay described above were found to have pKi values > 6.0 at 5-HT_{1A}, 5-HT_{1B} and 5-HT_{1D} receptors, with many showing a considerably higher affinity (having pKi values in the range 8.0 - 10.0).

The intrinsic activity of the compounds of this invention can be determined according to the [³⁵S]GTPγS functional assay which is also described in WO 99/07700. It has been found, using the [³⁵S]GTPγS functional assay, that certain compounds of formula (I) appear to be antagonists at 5-HT₁ type receptors whilst others appear to be inverse agonists, agonists or partial agonists.

Compounds of formula (I) and their pharmaceutically acceptable salts are of use in the treatment of certain CNS disorders such as depression (both bipolar and unipolar), single or recurrent major depressive episodes with or without psychotic features, catatonic features, melancholic features, atypical features or postpartum onset, seasonal affective disorder and dysthymia, anxiety disorders, including generalised anxiety, schizophrenia, panic disorder, agoraphobia, social phobia, obsessive compulsive disorder and post-traumatic stress disorder; pain (particularly neuropathic pain); memory disorders, including dementia, amnesic disorders and age-associated memory impairment; disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), sedative ipnotic, amphetamine or amphetamine-related drugs (e.g. dextroamphetamine, methylamphetamine) or a combination thereof, motor disorders such as Parkinson's disease, dementia in Parkinson's disease, neuroleptic-induced Parkinsonism and tardive dyskinesias, as well as other psychiatric disorders. Depressive disorders which may be treated or prevented by the compounds of formula (I) and their pharmaceutically acceptable salts may also result from a general medical condition including, but not limited to, myocardial infarction, diabetes, miscarriage or abortion, etc. Compounds of formula (I) may also have utility in the treatment of certain gastrointestinal disorders such as irritable bowel syndrome.

It is to be understood that "treatment" as used herein includes prophylaxis as well as alleviation of established symptoms.

Thus the invention also provides a compound of formula (I) or a pharmaceutically acceptable salt thereof, for use as a therapeutic substance, in particular in the treatmentof the above disorders. In particular the invention provides a compound of formula (I) or a pharmaceutically acceptable salt thereof for use as a therapeutic substance in the treatment of a CNS disorder, particularly depression or anxiety.

Compounds of the invention may be administered in combination with other active substances such as 5HT3 antagonists, serotonin agonists, NK-1 antagonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of the combination or composition may be administered simultaneously (either in the same or different pharmaceutical formulations), separately or sequentially.

The invention further provides a method of treatment of the above disorders, particularly a CNS disorder such as depression or anxiety, in mammals including humans, which comprises administering to the sufferer a therapeutically safe and effective amount of a compound of formula (I) or a pharmaceutically acceptable salt thereof.

In another aspect, the invention provides for the use of a compound of formula (I) or a pharmaceutically acceptable salt thereof in the manufacture of a medicament for use in the treatment of the above disorders, particularly a CNS disorder such as depression or anxiety.

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose);, fillers (e.g. lactose, microcrystalline cellulose or calcium hydrogen phosphate);, tabletting lubricants lubricants (e.g. magnesium stearate, talc or silica);, disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of the invention or pharmaceutically acceptable salt thereof and a sterile vehicle. Formulations for injection may be presented in unit dosage form e.g. in ampoules or in multi-dose, utilising a compound of the invention or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

Thus compounds of formula (I) may be formulated for oral, buccal, parenteral, topical (including ophthalmic and nasal), depot or rectal administration or in a form suitable for administration by inhalation or insufflation (either through the mouth or nose).

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The composition may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months.

The following Descriptions and Examples illustrate the preparation of compounds of the invention.

### Description 1

### Diethyl (3-nitrobenzyl)phosphonate (D1)

A mixture of 3-nitrobenzyl bromide (17.3 g, 0.08 mol), triethyl phosphite (13.3 g, 0.08 mol) in toluene (200 mL) was stirred at reflux for 24 h. The reaction mixture was cooled and evaporated *in vacuo*. Chromatography of the residues on SiO₂ eluting from 0-100% ethyl acetate in petroleum ether (60-80°C) gave the title compound (14.8 g, 68%) as a yellow liquid.
Mass spectrum (API⁺): Found 274 (MH⁺). C₁₁H₁₆NO₅P requires 273.
¹H NMR (CDCl₃) δ: 1.28 (6H, m), 3.26 (2H, d, J = 22 Hz), 4.11 (4H, m), 7.51 (1H, t, J = 8 Hz), 7.67 (1H, m), 8.15 (2H, m).

### Description 1a

### Diethyl (2-fluoro-4-methoxy-5-nitrobenzyl)phosphonate (D1a)

The **title compound** was prepared from 2-fluoro-4-methoxy-5-nitrobenzyl bromide in an analogous fashion to Description 1.

### Description 1b

### Diethyl (3-fluoro-4-methoxy-5-nitrobenzyl)phosphonate (D1 b)

The **title compound** was prepared from 3-fluoro-4-methoxy-5-nitrobenzyl bromide in an analogous fashion to Description 1.

### Description 2

### tert-Butyl 4-(3-nitrobenzylidene)piperidine-1-carboxylate (D2)

A mixture of diethyl (3-nitrobenzyl)phosphonate (8.4 g, 0.031 mol) and *tert*-butyl 4-oxopiperidine-1-carboxylate (6.12 g, 0.031 mol) in dry tetrahydrofuran (120 mL) was treated with a 60% suspension of sodium hydride in oil (1.36 g, 0.034 mmol). The resulting mixture was stirred at room temperature for 4h, then partitioned between dichloromethane (500 mL) and water (500 mL). The organic extract was dried (Na₂SO₄) and evaporated *in vacuo* to give the title compound (9.86 g, 100%) as a solid.
¹H NMR (CDCl₃) δ: 1.48 (9H, s), 2.38 (2H, m), 2.45 (2H, m), 3.43 (2H, m), 3.53 (2H, m), 6.39 (1 H, s), 7.49 (2H, m), 8.06 (2H, m),

### Description 3

### 4-(3-Nitrobenzylidene)piperidine hydrochloride (D3)

A mixture of *tert*-butyl 4-(3-nitrobenzylidene)piperidine-1-carboxylate (9.86 g, 0.031 mol), methanol (20 mL) and 1 M HCl in diethyl ether (200 mL) was stirred at 20 °C for 72h. The reaction mixture was evaporated *in vacuo* and the residue triturated in diethyl ether (3 x 100 mL) to give the title compound (6.67 g, 85%) as a solid.
Mass spectrum (API⁺): Found 219 (MH⁺). C₁₂H₁₄N₂O₂ requires 218.
¹H NMR (d⁶DMSO) δ: 2.61 (2H, m), 2.65 (2H, m), 3.39 (1H, m), 3.90 - 4.04 (4H, m), 6.58 (1H, s), 7.66 (1H, t, J = 8 Hz), 7.72 (1H, m), 8.04 (1H, m), 8.11 (1H, m), 9.40 (1H, bs).

### Description 4

### 3-(1-(2-(2-Methylquinolin-5-yloxy)ethyl)piperidin-4-ylidinemethyl)aniline dihydrochloride (D4)

A solution of 3-(1-(2-(2-methylquinolin-5-yloxy)ethyl)piperidin-4-ylidenemethyl)nitrobenzene (0.48 g, 0.0012 mol) in glacial acetic acid (5 mL) was treated with reduced iron powder (0.2 g, 0.0036 mol). The mixture was stirred at 80°C for 24h, cooled and filtered through celite washing with dichloromethane. The filtrate was washed with 2 M sodium hydroxide solution (20 mL). The organic layer was dried (Na₂SO₄) and evaporated *in vacuo.* The residues were suspended in 2N HCl (10 mL) and stirred at reflux for 2h, evaporated *in vacuo* and the residue triturated in ether to give the title compound (0.29 g, 54%) as a solid.
Mass spectrum (API⁺): Found 374 (MH⁺). C₂₄H₂₇N₃O requires 373.
¹H NMR (CD₃OD) δ: 2.72 - 2.84 (2H, m), 3.01 (5H, m), 3.22 - 3.39 (2H, m), 3.87 (4H, m), 4.77 (2H, m), 4.83 (4H, bs), 6.62 (1 H, s), 7.31 (2H, m), 7.39 (1 H, m), 7.44 (1 H, d, J = 8 Hz), 7.54 (1H, t, J = 8 Hz), 7.77 (1H, d, J = 9 Hz), 7.93 (1H, d, J = 9 Hz), 8.09 (1 H, t, J = 8 Hz), 9.57 (1H, m),

### Description 5

### tert-Butyl (3-(1-(2-(2-methylquinolin-5-yloxy)ethyl)piperidin-4-ylmethyl)phenyl)-carbamate (D5)

A mixture of 3-(1-(2-(2-methylquinolin-5-yloxy)ethylpiperidin-4-ylmethyl)aniline dihydrochloride (0.3 g, 0.00067 mol), di-*tert*-butyldicarbonate (0.15 g, 0.00067 mol) triethylamine (0.074 g, 0.00074 mol), tetrahydrofuran (2 mL) and water (0.5 mL) was stirred at 20 °C for 18h. The reaction mixture was partitioned between ethyl acetate (15 mL) and water (3 x 10 mL). The organic layer was dried (Na₂SO₄) and evaporated *in vacuo.* Chromatography of the residues on SiO₄ eluting from 50 - 100% ethyl acetate in petroleum ether (60 - 80 °C) gave the title compound (0.22 g, 69%) as an oil.
Mass spectrum (API⁺): Found 476 (MH⁺). C₂₉H₃₇N₃O₃ requires 475.
¹H NMR (CDCl₃) δ:1.33 (2H, m), 1.51 (9H, s), 1.67 (3H, m), 2.13 (2H, m), 2.51 (2H, d, J = 7 Hz), 2.72 (3H, s), 2.92 (2H, m), 3.01 (2H, m), 4.27 (2H, m), 6.45 (1 H, br s), 6.80 (2H, m), 7.13 - 7.26 (4H, m), 7.56 (2H, m), 8.43 (1H, d, J = 9 Hz).

### Description 6

### 4-Methyl-3-{1-[2-(2-methylquinolin-5-yloxy)ethyl]piperidin-4-ylidenemethyl}aniline (D6)

To a stirred solution of 2-methyl-5-{2-[4-(2-methyl-5-nitrobenzylidene)piperidin-1-yl]ethoxy}quinoline (prepared using an analogous route and intermediates to Example 1; 0.21 g, 0.51 mmol) in methanol (10 mL) was added concentrated hydrochloric acid (2.0 mL) and SnCl₂ (0.39 g, 2.04 mmol) and the mixture stirred at reflux under argon for 3 h. On cooling the mixture was evaporated *in vacuo*, the residue partitioned between dichloromethane and water and the suspension treated with 40% NaOH solution. The organic layers were separated, dried (Na₂SO₄) and evaporated *in vacuo* to give the title compound (0.19g, 96%).
Mass spectrum (API⁺): Found 388 (MH⁺). C₂₅H₂₉N₃O requires 387.

The following compounds were similarly prepared:
**(a) 2-Chloro-5-{1-[2-(2-methylquinolin-5-yloxy)ethyl]piperidin-4-ylidenemethyl}aniline**
**(b) 4-Methoxy-3-{1-[2-(2-methylquinolin-5-yloxy)ethyl]piperidin-4-ylidenemethyl}aniline**
**(c) 3-{1-[2-(2-Methylquinolin-5-yloxy)ethyl]piperidin-4-ylidenemethyl}-5-trifluoromethylaniline**
**(d) 2-Methyl-3-{1-[2-(2-methylquinolin-5-yloxy)ethyl]piperidin-4-ylidenemethyl}aniline**
**(e) 2-Chloro-3-{1-[2-(2-methyl-quinolin-5-yloxy)ethyl]piperidin-4-ylidenemethyl}aniline**
**(f) 2-Isopropyl-5-{1-[2-(2-methylquinolin-5-yloxy)ethyl]piperidin-4-ylidenemethyl}aniline**
**(g) 5-{1-[2-(2-Methylquinolin-5-yloxy)ethyl]piperidin-4-ylidenemethyl}-2-trifluoromethylaniline**
**(h) 3-Chloro-5-{1-[2-(2-methylquinolin-5-yloxy)ethyl]piperidin-4-ylidenemethyl}aniline**

### Description 7

### 2-Chloro-N-(3-(1-(2-(2-methylquinolin-5-yloxy)ethyl)piperidin-4-ylmethyl)phenyl)-acetamide (D7)

A mixture of 3-(1-(2-(2-methylquinolin-5-yloxy)ethyl)piperidin-4-ylmethyl)aniline dihydrochloride (0.15 g, 0.00033 mol) and triethylamine (0.17 g, 0.0017 mol) in dichloromethane (5 mL) was treated with chloroacetyl chloride (0.037 g, 0.00033 mol) and stirred at 20 °C for 2h. The mixture was washed with water (5 mL), and the organic layer added directly onto SiO₂. Elution from 0-10% methanol in ethyl acetate gave the title compound (0.11 g, 74%) as an oil.
Mass spectrum (API⁺): Found 452 (MH⁺). C₂₆H₃₀ ³⁵ClN₃O₂ requires 451.
¹H NMR (CDCl₃) δ: 1.40 (2H, m), 1.52 - 1.69 (3H, m), 2.17 (2H, m), 2.55 (2H, d, J = 7 Hz), 2.72 (3H, s), 2.97 (2H, m), 3.09 (2H, m), 4.18 (2H, s), 4.31 (2H, m), 6.79 (1H, d, J = 7 Hz), 6.95 (1H, d, J = 8 Hz), 7.25 (2H, m), 7.37 (2H, m), 7.57 (2H, m), 8.19 (1H, bs), 8.41 (1H, d, J = 9 Hz).

### Description 8

### 5-Fluoro-2-methyl-3,4-dihydroquinazoline (D8)

A solution of 2-amino-6-fluorobenzylamine (1.1 g, 7.86 mmol) and triethylorthoacetate (1.58 mL, 8.64 mmol) in ethanol (30 mL) was heated at 80°C for 14 h. The reaction mixture was allowed to cool to room temperature and evaporated *in vacuo.* The yellow oil was triturated with diethyl ether to give the **title compound** as white solid (0.74 g, 57%).
Mass spectrum (API⁺): Found 165 (MH⁺). C₉H₉N₂F requires 164.
¹H NMR (CDCl₃) δ: 2.02 (3H, s), 4.67 (2H, s), 6.34-6.71 (2H, m), 7.03-7.12 (1H, m).

### Description 9

### 5-Fluoro-2-methylquinazoline (D9)

To a solution of 5-fluoro-2-methyl-3,4-dihydroquinazoline (0.74 g, 4.51 mmol) in chloroform (100 mL) at room temperature was added manganese (IV) oxide (4.0 g, 46.0 mmol) and the reaction mixture stirred at room temperature for 20 h. The reaction mixture was filtered through a plug of celite, washing with dichloromethane. The filtrate was evaporated *in vacuo* to give the **title compound** as a yellow solid (0.715 g, 98%).
Mass spectrum (API⁺): Found 163 (MH⁺). C₉H₇N₂F requires 162.
¹H NMR (CDCl₃) δ: 2.92 (3H, s), 7.19-7.27 (1H, m), 7.77-7.83 (2H, m), 9.60 (1H, s).

### Description 10

### 2-(2-Methylquinazolin-5-yloxy)ethanol (D10)

To a solution of ethylene glycol (3.05 mL, 55.6 mmol) in *N,N*-dimethylformamide (50 mL) at room temperature was added sodium hydride (60% dispersion in oil, 0.30 g, 7.50 mmol) portionwise. The reaction mixture was allowed to stir at room temperature for 30 minutes. A solution of 5-fluoro-2-methylquinazoline (2.22 g, 55.6 mmol) in *N*,*N-*dimethylformamide (5 mL) was added and the reaction mixture heated at 85 °C for 14 h. The mixture was allowed to cool to room temperature, quenched by the addition of water and concentrated *in vacuo.* Chromatography of the residue on SiO₂ eluting with 40% ethyl acetate in dichloromethane to ethyl acetate gave the title compound as a yellow solid (0.39 g, 10%).
Mass spectrum (API⁺): Found 205 (MH⁺) C₁₁H₁₂N₂O₂ requires 204.
¹H NMR (CDCl₃) δ: 2.87 (3H, s), 4.13-4.16 (2H, m), 4.31-4.33 (2H, m), 6.88(1H, d, J = 8Hz), 7.50 (1H, d, J = 9 Hz), 7.72-7.76 (1H, m), 9.64 (1H, s).

### Description 11

### 5-[2-(Methanesulfonyloxy)ethoxy]-2-methylquinazoline (D11)

To a solution of 2-(2-methylquinazolin-5-yloxy)ethanol (0.330 g, 1.62 mmol) in dichloromethane (20 mL) and triethylamine (0.34 mL, 2.43 mmol) was added methane sulfonyl chloride (0.14 mL, 1.78 mmol) dropwise. The reaction mixture was allowed to stir at room temperature for 2 h. The reaction mixture was diluted with further dichloromethane and partitioned with saturated aqueous NaHCO₃ solution. The organic phase was washed with brine, dried (MgSO₄) and evaporated *in vacuo* to give the title compound as a cream solid (0.452 g, 99%).
Mass spectrum (ES⁺): Found 283 (MH⁺) C₁₂H₁₄N₂O₄S requires 282.
¹H NMR (CDCl₃) δ: 2.89 (3H, s), 3.10 (3H, s), 4.46-4.48 (2H, m). 4.71-4.73 (2H, m), 6.86 (1H, d, J = 8Hz), 7.55 (1H, d, J = 9 Hz), 7.74-7.78 (1H, m), 9.69 (1H, s).

### Description 12

### 2-Fluoro-4-methoxy-5-nitrobenzaldehyde (D12)

2-Fluoro-4-methoxy-benzaldehyde (36g) was added to mechanically stirred concentrated sulfuric acid (250 mL) at 0 °C. The solution was maintained at -15°C while nitric acid (70% *w*/*w*) (22 g) was added dropwise. Further stirring was allowed for 45 mins. at this temperature before the mixture was poured onto crushed ice (800 mL). The resulting precipitate was collected by filtration and partitioned between dichloromethane (800 mL) and saturated aqueous NaHCO₃ solution (1 L). The organic layer was dried (Na₂SO₄) and evaporated *in vacuo* to give the **title compound** (44.2 g, 95%) as a light yellow solid.
¹H NMR (CDCl₃) δ: 4.06 (3H, s), 6.87 (1H, d, J = 12 Hz), 8.46 (1H, d, J = 8 Hz), 10.22 (1H, s).

### Description 12a

### 3-Fluoro-4-methoxy-5-nitrobenzaldehyde (D12a)

The **title compound** was prepared in an analogous manner to Description 12.

### Description 13

### 2-Fluoro-4-methoxy-5-nitrobenzyl alcohol (D13)

Sodium borohydride (1.6 g) was added in portions to a stirring solution of 2-fluoro-4-methoxy-5-nitrobenzaldehyde (5.35 g) in methanol (50 mL) at 0 °C. The methanol was then removed *in vacuo* and the resulting residue was partitioned between cold water (100 mL) and dichloromethane (200 mL). The organic layer was dried (Na₂SO₄) and evaporated *in vacuo.* The residue was purified by silica gel chromatography eluting with ethyl acetate in hexane to give the **title compound** (3.35 g, 66%) as a light yellow solid.
¹H NMR (CDCl₃) δ: 1.85 (1H, t, J = 6 Hz), 3.96 (3H, s), 4.74 (2H, d, J = 6 Hz), 6.87 (1H, d, J = 12 Hz), 8.46 (1H, d, J = 8 Hz).

### Description 13a

### (3-Fluoro-4-methoxy-5-nitrobenzyl alcohol (D13a)

The title compound was prepared in an analogous manner to Description 13.

### Description 14

### 1-tert-Butoxycarbonyl-4-(3-nitrophenoxy)piperidine (D14)

A stirred solution of 1-*tert*-butoxycarbonylpiperidin-4-ol (2.0 g, 10 mmol), 3-nitrophenol (1.5 g, 11 mmol) and triphenylphosphine (5.2 g, 20 mmol) in dry THF (40 mL) at 0 °C under argon was treated dropwise over 10 minutes with diisopropyl azodicarboxylate (4.0 g, 20 mmol). The mixture was maintained at 20 °C for 2 h, then concentrated *in vacuo.* The residue was dissolved in diethyl ether (150 mL), washed with 1 M NaOH solution (100 mL) and dilute NaCl solution, then dried (Na₂SO₄) and concentrated *in vacuo.* Chromatography of the residue on silica gel eluting with 30-70% ether/60-80 petrol afforded a pale yellow oil (4.36 g) containing the title compound in approximately 72% purity, together with product from diisopropyl azodicarboxylate.
¹H NMR (CDCl₃) δ: 1.48 (9H, s), 1.73-1.82 (2H, m), 1.91-2.00 (2H, m), 3.33-3.42 (2H, m), 3.67-3.75 (2H, m), 4.54-4.61 (1 H, m), 7.23 (1 H, dd), 7.43 (1 H, t), 7.73 (1 H, t), 7.81 (1 H, dd).

### Description 15

### 4-(3-Nitrophenoxy)piperidine (D15)

The title compound was prepared from 1-*tert*-butoxycarbonyl-4-(3-nitrophenoxy)piperidine using a similar procedure to that described in Description 3.
Mass spectrum (API⁺): Found 223 (MH⁺). C₁₁H₁₄N₂O₃ requires 222.

### Description 16

### 5-{2-[4-(3-Aminophenoxy)piperidin-1-yl]ethoxy}-2-methylquinoline (D16)

The title compound was prepared from 5-{2-[4-(3-nitrophenoxy)piperidin-1-yl]ethoxy}-2-methylquinoline using a similar procedure to that described in Example 2.
Mass spectrum (API⁺): Found 378 (MH⁺). C₂₃H₂₇N₃O₂ requires 377.

### Description 17

### 5-[2-(4-Hydroxypiperidin-1-yl)ethoxy]-2-methylquinoline (D17)

The **title compound** was prepared from 5-(2-bromoethoxy)-2-methylquinoline and 4-hydroxypiperidine following the method of Example 1.
¹H NMR (CDCl₃) δ: 1.60-1.70 (2H, m), 1.88-1.98 (2H, m), 2.35-2.43 (2H, m), 2.73 (3H, s), 2.88-3.00 (2H, t +2H, m), 3.70-3.79 (1H, m), 4.27 (2H, t), 6.80 (1H, dd), 7.25 (1H, d), 7.53-7.62 (2H, m), 8.44 (1H, d). OH not discernible from spectrum.

### Description 18

### 5-{2-[4-(4-Chloro-3-nitrophenoxy)piperidin-1-yl]ethoxy}-2-methylquinoline (D18)

The title compound was prepared from 5-[2-(4-hydroxypiperidin-1-yl)ethoxy]-2-methylquinoline and the appropriate substituted phenol using a similar procedure to that described in Description 14.
Mass spectrum (API⁺): Found 442 (MH⁺). C₂₃H₂₄³⁵CIN₃O₄ requires 441.

### Description 19

### 5-{2-[4-(3-Amino-4-chlorophenoxy)piperidin-1-yl]ethoxy}-2-methylquinoline (D19)

The title compound was prepared from 5-{2-[4-(4-chloro-3-nitrophenoxy)piperidin-1-yl]ethoxy}-2-methylquinoline using a similar procedure to that described in Example 2
Mass spectrum (API⁺): Found 412 (MH⁺). C₂₃H₂₆³⁵ClN₃O₂ requires 411.

### Description 20

### Diethyl (3-bromobenzyl)phosphonate (D20)

The title compound was prepared from 3-bromobenzyl bromide in a similar manner to description D1.
Mass spectrum (API⁺): Found 307 (MH⁺). C₁₁H₁₆⁷⁹BrPO₃ requires 306.

### Description 21

### tert-Butyl 4-(3-bromobenzylidene)piperidine-1-carboxylate (D21)

The title compound was prepared from diethyl (3-bromobenzyl)phosphonate in a similar manner to Description 2.
¹H NMR (CDCl₃) δ: 1.48 (9H, s), 2.33 (2H, m), 2.43 (2H, m), 3.41 (2H, m), 3.50 (2H, m), 6.29 (1H, s), 7.11 (1H, m), 7.18 (1H, t, J = 8 Hz), 7.34 (2H, m).

### Description 22

### 4-(3-Bromobenzylidene)piperidine hydrochloride (D22)

The title compound was prepared from *tert*-butyl 4-(3-bromobenzylidene)piperidine-1-carboxylate in a similar manner to Description 3.
Mass spectrum (API⁺): Found 252 (MH⁺). C₁₂H₁₄⁷⁹BrN requires 251.

### Description 23

### 3-(1-(2-(2-Methylquinolin-5-yloxy)ethyl)piperidin-4-ylidenemethyl)bromobenzene (D23)

The title compound was prepared from 4-(3-bromobenzylidene)piperidine hydrochloride in a similar manner to Description 25.
Mass spectrum (API⁺): Found 437 (MH⁺). C₂₄H₂₅⁷⁹BrN₂O requires 436.

### Description 24

### 5-(2-(4-(3-Bromobenzylidene)piperidin-1-yl)ethoxy)-2-methylquinazoline (D24)

The title compound was prepared from 4-(3-bromobenzylidene)piperidine hydrochloride and 5-[2-(methanesulfonyloxy)ethoxy]-2-methylquinazoline in a similar manner to Description 25.
Mass spectrum (API⁺): Found 438 (MH⁺). C₂₃H₂₄⁷⁹BrN₃O requires 437.

### Description 25

### tert-Butyl 4-[2-(2-methylquinolin-5-yloxy)ethyl]piperazine-1-carboxylate (D25)

*tert*-Butyl piperazine-1-carboxylate (1.4 g, 7.52 mmol) was added to a mixture of 5-(2-bromoethoxy)-2-methylquinoline (2g, 7.52 mmol) and potassium carbonate (4.16 g, 30.1 mmol) in *N*,*N*-dimethylformamide (20 mL). The reactants were heated at 70°C for 16 h under an atmosphere of argon. The reaction mixture was poured into water (200 mL) and extracted into ethyl acetate (3 x 200 mL). The organic layers were combined, dried (Na₂SO₄) and concentrated *in vacuo.* The residue was purified by column chromatography, eluting with 30% ethyl acetate in hexane affording the title compound as a tan solid (1.04 g, 37%).
Mass spectrum (API⁺): Found 372.3 (MH⁺). C₂₁H₂₉N₃O₃ requires 371.
¹H NMR (CDCl₃) δ: 1.46 (9H, s), 2.59 (4H, t), 2.73 (3H, s), 2.96 (2H, t), 3.46 (4H, t), 4.29 (2H, t), 6.80 (1 H, dd), 7.26 (1 H, d), 7.58 (2H, m), 8.43 (1 H, d).

### Description 26

### 2-Methyl-5-(2-piperazin-1-ylethoxy)quinoline (D26)

tert-Butyl 4-[2-(2-methylquinolin-5-yloxy)ethyl]piperazine-1-carboxylate (1.04 g, 2.8 mmol) was dissolved in ethanol (60 mL) and treated with 1 M hydrochloric acid in diethyl ether (16 mL, 16 mmol) and stirred at 40 °C for 17 h. The reaction mixture was filtered and the white solid was collected and dried *in vacuo*. The hydrochloride salt precipitate was dissolved in water (25 mL) and potassium carbonate was added until the pH reached 10. The aqueous layer was washed with 5% methanol in dichloromethane (4 x 100 mL) then 10% methanol in dichloromethane (4 x 100 mL). The organic layers were combined, dried (Na₂SO₄) and concentrated *in vacuo*, affording the **title compound** as a brown oil (0.69 g, 91%).
Mass spectrum (API⁺): Found 272 (MH⁺). C₁₆H₂₁N₃O requires 271.
¹H NMR (CDCl₃) δ: 2.62 (4H, m), 2.73 (3H, s), 2.92 (6H, m), 3.47 (1 H, s), 4.29 (1 H, d), 6.80 (1 H, dd), 7.50 (1 H, d), 7.58 (2H, m), 8.45 (1 H, d). NH not discernible.

### Description 27

### 2-(5-Quinolinyloxy)ethyl bromide (D27)

A mixture of 5-hydroxyquinoline (0.3 g, 2.1 mmol), 1,2-dibromoethane (3.9 g, 21 mmol) and potassium carbonate (1.5 g, 11 mmol) in methyl ethyl ketone (15 mL) was allowed to stir at 85 °C for 24 h. The mixture was evaporated *in vacuo* and the residue was partitioned between ether (200 mL) and water (200 mL). The organic layer was dried (Na₂SO₄) and evaporated *in vacuo* to give the **title compound** (0.53 g).
¹H NMR (CDCl₃) δ: 3.80 (2H, m), 4.49 (2H, m), 6.86 (1H, d, J = 8 Hz), 7.41 (1H, dd, J = 8, 4 Hz), 7.61 (1H, t, J = 8 Hz), 7.73 (1H, d, J = 8 Hz), 8.64 (1H, d, J = 8 Hz), 8.91 (1H, m).

### Description 28

### 5-Hydroxy-2-methylquinoline (D28)

A mixture of 2-methyl-5,6,7,8-tetrahydroquinolin-5-one [E.Reimann, J. Freisinger, *Arch. Pharm.* (*Weinheim*)*,* **318,** 871 (1985)] (0.57 g, 3.5 mmol) and 48% aqueous HBr (3.5 mL) was warmed to 60 °C and treated dropwise with bromine (0.19 mL, 0.59 g, 3.6 mmol), with vigorous stirring. The resulting mixture was stirred at 60 °C for 1 h, then evaporated *in vacuo.* The residue was treated with isopropanol with stirring, then the mixture was evaporated *in vacuo* to give a waxy solid, which was triturated with 1:1 isopropanol - ether to give a beige powder (0.9 g). A mixture of this material, lithium carbonate (0.48 g, 6.7 mmol), lithium bromide (0.28 g, 3.2 mmol) and *N*,*N*-dimethylformamide (10 mL) was heated at 150 °C under argon with stirring for 2 h. The mixture was cooled then evaporated *in vacuo*. Chromatography of the residue on silica with 0 - 100% ethyl acetate - hexane gradient elution gave the **title compound** (0.28 g, 49%) as a solid.
Mass spectrum (API⁺): Found 160 (MH⁺). C₁₀H₉NO requires 159.

### Description 29

### 5-(2-Bromoethoxy)-2-methylquinoline (D29)

The **title compound** was prepared from 5-hydroxy-2-methylquinoline and 1,2-dibromoethane using a similar procedure to Description 27, in 91 % yield.
Mass spectrum (API⁺): Found 266 (MH⁺). C₁₂H₁₂⁷⁹BrNO requires 265.

### Description 30

### 5-Bromo-2-methoxy-3-nitrobenzyl bromide (D30)

Sodium borohydride (4 g) was added in portions to a stirring solution of crude 5-bromo-2-methoxy-3-nitrobenzaldehyde (20.5 g) in methanol (350 mL) and tetrahydrofuran (150 mL) at 0 °C. After 1h, the methanol was removed *in vacuo.* The residue was treated with cold water (150 mL) and extracted with diethyl ether (2 x 150 mL). The combined organic layer was evaporated *in vacuo* to give a crude oil. Silica gel chromatography eluting with ethyl acetate in petroleum ether (10-40%) gave the **title compound** (17 g) as a solid.
¹H NMR (CDCl₃) δ: 2.00 (1 H, t, J = 6 Hz), 3.92 (3H, s), 4.80 (2H, d, J = 6 Hz), 7.84 (1 H, d, J = 2 Hz), 7.91 (1H, d, J = 2 Hz).

### Description 31

### 5-Bromo-2-methoxy-3-nitrobenzyl alcohol (D31)

2,6-Lutidine (10.5 mL) was added to a stirring solution of 5-bromo-2-methoxy-3-nitrobenzyl alcohol (13.6 g) and lithium bromide (11.75 g) in anhydrous tetrahydrofuran (200 mL) at 0 °C. A solution of methanesulfonic anhydride (11.8 g) in anhydrous tetrahydrofuran (20 mL) was added dropwise. The resulting mixture was left to stir at room temperature for 16 h. It was partitioned between diethyl ether (250 mL) and saturated sodium hydrogen carbonate (150 mL). The organic layer was dried (sodium sulfate) and evaporated *in vacuo.* Silica gel chromatography of the crude residue eluting with diethyl ether in petroleum ether gave the **title compound** (20 g) as an amber oil.
¹H NMR (CDCl₃) δ: 4.06 (3H, s), 4.48 (2H, s), 7.76 (1H, d, J = 2 Hz), 7.93 (1H, d, J = 2Hz).

### The following two compounds were prepared in an analogous manner to Description 31:

### Description 31a

### 2-Fluoro-4-methoxy-5-nitrobenzyl bromide (D31a)

### Description 31b

### 3-Fluoro-4-methoxy-5-nitrobenzyl bromide (D31b)

### Description 32

### 8-Chloro-4-hydroxy-5-methoxy-2-(trifluoromethyl)quinoline (D32)

A mixture of 2-chloro-5-methoxyaniline hydrochloride (10.0 g, 0.063 mol) and ethyl (trifluoromethyl)acetoacetate (10.3 mL, 0.070mol) in polyphosphoric acid (40 mL) was heated to 160°C under argon for 2 h. Water (200 mL) was added with care and the crude product extracted (EtOAc x 2). Chromatography (SiO₂; eluant 20% 60-80° petrol/EtOAc) afforded the title compound as a dark yellow solid (3.38 g, 19%).
¹H NMR (400MHz, CDCl₃) δ: 4.13 (s, 3H), 6.85 (d, 1 H), 7.19 (s, 1 H), 7.75 (d, 1 H), 10.05 (s, 1H).

### Description 33

### 4,8-Dichloro-5-methoxy-2-(trifluoromethyl)quinoline (D33)

A mixture of 8-chloro-4-hydroxy-5-methoxy-2-(trifluoromethyl)quinoline (3.38 g, 0.012 mol) and phosphorus pentachloride (2.08 g, 0.01 mol) in phosphorus oxychloride (20 mL) was heated at reflux for 2.5 h. On cooling, water (100 mL) was added with care and the product extracted (CH₂Cl₂ x2). The organics were dried (Na₂SO₄) and evaporated *in vacuo* to give the **title compound** as a yellow solid (3.19 g, 90%).
¹H NMR (400MHz, CDCl₃) δ: 4.00 (s, 3H), 6.97 (d, 1 H), 7.79 (s, 1H), 7.85 (d, 1H).

### Description 34

### 5-Methoxy-2-(trifluoromethyl)quinoline (D34)

A solution of 4,8-dichloro-5-methoxy-2-(trifluoromethyl)quinoline (2.9 g, 9.8 mmol) in 1 M ethanolic potassium hydroxide (100 mL) was hydrogenated over 10% palladium on carbon (50% aqueous paste; 500 mg) at atmospheric temperature and pressure for 18 h. The mixture was filtered through celite, the filtrate evaporated *in vacuo* and the residue partitioned between CH₂Cl₂ and water. The organics were dried (Na₂SO₄) and evaporated *in vacuo.* Chromatography (SiO₂; eluant 20% EtOAc/60-80° petrol) afforded the title compound as a yellow solid (850 mg, 38%).
¹H NMR (400MHz, CDCl₃) δ: 4.04 (s, 3H), 6.96 (d, 1 H), 7.70-7.73 (m, 2H), 7.80 (d, 1H), 8.76 (d, 1H).

### Description 35

### 2-(Trifluoromethyl)quinolin-5-ol (D35)

To a solution of 5-methoxy-2-(trifluoromethyl)quinoline (830 mg, 3.65 mmol) in CH₂Cl₂ (20mL) at 0°C was added dropwise boron tribromide (1.0 mL, 10.95 mmol). The mixture was stirred under argon whilst allowing to warm to room temp. for 2h. Water (50 mL) was added with care and the organics separated, dried (Na₂SO₄) and evaporated *in vacuo* to give a pale yellow oil (570 mg, 73%).
¹H NMR (400MHz, CDCl₃) δ: 5.97 (br s, 1 H), 6.97 (d, 1H), 7.63 (t, 1H), 7.73 (d, 1H), 7.82 (d, 1 H), 8.77 (d, 1H).

### Description 36

### 5-(2-Bromoethoxy)-2-(trifluoromethyl)quinoline (D36)

A mixture of 2-(trifluoromethyl)quinolin-5-ol (563 mg, 2.64 mmol), potassium carbonate (1.8 g, 13.0 mmol) and 1,2-dibromoethane (2.3 mL, 26.0 mmol) in methyl ethyl ketone (20 mL) was heated at reflux under argon for 16 h. The solvent was removed *in vacuo* and the residue partitioned between water and CH₂Cl₂. The organics were dried (Na₂SO₄) and evaporated. Chromatography (SiO₂; eluant 20% to 50% EtOAc/60-80° petrol) afforded the **title compound** as a buff solid (600 mg, 71%).
¹H NMR (400MHz, CDCl₃) δ: 3.80 (t, 2H), 4.52 (t, 2H), 6.95 (d, 1H), 7.69-7.74 (m, 2H), 7.84 (d, 1H), 8.82 (d, 1H).

### Description 37

### C-[(2-Hydroxyethyl)methylamino]-N-(3-{1-[2-(2-methylquinolin-5-yloxy)ethyl]piperidin-4-ylmethyl}phenyl)acetamide (D37)

The **title compound** was prepared from 2-chloro-*N*-(3-{1-[2-(2-methylquinolin-5-yloxy)ethyl]piperidin-4-ylmethyl}phenyl)acetamide in a manner similar to Example 46.
Mass spectrum (API⁺): Found 491. C₂₉H₃₈N₄O₃ requires 490.

### Description 38

### C-[Benzyl-(2-hydroxyethyl)amino]-N-(3-{1-[2-(2-methylquinolin-5-yloxy)ethyl]piperidin-4-ylmethyl}phenyl)acetamide (D38)

The title compound was prepared from 2-chloro-*N*-(3-{1-[2-(2-methylquinolin-5-yloxy)ethyl]piperidin-4-ylmethyl}phenyl)acetamide in a manner similar to Example 46.
Mass spectrum (API⁺): Found 567. C₃₅H₄₂N₄O₃ requires 566.

### Description 39

### C-[(2-Chloroethyl)methylamino]-N-(3-{1-[2-(2-methylquinolin-5-yloxy)ethyl]piperidin-4-ylmethyl}phenyl)acetamide (D39)

Methanesulfonyl chloride (40 mg, 0.35 mmol) was added to a solution of *C*-[(2-hydroxyethyl)methylamino]-*N*-(3-{1-[2-(2-methylquinolin-5-yloxy)ethyl]piperidin-4-ylmethyl}phenyl)acetamide (170 mg, 0.35 mmol), in pyridine (2 ml) and stirred, under argon, at ambient temperature for 1 h. The reaction mixture was partitioned between ethyl acetate (5 ml) and water (5 ml). The organic layer was removed and dried over Na₂SO₄, filtered and the solvent removed *in vacuo* to give the **title compound** (130 mg, 74%) as a brown oil.
Mass spectrum (API⁺): Found 509. C₂₉H₃₇³⁵ClN₄O₂ requires 508.

### Description 40

### C-[Benzyl-(2-chloroethyl)amino]-N-(3-{1-[2-(2-methylquinolin-5-yloxy)ethyl]piperidin-4-ylmethyl}phenyl)acetamide (D40)

The title compound was prepared from *C*-[benzyl-(2-hydroxyethyl)amino]-*N*-(3-{1-[2-(2-methylquinolin-5-yloxy)ethyl]piperidin-4-ylmethyl}phenyl)acetamide in a manner similar to Description 39.
Mass spectrum (API⁺): Found 585. C₃₅H₄₁³⁵CIN₄O₂ requires 584.

### Description 41

### tert-Butyl 4-(3-iodobenzylidene)piperidine-1-carboxylate (D41)

The **title compound** was prepared in a manner analogous to *tert-*butyl 4-(3-bromobenzylidene)piperidine-1-carboxylate (Description 22).
1H NMR (CDCl₃) δ: 1.48 (9H, s), 2.32 (2H, m), 2.42 (2H, m), 3.40 (2H, m), 3.50 (2H, m), 6.26 (1H, s), 7.04 (1H, t J 8), 7.14 (1H, m), 7.54 (2H, m).

### Description 42

### tert-Butyl 4-(3-pyrazol-1-ylbenzylidene)piperidine-1-carboxylate (D42)

A mixture of *tert*-butyl 4-(3-iodobenzylidene)piperidine-1-carboxylate (500 mg, 1.25 mmol), pyrazole (95 mg, 1.4 mmol), copper (I) iodide (5 mol%, 12 mg, 0.06 mmol), *trans-*1,2-diaminocyclohexane (10 mol%, 14 mg, 0.13 mmol), and potassium phosphate (530 mg, 2.5 mmol) in 1,4-dioxan (5 ml) was stirred at 110 °C for 24 h. The reaction mixture was cooled to room temperature and partitioned between ethyl acetate (10 ml) and water (10 ml). The organic layer was separated and evaporated to dryness *in vacuo.* The residue was purified by SiO₂ chromatography on a 10 g pre-packed column, eluting from 30-100% ethyl acetate in petroleum ether (60-80 °C) to give the **title compound** (65 mg, 15%) as a colourless oil.
Mass spectrum (API⁺): Found 340. C₂₀H₂₅N₃O₂ requires 339.

### Description 43

### 4-(3-Pyrazol-1-ylbenzylidene)piperidine (D43)

The **title compound** was prepared from *tert*-butyl 4-(3-pyrazol-1-ylbenzylidene)piperidine-1-carboxylate in a manner similar to Description 3.
Mass spectrum (API⁺): Found 240. C₁₅H₁₇N₃ requires 239.

### Description 44

### tert Butyl 4-(3-bromo-4-fluorobenzylidene)piperidine-1-carboxylate (D44)

The title compound was prepared using analogous routes and intermediates to those used to prepare Description 21.
Mass spectrum (API⁺): Found 270. C₁₇H₂₁BrFNO₂ requires 370.

### Description 45

### tert butyl 4-[4-fluoro-3-(4-methylpiperazin-1-yl)benzylidene]piperidine-1-carboxylate (45)

The **title compound** was prepared from *tert* butyl 4-(3-bromo-4-fluorobenzylidene)piperidine-1-carboxylate using the method of Example 58.

### Description 46

### 1-(2-Fluoro-5-(piperidin-4-ylidenemethyl)phenyl)-4-methylpiperazine (D46)

The **title compound** was prepared from *tert* butyl 4-[4-fluoro-3-(4-methyl-piperazin-1-yl)-benzylidene]-piperidine-1-carboxylate using the method of Description 3.
Mass spectrum (API⁺): Found 290. C₁₇H₂₄FN3 requires 289.

### Description 47

### 1-(2-Fluoro-5-(piperidin-4-ylmethyl)phenyl)-4-methylpiperazine (D47)

The **title compound** was prepared from 1-(2-fluoro-5-(piperidin-4-ylidenemethyl)phenyl)-4-methylpiperazine using the method of Example 1.
Mass spectrum (API⁺): Found 292 (MH⁺). C₁₇H₂₆N₃F requires 291.
¹H NMR (400 MHz, CDCl₃) δ1.24-1.36 (2H,m), 1.59-1.64 (1H, m br), 1.68 (2H, d *J* 13.8), 2.35 (3H, s), 2.48 (2H, d *J* 7.0), 2.61 (4H, t *J* 4.7), 2.63 (2H, d *J* 12.2), 3.11 (4H, t *J* 4.7), 3.17 (2H, d *J* 12.2), 4.63 (1H, s br), 6.68 (2H, m), 6.91 (1 H, m).

### Description 48

### tert-Butyl 4-cyano-4-(3-nitrobenzyl)piperidine-1-carboxylate (D48)

A stirred solution of diisopropylamine (0.58 g, 5.8 mmol) in dry THF (30 ml) at -60 °C under argon was treated with 1.6M n-butyllithium in hexane (3.2 ml, 5.2 mmol) and maintained at -60°C for 10 minutes. The mixture was treated with a solution of *tert*-butyl 4-cyanopiperidine-1-carboxylate (1.0 g, 4.8 mmol) in THF and maintained at this temperature for a further 15 minutes, then treated with a solution of 3-nitrobenzyl bromide (1.08 g, 5 mmol) in THF and solution kept at this temperature for 30 minutes. The mixture was allowed to reach room temperature and stir for one hour before the addition of NH₄Cl solution (10 ml). The resulting mixture was concentrated *in vacuo* and the aqueous treated with 10% Na₂CO₃ solution and extracted with ethyl acetate. The extract was dried (Na₂SO₄) and solvent removed *in vacuo* to afford a brown oil, which was chromatographed on silica gel eluting with 10-40% ethyl acetate/60-80 petrol to afford the title compound as a clear gum. (255 mg, 15 %).
Mass spectrum (API⁺): Found 246 (MH⁺). C₁₈H₂₃N₃O₄ requires 345.
¹H NMR (400 MHz, CDCl₃) δ 1.46 (9H, s), 1.54 (2H, br), 1.83-1.87 (2H, br), 2.98 (2H, s), 2.99 (2H, br), 4.20 (2H, br), 7.56 (1H, t *J* 8.0), 7.68 (1H, d *J* 7.6), 8.11 (1H, s), 8.19 (1H, d *J* 8.4).

### Description 49

### 5-Hydroxyquinoline-N-oxide (D49)

A stirred suspension of 5-hydroxyquinoline (1.0 g, 6.9 mmol) in DCM (20 ml) at room temperature was treated with a solution of 3-chloroperbenzoic acid (8.0 mmol) in DCM (15 ml). After 2 h the precipitate was filtered off, washed with DCM and dried to afford the **title compound** as a white solid (1.0 g, 90%).
¹H NMR (d⁶DMSO) δ: 7.07 (1H, d), 7.38 (1 H, dd), 7.60 (1H, t), 7.97 (1 H, d), 8.02 (1 H, d), 8.54 (1H, d), 10.88 (1 H, s).

### Description 50

### 5-Hydroxyquinoline-2-carbonitrile (D50)

A stirred mixture of 5-hydroxyquinoline-N-oxide (1.0 g, 6.2 mmol), sodium cyanide (0.60 g, 12 mmol) and triethylamine (3.1 g, 31 mmol) in DMF (12 ml) at room temperature under argon was treated dropwise over 0.5 h with chlorotrimethylsilane (2.9 g, 24 mmol), then heated at 100 °C for 3 h. The cooled mixture was filtered, then concentrated under vacuum and the residue treated with methanol (40 ml) and maintained at room temperature for 40 mins, then concentrated under vacuum. The residue was chromatographed on silica gel eluting with DCM/ether to afford the **title compound** as a yellow solid (0.44 g, 42%).
¹H NMR (d⁶DMSO) δ: 7.11 (1H, d), 7.59 (1H, d), 7.74 (1H, t), 7.95 (1 H, d), 8.73 (1 H, d), 10.92 (1H, s).

### Description 51

### 5-(2-Bromoethoxy)quinoline-2-carbonitrile (D51)

A stirred mixture of 5-hydroxyquinoline-2-carbonitrile (0.27 g, 1.6 mmol), potassium carbonate (0.66 g, 4.8 mmol) and 1,2-dibromoethane (3.0 g, 16 mmol) in 2-butanone (30 ml) was heated at reflux for 4 h, then concentrated under vacuum. The residue was treated with water and extracted with ethyl acetate. The extract was dried (Na₂SO₄), concentrated under vacuum and chromatographed on silica gel eluting with 20-50% ethyl acetate/60-80 petrol, followed by crystallisation from 20% ethyl acetate/60-80 petrol, to afford the **title compound** as pale yellow solid (0.27 g, 63%).
Mass spectrum (API+): Found 277/279 (MH⁺). C₁₂H₉BrN₂O requires 276/278.
¹H NMR (CDCl₃) δ: 3.80 (2H, t), 4.50 (2H, t), 6.98 (1H, d), 7.68-7.82 (m, 3H), 8.78 (1H, d).

### Description 52

### 3-{1-[2-(2-Cyanoquinolin-5-yloxy)ethyl]piperidin-4-ylidenemethyl}bromobenzene (D52)

A mixture of 5-(2-bromoethoxy)quinoline-2-carbonitrile (100 mg, 0.36 mmol), 4-(3-bromobenzylidene)piperidine hydrochloride (0.42 mmol), potassium carbonate (150 mg, 1.1 mmol) and sodium iodide (165 mg, 1.1 mmol) in DMF (5 ml) was stirred at room temperature for 48 h, then concentrated under vacuum and the residue treated with 10% sodium carbonate solution and extracted with ethyl acetate. The extract was dried (Na₂SO₄), concentrated under vacuum and the residue chromatographed on silica gel eluting with ethyl acetate to afford the **title compound** as a yellow oil (140 mg, 87%).
Mass spectrum (API+): Found 448/450 (MH⁺). C₂₄H₂₂BrN₃O requires 447/449.
¹H NMR (CDCl₃) δ: 2.40-2.46 (2H, m), 2.50-2.55 (2H, m), 2.58-2.64 (2H, m), 2.70-2.76 (2H, m), 2.97 (2H, t), 4.33 (2H, t), 6.23 (1H, s), 7.00 (1H, dd), 7.08-7.13 (1H, m), 7.13-7.20 (1H, m), 7.29-7.36 (2H, m), 7.64-7.78 (3H, m), 8.70 (1H, d).

### Example 1

### 3-(1-(2-(2-Methylquinolin-5-yloxy)ethyl)piperidin-4-ylidenemethyl)nitrobenzene (E1)

A mixture of 5-(2-bromoethoxy)-2-methylquinoline (0.52 g, 1.95 mmol), 4-(3-nitrobenzylidene)piperidine hydrochloride (0.5 g, 1.96 mmol), and potassium carbonate (0.83 g, 5.97 mmol) in *N*,*N*-dimethylformamide (5 mL) was stirred at 100 °C for 16 h. The reaction mixture was cooled and partitioned between ethyl acetate (25 mL) and water (3 x 20 mL). The organic layer was dried (Na₂SO₄) and evaporated *in vacuo.* Chromatography of the residue on SiO₂ eluting with 30 - 100% ethyl acetate in hexane gave the **title compound** (0.62 g, 78%) as an oil.
Mass spectrum (API⁺): Found 404 (MH⁺). C₂₄H₂₅N₃O₃ requires 403.
¹H NMR (CDCl₃) δ: 2.47 (2H, m), 2.54 (2H, m), 2.66 (2H, m), 2.73 (3H, s), 2.77 (2H, m), 2.99 (2H, m), 4.30 (2H, m), 6.33 (1H, s), 6.82 (1H, d, J = 7 Hz), 7.26 (1H, m), 7.50 (2H, m), 7.58 (2H, m), 8.05 (2H, m), 8.44 (1H, d, J = 8 Hz).

### Example 2

### 3-(1-(2-(2-(Methylquinolin-5-yloxy)ethyl)piperidin-4-ylmethyl)aniline dihydrochloride (E2)

A mixture of 3-(1-(2-(2-methylquinolin-5-yloxy)piperidin-4-ylidenemethyl)nitrobenzene (0.99 g, 2.46 mmol) and 10% palladium on charcoal (paste) (0.1 g) in methanol (40 mL) was stirred at 20 °C, under an atmosphere of hydrogen (1 atm) for 24 h. The reaction mixture was filtered through celite and the filtrate treated with 1 M HCl in ether (10 mL) and evaporated *in vacuo* to give the **title compound** (1.10 g, 100%) as a solid.
Mass spectrum (API⁺): Found 376 (MH⁺). C₂₄H₂₉N₃O requires 375.
¹H NMR (CD₃OD) δ: 1.78 (2H, m), 1.93 (2H, m), 2.01 (1H, m), 2.72 (2H, m), 3.01 (3H, m), 3.21 (2H, m), 3.76 (4H, m), 4.73 (2H, m), 4.84 (4H, m), 7.27 (2H, m), 7.36 (1H, m), 7.42 (1H, d, J = 8 Hz), 7.48 (1H, t, J = 8 Hz), 7.77 (1 H, m), 7.92 (1H, d, J = 8 Hz), 8.07 (1H, m), 9.52(1H,d,J=9Hz).

### Example 3

### N-(3-(1-(2-(2-Methylquinolin-5-yloxy)ethyl)piperidin-4-ylmethyl)phenyl)methanesulfonamide (E3)

A mixture of 3-(1-(2-(2-methylquinolin-5-yloxy)ethyl)piperidin-4-ylmethyl)aniline dihydrochloride (0.17 g, 0.38 mmol) and triethylamine (0.15 g, 1.49 mmol) in dichloromethane (8 mL) was treated with methanesulfonic anhydride (0.073 g, 0.42 mmol) and stirred at 20 °C for 1 h. The reaction mixture was washed with water (5 mL) and the organic layer separated and added to SiO₂, eluting from 0-15% methanol in ethyl acetate gave the **title compound** (0.084 g, 49%) as an oil.
Mass spectrum (API⁺): Found 454 (MH⁺). C₂₅H₃₁N₃O₃S requires 453.
¹H NMR (CDCl₃) δ: 1.36 (2H, m), 1.54 (1 H, m), 1.64 (2H, m), 2.14 (2H, m), 2.53 (2H, d, J = 7 Hz), 2.72 (3H, s), 2.93 (2H, m), 3.00 (3H, s), 3.04 (2H, m), 4.27 (2H, m), 6.78 (1 H, d, J = 7 Hz), 6.96 (1 H, d, J = 8 Hz), 7.04 (2H, m), 7.24 (2H, m), 7.56 (2H, m), 8.43 (1 H, d, J = 9 Hz).

### Example 4

### N-Methyl-N-(3-(1-(2-(2-methylquinolin-5-yloxy)ethyl)piperidin-4-ylmethyl)phenyl)methanesulfonamide (E4)

A solution of *N-*(3*-*(1-(2-(2-methylquinolin-5-yloxy)ethyl)piperidin-4-ylmethyl)phenyl)methanesulfonamide (0.03 g, 0.066 mmol) in dry tetrahydrofuran (6 mL) was treated with a 60% suspension of sodium hydride in oil (0.01 g, 025 mmol) and the mixture stirred at 20 °C for 0.2 h. Methyl iodide (0.05 g, 0.35 mmol) was added and the mixture stirred for a further 1 h. The reaction mixture was partitioned between ethyl acetate (15 mL) and water (3 x 10 mL) and the organic layer was evaporated *in vacuo.* Chromatography of the residue on SiO₂ eluting from 0-15% methanol in ethyl acetate gave the **title compound** (0.0067 g, 22%) as an oil.
Mass spectrum (API⁺): Found 468 (MH)⁺. C₂₆H₃₃N₃O₃S requires 467.
¹H NMR (CDCl₃) δ: 1.41 (2H, m), 1.57 (1H, m), 1.67 (2H, m), 2.20 (2H, m), 2.56 (2H, m), 2.73 (3H, s), 2.84 (3H, s), 2.97 (2H, m), 3.08 (2H, m), 3.31 (3H, s), 4.30 (2H, m), 6.79 (1H, d, J = 7 Hz), 7.08 (1H, d, J = 8 Hz), 7.18 (1H, m), 7.27 (3H, m), 7.57 (2H, m), 8.42 (1H, d, J = 9 Hz).

### Example 5

### 1-(3-(1-(2-(2-Methylquinolin-5-yloxy)ethyl)piperidin-4-ylmethyl)phenyl)pyrrolidin-2-one (E5)

A mixture of 3-(1-(2-(2-methylquinolin-5-yloxy)ethyl)piperidin-4-ylmethyl)aniline hydrochloride (0.2 g, 0.45 mmol) and triethylamine (0.15 g, 1.49 mmol) in dichloromethane (10 mL) was treated with 4-bromobutyryl chloride (0.093 g, 0.5 mmol) and stirred at 20 °C for 1 h. The reaction mix was washed with water (10 mL) and the organic layer separated, dried (Na₂SO₄) and evaporated *in vacuo* to give an oil (0.2g) which was dissolved in tetrahydrofuran (10 mL) and treated with a 60% dispersion of sodium hydride in oil (16 m g, 0.4 mmol). The mixture was stirred at 20 °C for 18h, then partitioned between ethyl acetate (20 mL) and water (3x10 mL) and the organic layer was evaporated *in vacuo.* Chromatography of the residue on SiO₂ eluting with 0 - 20% methanol in ethyl acetate gave the **title compound** (0.086 g, 43%) as an oil.
Mass spectrum (API⁺): Found 444 (MH⁺). C₂₈H₃₃N₃O₂ requires 443.
¹H NMR (CDCl₃) δ: 1.35 (2H, m), 1.56 (1H, m), 1.68 (2H, m), 2.15 (4H, m), 2.51 (2H, m), 2.72 (3H, s), 2.75 (2H, m), 2.92 (2H, m), 3.03 (2H, m), 4.27 (2H, m), 4.31 (2H, m), 6.79 (1H, d, J = 7 Hz), 6.84 (1H, m), 6.93 (1H, m), 7.22 (3H, m), 7.56 (2H, m), 8.43 (1H, d, J = 8 Hz).

### Example 6

### N-(3-(1-(2-(2-Methylquinolin-5-yloxy)ethyl)piperidin-4-ylmethyl)phenyl)acetamide (E6)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 418 (MH⁺). C₂₆H₃₁N₃O₂ requires 417.
¹H NMR (CDCl₃) δ: 1.61 (3H, m), 1.71 (2H, m), 2.18 (3H, s), 2.40 (2H, m), 2.52 (2H, m), 2.73 (3H, s), 3.15 (2H, m), 3.27 (2H, m), 4.44 (2H, m), 6.81 (1H, d, J = 7 Hz), 6.85 (1H, m), 7.19 (1 H, m), 7.26 (1H, m), 7.33 (1 H, m), 7.38 (1H, m), 7.55 (1H, m), 7.63 (2H, m), 8.39 (1H, d, J = 8 Hz).

### Example 7

### N-(3-(1-(2-(2-Methylquinolin-5-yloxy)ethyl)piperidin-4-ylmethyl)phenyl)propionamide (E7)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 432 (MH⁺). C₂₇H₃₃N₃O₂ requires 431.
¹H NMR (CDCl₃) δ: 1.26 (3H, m), 1.56 (3H, m), 1.70 (2H, m), 2.36 (4H, m), 2.53 (2H, m), 2.73 (3H, s), 3.11 (2H, m), 3.23 (2H, m), 4.40 (2H, m), 6.80 (1 H, d, J = 8 Hz), 6.85 (1 H, m), 7.20 (1H, m), 7.28 (2H, m), 7.35 (1H, m), 7.42 (1H, m), 7.55 (1H, t, J = 8 Hz), 7.62 (1H, d, J = 8 Hz), 8.40 (1H, d, J = 9 Hz).

### Example 8

### N-(3-(1-(2-(2-Methylquinolin-5-yloxy)ethyl)piperidin-4-ylmethyl)phenyl)isobutyramide (E8)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 446 (MH⁺). C₂₈H₃₅N₃O₂ requires 445.
¹H NMR (CDCl₃) δ: 1.24 (6H, d, J = 7 Hz), 1.34 (2H, m), 1.53 (1H, m), 1.65 (2H, m), 2.13 (2H, m), 2.47 (1H, m), 2.51 (2H, m), 2.72 (3H, s), 2.92 (2H, m), 3.02 (2H, m), 4.26 (2H, m), 6.78 (1H, m), 6.87 (1H, m), 7.21 (3H, m), 7.33 (1H, m), 7.44 (1H, m), 7.56 (2H, m), 8.43 (1H, d, J = 9 Hz).

### Example 9

### N-(3-(1-(2-(2-Methylquinolin-5-yloxy)ethyl)piperidin-4-ylmethyl)phenyl)-2,2,2-trifluoroacetamide (E9)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 472 (MH⁺). C₂₆H₂₈F₃N₃O₂ requires 471.
¹H NMR (CDCl₃) δ: 1.34 (2H, m), 1.54 (1H, m), 1.63 (2H, m), 2.13 (2H, m), 2.54 (2H, d, J = 7 Hz), 2.71 (3H, s), 2.92 (2H, m), 3.02 (2H, m), 4.26 (2H, m), 6.79 (1 H, d, J = 7 Hz), 7.01 (1H, d, J = 8 Hz), 7.26 (2H, m), 7.39 (2H, m), 7.56 (2H, m), 8.31 (1H, bs), 8.43 (1H, d, J = 9 Hz).

### Example 10

### N-Methyl-N-(3-(1-(2-(2-methylquinolin-5-yloxy)ethyl)piperidin-4-ylmethyl)phenyl acetamide (E10)

The **title compound** was prepared from *N*-(3-(1-(2-(2-methylquinolin-5-yloxy)ethyl)piperidin-4-ylmethyl)phenyl)acetamide following a method analogous to that of Example 4.
Mass spectrum (API⁺): Found 432 (MH⁺). C₂₇H₃₃N₃O₂ requires 431.
¹H NMR (CDCl₃)δ: 1.37 (2H, m), 1.56 (1H, m), 1.66 (2H, m), 1.89 (3H, s), 2.16 (2H, m), 2.57 (2H, d, J = 7 Hz), 2.72 (3H, s), 2.94 (2H, m), 3.05 (2H, m), 3.26 (3H, s), 4.28 (2H, m), 6.79 (1H, d, J = 7 Hz), 6.97 (1H, m), 7.01 (1H, m), 7.11 (1H, m), 7.28 (2H, m), 7.57 (2H, m), 8.43 (1H, d, J = 8 Hz).

### Example 11

### N-Ethyl-N-(3-(1-(2-(2-methylquinolin-5-yloxy)ethyl)piperidin-4-ylmethyl)phenyl)acetamide (E11)

The title compound was prepared from N-(3-(1-(2-(2-methylquinolin-5-yloxy)ethyl)piperidin-4-ylmethyl)phenyl)acetamide following a method analogous to that of Example 4.
Mass spectrum (API⁺): Found 446 (MH⁺). C₂₈H₃₅N₃O₂ requires 445.
¹H NMR (CDCl₃) δ: 1.11 (3H, t, J = 7 Hz), 1.35 (2H, m), 1.56 (1 H, m), 1.65 (2H, m), 1.81 (3H, s), 2.15 (2H, m), 2.57 (2H, d, J = 7 Hz), 2.72 (3H, s), 2.93 (2H, m), 3.04 (2H, m), 3.73 (2H, q, J = 7 Hz), 4.27 (2H, m), 6.79 (1 H, d, J = 8 Hz), 6.93 (1 H, s), 6.98 (1 H, m), 7.12 (1 H, m), 7.24 (1 H, d, J = 9 Hz), 7.32 (1 H, t, J = 8 Hz), 7.56 (2H, m), 8.43 (1 H, d, J = 9 Hz).

### Example 12

### 1-Ethyl-3-(3-(1-(2-(2-methylquinolin-5-yloxy)ethyl)piperidin-4-ylmethyl)phenyl)urea (E12)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 447 (MH⁺). C₂₇H₃₄N₄O₂ requires 446.
¹H NMR (CDCl₃) δ: 1.10 (3H, m), 1.29 (2H, m), 1.53 (1H, m), 1.62 (2H, m), 2.10 (2H, m), 2.46 (2H, d, J = 7 Hz), 2.72 (3H, s), 2.90 (2H, m), 3.00 (2H, m), 3.25 (2H, m), 4.25 (2H, m), 5.34 (1H, m), 6.78 (2H, m), 7.07 (1 H, m), 7.15 (2H, m), 7.24 (2H, m), 7.56 (2H, m), 8.43(1H, d, J = 9 Hz).

### Example 13

### N-(3-(1-(2-(2-Methylquinolin-5-yloxy)ethyl)piperidin-4-ylidenemethyl)phenyl)acetamide (E13)

The **title compound** was prepared from 3-(1-(2-(2-methylquinolin-5-yloxy)ethyl)piperidin-4-ylidenemethyl)anilne dihydrochloride following a method analogous to that of Example 3.
Mass spectrum (API⁺): Found 416 (MH⁺). C₂₆H₂₉N₃O₂ requires 415.
¹H NMR (CDCl₃) δ: 2.14 (3H, s), 2.40 (2H, m), 2.53 (2H, m), 2.59 (2H, m), 2.72 (5H, m), 2.95 (2H, m), 4.28 (2H, m), 6.25 (1H, s), 6.80 (1H, d, J = 7 Hz), 6.93 (1H, m), 7.24 (2H, m), 7.33 (1H, m), 7.42 (1H, m), 7.57 (2H, m), 7.89 (1H, bs), 8.45 (1H, d, J = 8 Hz).

### Example 14

### N-(3-(1-(2-(2-Methylquinolin-5-yloxy)ethyl)piperidin-4-ylmethyl)phenyl)ethanesulfonamide (E14)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 468 (MH⁺). C₂₆H₃₃N₃O₃S requires 467.
¹H NMR (CDCl₃) δ: 1.36 (3H, m), 1.45 (2H, m), 1.61 (1H, m), 1.68 (2H, m), 2.27 (2H, m), 2.54 (2H, m), 2.72 (3H, s), 3.04 (2H, m), 3.12 (4H, m), 4.33 (2H, m), 6.80 (1H, d, J = 8 Hz), 6.93 (1 H, m), 7.04 (2H, m), 7.24 (2H, m), 7.57 (2H, m), 8.41 (1 H, d, J = 8 Hz).

### Example 15

### N-(3-(1-(2-(2-Methylquinolin-5-yloxy)ethyl)piperidin-4-ylmethyl)phenyl)trifluoromethanesulfonamide (E15)

The **title compound** was prepared from 3-(1-(2-(2-methylquinolin-5-yloxy)ethyl)piperidin-4-ylmethyl)aniline dihydrochloride following a method analogous to that of Example 3.
Mass spectrum (API⁺): Found 508 (MH⁺). C₂₅H₂₈F₃N₃O₃S requires 507.
¹H NMR (DMSO-d⁶) δ: 1.24 (2H, m), 1.76 (3H, m), 2.41 (2H, m), 2.64 (3H, s), 3.17 (2H, d, J = 5 Hz), 3.60 (2H, m), 4.08 (2H, m), 4.46 (2H, m), 6.54 (1H, m), 6.78 (2H, m), 6.95 (1H, m), 7.02 (2H, m), 7.41 (1H, m), 7.52 (1H, m), 7.62 ( H, m), 8.48 (1 H, m).

### Example 16

### N-(3-(1-(2-(2-Methylquinolin-5-yloxy)ethyl)piperidin-4-ylidenemethyl)phenyl)isobutyramide (E16)

The **title compound** was prepared from 3-(1-(2-(2-methylquinolin-5-yloxy)ethyl)piperidin-4-yldienemethyl)aniline dihydrochloride following a method analogous to that of Example 3.
Mass spectrum (API⁺): Found 444 (MH⁺). C₂₈H₃₃N₃O₂ requires 443.
¹H NMR (CDCl₃) δ: 1.25 (6H, d, J = 7 Hz), 2.42 (2H, m), 2.50 (1H, m), 2.56 (2H, m), 2.63 (2H, m), 2.73 (5H, m), 2.97 (2H, m), 4.29 (2H, m), 6.26 (1H, s), 6.81 (1H, d, J = 7 Hz), 6.94 (1H, m), 7.25 (3H, m), 7.33 (1H, m), 7.44 (1 H, bs), 7.57 (2H, m), 8.44 (1H, d, J = 9 Hz).

### Example 17

### N-(3-(1-(2-(2-Methylquinolin-5-yloxy)ethyl)piperidin-4-ylidenemethyl)phenyl)methanesulfonamide (E17)

The **title compound** was prepared from 3-(1-(2-(2-methylquinolin-5-yloxy)ethyl)piperidin-4-yldienemethyl)aniline dihydrochloride following a method analogous to that of Example 3.
Mass spectrum (API⁺): Found 452 (MH⁺). C₂₅H₂₉N₃O₃S requires 451.
¹H NMR (CDCl₃) δ: 2.43 (2H, m), 2.53 (2H, m), 2.64 (2H, m), 2.73 (3H, s), 2.74 (2H, m), 2.98 (2H, m), 3.01 (3H, s), 4.30 (2H, m), 6.25 (1H, s), 6.81 (1 H, d, J = 7 Hz), 7.04 (3H, m), 7.27 (2H, m), 7.57 (2H, m), 8.44 (1 H, d, J = 9 Hz).

### Example 18

### 1-Ethyl-3-(3-(1-(2-(2-methylquinolin-5-yloxy)ethyl)piperidin-4-ylidenemethyl)phenyl)urea (E18)

The **title compound** was prepared from 3-(1-(2-(2-methylquinolin-5-yloxy)ethyl)piperidin-4-yldienemethyl)aniline dihydrochloride following a method analogous to that of Example 3.
Mass spectrum (API⁺): Found 445 (MH⁺). C₂₇H₃₂N₄O₂ requires 444.
¹H NMR (CDCl₃) δ: 1.12 (3H, m), 2.40 (2H, m), 2.53 (2H, m), 2.58 (2H, m), 2.72 (5H, m), 2.96 (2H, m), 3.20 (2H, m), 4.28 (2H, m), 5.03 (1 H, m), 6.22 (1 H, s), 6.85 (3H, m), 7.13 (2H, m), 7.23 (2H, m), 7.57 (2H, m), 8.44 (1 H, d, J = 8 Hz).

### Example 19

### 1-(3-(1-(2-(2-Methylquinolin-5-yloxy)ethyl)piperidin-4-ylmethyl)imidazolidin-2-one (E19)

The **title compound** was prepared from 3-(1-(2-(2-(methylquinolin-5-yloxy)ethyl)piperidin-4-ylmethyl)aniline dihydrochloride following a method analogous to that of Example 5.
Mass spectrum (API⁺): Found 445 (MH⁺). C₂₇H₃₂N₄O₂ requires 444.
¹H NMR (CDCl₃) δ: 1.33 (2H, m), 1.56 (1H, m), 1.67 (2H, m), 2.14 (2H, m), 2.55 (2H, m), 2.72 (3H, s), 2.92 (2H, m), 3.03 (2H, m), 3.56 (2H, m), 3.94 (2H, m), 4.27 (2H, m), 4.62 (1H, bs), 6.79 (1H, d, J = 8 Hz), 6.85 (1H, m), 7.24 (2H, m), 7.31 (1H, m), 7.41 (1H, m), 7.56 (2H, m), 8.43 (1H, d, J = 8 Hz).

### Example 20

### N-(3-(1-(2-(2-Methylquinolin-5-yloxy)ethyl)piperidin-4-ylmethyl)phenyl)thiophene-2-carboxamide (E20)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 486 (MH⁺). C₂₉H₃₁N₃O₂S requires 485.
¹H NMR (CDCl₃) δ: 1.35 (2H, m), 1.56 (1H, m), 1.67 (2H, m), 2.14 (2H, m), 2.54 (2H, d, J = 7 Hz), 2.72 (3H, s), 2.92 (2H, m), 3.02 (2H, m), 4.27 (2H, m), 6.79 (1H, d, J = 7 Hz), 6.93 (1H, d, J = 8 Hz), 7.11 (1H, m), 7.25 (2H, m), 7.38 (1H, m), 7.48 (1H, m), 7.58 (4H, m), 7.77 (1H, bs), 8.43 (1H, d, J = 9 Hz).

### Example 21

### N-(3-(1-(2-(2-Methylquinolin-5-yloxy)ethyl)piperidin-4-ylmethyl)phenyl)furan-2-carboxamide (E21)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 470 (MH⁺). C₂₉H₃₁N₃O₃ requires 469.
¹H NMR (CDCl₃) δ: 1.36 (2H, m), 1.57 (1H, m), 1.68 (2H, m), 2.14 (2H, m), 2.55 (2H, d, J = 7 Hz), 2.72 (3H, s), 2.92 (2H, m), 3.03 (2H, m), 4.27 (2H, m), 6.55 (1H, m), 6.79 (1H, d, J = 7 Hz), 6.93 (1H, d, J = 8 Hz), 7.25 (3H, m), 7.44 (1H, m), 7.50 (1H, m), 7.56 (3H, m), 8.06 (1H, bs), 8.43 (1H, d, J = 8 Hz).

### Example 22

### N-(3-(1-(2-(2-Methylquinolin-5-yloxy)ethyl)piperidin-4-ylmethyl)phenyl)pyridine-3-carboxamide (E22)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 481 (MH⁺). C₃₀H₃₂N₄O₂ requires 480.
¹H NMR (CDCl₃) δ: 1.36 (2H, m), 1.55 (1H, m), 1.67 (2H, m), 2.14 (2H, m), 2.55 (2H, d, J = 7 Hz), 2.71 (3H, s), 2.93 (2H, m), 3.04 (2H, m), 4.27 (2H, m), 6.78 (1H, dd, J = 7, 2 Hz), 6.96 (1H, d, J = 8 Hz), 7.26 (2H, m), 7.41 (2H, m), 7.48 (1 H, m), 7.57 (2H, m), 8.19 (2H, m), 8.43 (1H, d, J = 9 Hz), 8.75 (1H, dd, J = 5, 2 Hz), 9.09 (1H, d, J = 2 Hz).

### Example 23

### N-(3-(1-(2-(2-Methylquinolin-5-yloxy)ethyl)piperidin-4-ylmethyl)phenyl)isoxazole-5-carboxamide (E23)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 471 (MH⁺). C₂₈H₃₀N₄O₃ requires 470.
¹H NMR (CDCl₃) δ: 1.36 (2H, m), 1.57 (1H, m), 1.68 (2H, m), 2.15 (2H, m), 2.57 (2H, d, J = 7 Hz), 2.72 (3H, s), 2.93 (2H, m), 3.04 (2H, m), 4.27 (2H, m), 6.79 (1 H, m), 7.02 (2H, m), 7.28 (2H, m), 7.45 - 7.61 (4H, m), 8.22 (1H, bs), 8.42 (2H, m).

### Example 24

### N-(3-(1-(2-(2-Methylquinolin-5-yloxy)ethyl)piperidin-4-ylmethyl)phenyl)pyridine-4-carboxamide (E24)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 481 (MH⁺). C₃₀H₃₂N₄O₂ requires 480.
¹H NMR (CDCl₃) δ: 1.39 (2H, m), 1.57 (1H, m), 1.68 (2H, m), 2.18 (2H, m), 2.57 (2H, d, J = 7 Hz), 2.72 (3H, s), 2.95 (2H, m), 3.06 (2H, m), 4.28 (2H, m), 6.79 (1H, dd, J = 7, 2 Hz), 6.98 (1H, d, J = 8 Hz), 7.27 (2H, m), 7.46 (2H, m), 7.57 (2H, m), 7.70 (2H, m), 7.95 (1H, bs), 8.42 (1H, d, J = 9 Hz), 8.79 (2H, m).

### Example 25

### N-(3-(1-(2-(2-Methylquinolin-5-yloxy)ethyl)piperidin-4-ylmethyl)phenyl)propane-2-sulfonamide (E25)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 482 (MH⁺). C₂₇H₃₅N₃O₃S requires 481.
¹H NMR (CDCl₃) δ: 1.40 (8H, m), 1.55 (1H, m), 1.65 (2H, m), 2.19 (2H, m), 2.53 (3H, m), 2.72 (3H, s), 2.97 (2H, m), 3.08 (2H, m), 4.29 (2H, m), 6.77 - 6.93 (3H, m), 7.03 (1H, m), 7.22 (3H, m), 7.56 (2H, m), 8.42 (1H, d, J = 9 Hz).

### Example 26

### N-Methyl-N-(3-(1-(2-(2-methylquinolin-5-yloxy)ethyl)piperidin-4-ylidenemethyl)phenyl)methanesulfonamide (E26)

The **title compound** was prepared from *N*-(3-(1-(2-(2-methylquinolin-5-yloxy)ethyl)piperidin-4-ylidenemethyl)phenyl)methanesulfonamide following a method analogous to that of Example 4.
Mass spectrum (API⁺): Found 466 (MH⁺). C₂₆H₃₁N₃O₃S requires 465.
¹H NMR (CDCl₃) δ: 2.44 (2H, m), 2.55 (2H, m), 2.65 (2H, m), 2.75 (5H, m), 2.84 (3H, s), 2.99 (2H, m), 3.32 (3H, s), 4.31 (2H, m), 6.28 (1H, s), 6.81 (1 H, m), 7.13 (1H, d, J = 8 Hz), 7.21 (1H, m), 7.26 (2H, m), 7.33 (1H, t, J = 8 Hz), 7.57 (2H, m), 8.45 (1H, d, J = 9 Hz)

### Example 27

### 5-(2-(4-(3-(1,1 -Dioxo-1-isothiazolidin-2-yl)benzyl)piperidin-1-yl)ethoxy)-2-methylquinoline (E27)

The **title compound** was prepared from 3-(1-(2-(2-(methylquinolin-5-yloxy)ethyl)piperidin-4-ylmethyl)aniline dihydrochloride following a method analogous to that of Example 5.
Mass spectrum (API⁺): Found 480 (MH⁺). C₂₇H₃₃N₃O₃S requires 479.
¹H NMR (CDCl₃) δ: 1.36 (2H, m), .1.56 (1H, m), 1.67 (2H, m), 2.16 (2H, m), 2.52 (4H, m), 2.72 (3H, s), 2.95 (2H, m), 3.06 (2H, m), 3.70 (2H, t, J = 8 Hz), 3.77 (2H, t, J = 7 Hz), 4.28 (2H, m),6.79 (1H, d, J = 7 Hz), 6.93 (1H, m), 7.06 (2H, m), 7.25 (2H, m), 7.56 (2H, m), 8.42 (1H, d, J = 9 Hz).

### Example 28

### N-(3-(1-(2-(2-Methylquinolin-5-yloxy)ethyl)piperidin-4-ylmethyl)phenyl)propane-1-sulfonamide (E28)

The **title compound** was prepared from 3-(1-(2-(2-(methylquinolin-5-yloxy)ethyl)piperidin-4-ylmethyl)aniline dihydrochloride following a method analogous to that of Example 3.
Mass spectrum (API⁺): Found 482 (MH⁺). C₂₇H₃₅N₃O₃S requires 481.
¹H NMR (CDCl₃) δ: 1.01 (3H, m), 1.33 (2H, m), 1.55 (1H, m), 1.64 (2H, m), 1.85 (2H, m), 2.14 (2H, m), 2.53 (2H, d, J = 7 Hz), 2.72 (3H, s), 2.93 (2H, m), 3.05 (4H, m), 4.27 (2H, m), 6.79 (1H, d, J = 8 Hz), 6.94 (1H, d, J = 8 Hz), 6.99 (1H, s), 7.02 (1H, m), 7.24 (2H, m), 7.56 (2H, m), 8.42 (1H, d, J = 8 Hz).

### Example 29

### N-(3-(1-(2-(2-Methylquinolin-5-yloxy)ethyl)piperidin-4-ylmethyl)phenyl)methylamine (E29)

A solution of *tert*-butyl (3-(1-(2-(2-methylquinolin-5-yloxy)ethyl)piperidin-4-ylmethyl)phenyl)carbamate (0.22 g, 0.46 mmol) in dry tetrahydrofuran (10 mL) was cooled to <0°C (ice/methanol). A 1.0M solution of lithium aluminium hydride in diethyl ether (1 mL, 1 mmol) was added dropwise with stirring and under argon. The mixture was allowed to warm up to 20°C and then stirred at reflux for 24h. The reaction mixture was cooled and treated with 1 N aqueous sodium hydroxide (2 mL), then partitioned between ethyl acetate (15 mL) and water (2 x 10 mL). The organic layer was dried (Na₂SO₄) and evaporated *in vacuo.* Chromatography of the residue on SiO₂ eluting from 0-10% methanol in ethyl acetate gave the **title compound** (0.12 g, 67%) as an oil.
Mass spectrum (API⁺): Found 390 (MH⁺). C₂₅H₃₁N₃O requires 389.
¹H NMR (CDCl₃) δ: 1.33 (2H, m), 1.54 (1H, m), 1.69 (2H, m), 2.13 (2H, m), 2.46 (2H, d, J = 7 Hz), 2.72 (3H, s), 2.82 (3H, s), 2.92 (2H, m), 3.03 (2H, m), 4.27 (2H, m), 6.39 (1 H, d, J = 2 Hz), 6.45 (1 H, dd, J = 7, 2 Hz), 6.51 (1 H, d, J = 7 Hz), 6.79 (1H, dd, J = 8, 1 Hz), 7.09 (1H, t, J = 8 Hz), 7.23 (1H, d, J = 9 Hz), 7.56 (2H, m), 8.43 (1H, d, J = 9 Hz).

### Example 30

### N-Methyl-N-(3-(1-(2-(2-methylquinolin-5-yloxy)ethyl)piperidin-4-ylmethyl)phenyl)propane-2-sulfonamide (E30)

A mixture of *N*-(3-(1-(2-(2-methylquinolin-5-yloxy)ethyl)piperidin-4-ylmethyl)phenyl)methylamine (0.092 g, 0.24 mmol), *iso*-propylsulfonyl chloride (0.27 g, 1.92 mmol) in pyridine (3 mL) was stirred at 85 °C for 5 h. Reaction mix was cooled and partitioned between dichloromethane (5 mL) and water (5 mL) and the organic layer was evaporated *in vacuo.* Chromatography of the residue on SiO₂ eluting from 0-15% methanol in ethyl acetate gave the **title compound** (0.037 g, 31%) as an oil.
Mass spectrum (API⁺): Found 496 (MH⁺). C₂₈H₃₇N₃O₃S requires 495.
¹H NMR (CDCl₃) δ: 1.38 (8H, m), 1.57 (1 H, m), 1.68 (2H, m), 2.22 (2H, m), 2.56 (2H, d, J = 7 Hz), 2.73 (3H, s), 3.00 (2H, m), 3.12 (2H, m), 3.28 (1H, m), 3.36 (3H, s), 4.31 (2H, m), 6.80 (1H, d, J = 8 Hz), 7.03 (1H, d, J = 7 Hz), 7.24 (4H, m), 7.57 (2H, m), 8.42 (1H, d, J = 9 Hz).

### Example 31

### N-(2-Fluoro-5-(1-(2-(2-methylquinolin-5-yloxy)ethyl)piperidin-4-ylmethyl)phenyl)methanesulfonamide (E31)

The title compound was prepared using an analogous route and intermediates to those used to prepare Example 3.
Mass spectrum (API⁺): Found 472 (MH⁺). C₂₅H₃₀FN₃O₃S requires 471.
¹H NMR (CDCl₃) δ: 1.36 (2H, m), 1.59 (1H, m), 1.64 (2H, m), 2.15 (2H, m), 2.57 (2H, d, J = 7 Hz), 2.72 (3H, s), 2.92 (2H, m), 2.97 (3H, s), 3.03 (2H, m), 4.26 (2H, m), 6.79 (1 H, dd, J = 7, 1 Hz), 7.02 (3H, m), 7.24 (1 H, d, J = 9 Hz), 7.56 (2H, m), 8.43 (1 H, d, J = 9 Hz).

### Example 32

### 5-{2-[4-(2-Fluoro-5-nitrobenzylidene)piperidin-1-yl]ethoxy}-2-methylquinoline (E32)

The **title compound** was prepared using an analogous route and intermediates to those used to prepare Example 1.
Mass spectrum (API⁺): Found 422 (MH⁺). C₂₄H₂₄FN₃O₃ requires 421.
¹H NMR (CDCl₃) δ: 2.44-2.51 (4H, m), 2.68 (2H, t J = 6Hz), 2.72 (3H, s), 2.78 (2H, t, J = 6Hz), 3.00 (2H, t, J = 6Hz), 4.30 (2H, t, J = 6Hz), 6.19 (1 H, s), 6.80 (1 H, d, J = 9Hz), 7.15-7.20 (1 H, m), 7.25-7.27 (1 H, m), 7.54-7.62 (2H, m), 8.08-8.11 (2H, m), 8.43 (1 H, d, J = 9Hz).

### Example 33

### N-(4-Fluoro-3-{1-[2-(2-methylquinolin-5-yloxy)ethyl]piperidin-4-ylmethyl}phenyl)methanesulfonamide (E33)

The title compound was prepared from 5-{2-[4-(2-fluoro-5-nitrobenzylidene)piperidin-1-yl]ethoxy}-2-methylquinoline following method analogous to that of Example 3.
Mass spectrum (API⁺): Found 472 (MH⁺). C₂₅H₃₀FN₃O₃S requires 471.
¹H NMR (CDCl₃) δ: 1.26-1.38 (2H, m), 1.56-1.66 (3H, m), 2.12-2.18 (2H, m), 2.57 (2H, d, J = 7Hz), 2.73 (3H, s), 2.93 (2H, t, J = 6Hz), 3.02 (3H, s), 3.05-3.29 (2H, m), 4.27 (2H, t, J = 6Hz), 6.78-6.80 (1H, m), 7.00-7.06 (2H, m), 7.23-7.26 (3H, m), 7.53-7.61 (2H), 8.42 (1H, d, J = 9Hz).

### Example 34

### N-(4-Methyl-3-{1-[2-(2-methylquinolin-5-yloxy)ethyl]piperidin-4-ylidenemethyl}phenyl)methanesulfonamide (E34)

The **title compound** was prepared from 4-methyl-3-{1-[2-(2-methylquinolin-5-yloxy)ethyl]piperidin-4-ylidenemethyl}aniline using analogous methods to Example 3.
Mass spectrum (API⁺): Found 466 (MH⁺). C₂₆H₃₁N₃O₃S requires 465.
¹H NMR (400 MHz, CDCl₃) δ: 2.22 (3H, s), 2.35 (2H, t), 2.44 (2H, t), 2.62 (2H,t), 2.73 (3H, s), 2.75 (2H, t), 2.97 (3H, s), 2.98 (2H, t), 4.29 (2H,t), 6.19 (1H, s), 6.35 (1H, br, s), 6.81 (1H, d), 6.99 (2H, d), 7.14 (1H, d), 7.26 (1H, d), 7.53-7.61 (2H, m), 8.44 (1H, d).

### Example 35

### N-(4-Methoxy-3-{1-[2-(2-methylquinolin-5-yloxy)ethyl]piperidin-4-ylidenemethyl}phenyl)methanesulfonamide (E35)

The title compound was prepared from 4-methoxy-3-{1-[2-(2-methylquinolin-5-yloxy)ethyl]piperidin-4-ylidenemethyl}aniline using analogous methods to Example 3.
Mass spectrum (API⁺) : Found 482 (MH⁺). C₂₆H₃₁N₃O₄S requires 481.
¹H NMR (400 MHz, CDCl₃) δ: 2.46 (4H, br m), 2.67 (2H, t), 2.73 (3H, s), 2.76 (2H, t), 2.94 (3H, s), 2.99 (2H, t), 3.82 (3H, s), 4.30 (2H, t), 6.25 (1H, s), 6.82 (2H, m), 7.04-7.10 (2H, m), 7.26 (1H, d), 7.59-7.67 (1 H, m), 7.55 (1 H, m), 8.45 (1 H, d). NH not discernible.

### Example 36

### N-(3-{1-[2-(2-Methylquinolin-5-yloxy)ethyl]piperidin-4-ylidenemethyl}-5-trifluoromethylphenyl)methanesulfonamide (E36)

The **title compound** was prepared from 3-{1-[2-(2-methylquinolin-5-yloxy)ethyl]piperidin-4-ylidenemethyl}-5-trifluoromethylaniline using methods analogous to Example 3.
Mass spectrum(API⁺) : Found 520 (MH⁺). C₂₆H₂₈F₃N₃O₃S requires 519.
¹H NMR (400 MHz, CDCl₃) δ: 2.44 (2H, t), 2.51 (2H, t), 2.66 (2H, t), 2.73 (3H, s), 2.76 (2H, t), 2.99 (2H, t), 3.05 (3H, s), 4.30 (2H, t), 6.26 (1H, s), 6.81 (1H, d), 7.26 (4H, m), 7.53-7.61 (2H, m), 8.44 (1H, d). NH not discernible.

### Example 37

### N-(2-Methyl-3-{1-[2-(2-methylquinolin-5-yloxy)ethyl]piperidin-4-ylidenemethyl}phenyl)methanesulfonamide (E37)

The title compound was prepared from 2-methyl-3-{1-[2-(2-methylquinolin-5-yloxy)ethyl]piperidin-4-ylidenemethyl}aniline using methods analogous to Example 3.
Mass spectrum (API⁺) : Found 466 (MH⁺). C₂₆H₃₁N₃O₃S requires 465.
¹H NMR (400 MHz, CDCl₃) δ 2.22 (3H, s), 2.29 (2H, t), 2.42 (2H, t), 2.59 (2H, t), 2.72 (3H, s), 2.74 (2H, t), 2.98 (2H, t), 3.01 (3H, s), 4.29 (2H, t), 6.22 (1 H, s), 6.80 (1 H, d), 6.99 (1 H, d), 7.14 (1H, t), 7.23 (1H, s), 7.32 (1H, d), 7.53-7.61 (2H, m), 8.43 (1H, d). NH not discernible.

### Example 38

### N-(2-Chloro-3-{1-[2-(2-methylquinolin-5-yloxy)ethyl]piperidin-4-ylidenemethyl}phenyl)methanesulfonamide (E38)

The **title compound** was prepared from 2-chloro-3-{1-[2-(2-methylquinolin-5-yloxy)ethyl]piperidin-4-ylidenemethyl}aniline using methods analgous to Example 3.
Mass spectrum (API⁺): Found 486 (MH⁺). C₂₅H₂₈³⁵ClN₃O₃S requires 485.
¹H NMR (400 MHz, CDCl₃) δ: 2.40 (2H, t), 2.48 (2H,t), 2.64 (2H, d), 2.73 (3H, s), 2.78 (2H, t), 3.00 (2H,t), 3.03 (3H, s), 4.30 (2H,t), 6.26 (1H, s), 6.80 (1H, d), 6.87 (1H, br, s), 7.06 (1H, d), 7.25 (2H, m), 7.53-7.62 (3H, m), 8.44(1H,d).

### Example 39

### N-(2-Chloro-5-{1-[2-(2-methylquinolin-5-yloxy)ethyl]piperidin-4-ylidenemethyl}phenyl)methanesulfonamide (E39)

The title compound was prepared from 2-chloro-5-{1-[2-(2-methylquinolin-5-yloxy)ethyl]piperidin-4-ylidenemethyl}aniline using methods analogous to Example 3.
Mass spectrum (API⁺): Found 486 (MH⁺). C₂₅H₂₈³⁵ClN₃O₃S requires 485.
¹H NMR (400 MHz, CDCl₃) δ 2.43 (2H, t), 2.53 (2H, t), 2.66 (3H, t), 2.73 (3H, s), 2.75 (2H, t), 2.99 (2H, t), 3.00 (3H, s), 4.30 (1H, t), 6.81 (1H, d), 6.97 (1H, d) 7.23 (1H, s), 7.26 (1H, d), 7.34 (1H, d), 7.48 (1H, m), 7.49-7.59 (1H, m), 7.61-7.67 (1H, m), 8.46 (1H, d). NH not discernible.

### Example 40

### N-(2-isopropyl-5-{1-[2-(2-methylquinolin-5-yloxy)ethyl]piperldin-4-ylidenemethyl}phenyl)methanesulfonamide (E40)

The **title compound** was prepared from 2-isopropyl-5-{1-[2-(2-methylquinolin-5-yloxy)ethyl]piperidin-4-ylidenemethyl}aniline using methods analogous to Example 3.
Mass spectrum (API⁺): Found 494 (MH⁺). C₂₈H₃₅N₃O₃S requires 493.
¹H NMR (400 MHz, CDCl₃) δ: 1.25 (6H, d), 2.42 (2H, t), 2.56 (2H, t), 2.65 (2H, t), 2.73 (3H, s), 2.75 (2H, t), 2.97 (2H, t), 3.02 (3H, s), 3.08-3.14 (1H, m), 4.30 (2H, d), 6.20 (1H, s), 6.24 (1H, s), 6.81 (1H, d), 7.09 (1H, d), 7.25-7.29 (3H, m), 7.54-7.61 (2H, m), 8.45 (1H, d).

### Example 41

### N-(5-{1-[2-(2-Methylquinolin-5-yloxy)ethyl]piperidin-4-ylldenemethyl}-2-trifluoromethylphenyl)methanesulfonamide (E41)

The title compound was prepared from 5-{1-[2-(2-methylquinolin-5-yloxy)ethyl]piperidin-4-ylidenemethyl}-2-trifluoromethylaniline using methods analogous to Example 3.
Mass spectrum (API⁺): Found 520 (MH⁺). C₂₆H₂₈N₃O₃SF₃ requires 519.
¹H NMR (400 MHz, CDCl₃) δ: 2.45 (2H, t), 2.56 (2H, t), 2.68 (2H, t), 2.73 (3H, s), 2.75 (2H, t), 2.99 (2H, t), 3.00 (3H, s), 4.30 (2H, t), 6.28 (1 H, s), 6.80 (1 H, d), 7.12 (1 H, d), 7.27 (1 H, d), 7.53-7.62 (3H, m), 7.65 (1 H, s), 8.44 (1 H, d). NH not discernible.

### Example 42

### N-(5-{1-[2-(2-Methylquinolin-5-yloxy)ethyl]piperidin-4-ylmethyl}-2-trifluoromethylphenyl)methanesulfonamide (E42)

The **title compound** was prepared from *N*-(5-{1-[2-(2-methylquinolin-5-yloxy)ethyl]piperidin-4-ylidenemethyl}-2-trifluoromethylphenyl)methanesulfonamide using methods analogous to Example 2.
Mass spectrum (API⁺): Found 522 (MH⁺). C₂₆H₃₀ F₃N₃O₃S requires 521
¹H NMR (400 MHz, CDCl₃) δ: 1.34-1.40 (3H, br m), 1.59-1.66 (2H, t), 2.15 (2H, t), 2.61 (2H, d), 2.73 (3H, s), 2.93 (2H, t), 2.99 (3H, s), 3.03 (2H, t), 4.26 (2H, t), 6.79 (1 H, d), 7.08 (1 H, d), 7.24 (2H, d), 7.53-7.61 (3H, m), 8.42 (1 H, d). NH not discernible.

### Example 43

### N-(4-Methyl-3-{1-[2-(2-methylquinolin-5-yloxy)ethyl]piperidin-4-ylmethyl}phenyl)methanesulfonamide (E43)

The **title compound** was prepared from *N*-(4-methyl-3-{1-[2-(2-methylquinolin-5-yloxy)ethyl]piperidin-4-ylidenemethyl}phenyl)methanesulfonamide using methods analogous to Example 2.
Mass spectrum (API⁺): Found 468 (MH⁺). C₂₆H₃₃N₃O₃S requires 467.
¹H NMR (400 MHz, CDCl₃) δ: 1.36-1.43 (2H, br t), 1.51-1.55 (1H, br m), 1.65 (2H, t), 2.11-2.17 (2H, br, t), 2.23 (3H, s), 2.53 (2H, d), 2.73 (3H, s), 2.93 (2H, t), 2.97 (3H, s), 3.04 (2H, t), 4.27(2H, t), 6.79 (1H, d), 6.96 (1H, d), 7.11 (1H, d), 7.25 (2H, d), 7.53-7.61 (2H, m), 8.43 (1H, d). NH not observed.

### Example 44

### N-(2-Isopropyl-5-{1-[2-(2-methylquinolin-5-yloxy)ethyl]piperidin-4-ylmethyl}phenyl)methanesulfonamide (E44)

The **title compound** was prepared from *N*-(2-isopropyl-5-{1-[2-(2-methylquinolin-5-yloxy)ethyl]piperidin-4-ylidenemethyl}phenyl)methanesulfonamide **using methods analogous** to Example 2.
Mass spectrum (API⁺): Found 496 (MH⁺). C₂₈H₃₇N₃O₃S requires 495.
¹HNMR (400 MHz, CDCl₃) δ: 1.24 (6H, d), 1.37 (2H, t), 1.53-1.68 (3H, m), 2.16 (2H, t), 2.52 (2H, d), 2.73 (3H, s), 2.93 (2H, t), 3.00 (3H, s), 3.03-3.08 (2H, t), 3.11 (1H, m), 4.27 (2H, t), 6.17 (1H, br, s), 6.79 (1H, d), 7.03 (1H, d), 7.23 (3H, m), 7.53-7.60 (2H, m), 8.43 (1 H, m).

### Example 45

### N-((3-(1-(2-(2-Methylquinolin-5-yloxy)ethyl)piperidin-4-ylmethyl)phenyl)-2-dimethylamino)acetamide (E45)

A mixture of 3-(1-(2-(2-methylquinolin-5-yloxy)ethyl)piperidin-4-ylmethyl)aniline dihydrochloride (0.072 g, 0.16 mmol) and triethylamine (0.081 g, 0.8 mmol) in dichloromethane (5 mL) was treated with dimethylaminoacetyl chloride hydrochloride (0.025 g, 0.16 mmol) and the mixture stirred at 20 °C for 2 h. Reaction mixture was washed with water (5 mL) and the organic layer added to SiO₂. Eluting with 0-10% methanol in ethyl acetate gave the title compound (0.041 g, 56%) as an oil.
Mass spectrum (API⁺): Found 461 (MH⁺). C₂₈H₃₆N₄O₂ requires 460.
¹H NMR (CDCl₃) δ: 1.36 (2H, m), 1.57 (1H, m), 1.68 (2H, m), 2.17 (2H, m), 2.38 (6H, s), 2.54 (2H, d, J = 7 Hz), 2.72 (3H, s), 2.95 (2H, m), 3.07 (4H, m), 4.29 (2H, m), 6.79 (1 H, d, J = 7 Hz), 6.89 (1 H, d, J = 8 Hz), 7.24 (2H, m), 7.40 (2H, m), 7.56 (2H, m), 8.42 (1 H, d, J = 9 Hz), 9.04 (1 H, bs).

### Example 46

### N-((3-(1-(2-(2-Methylquinolin-5-yloxy)ethyl)piperidin-4-ylmethyl)phenyl)-2-diethylamino)acetamide (E46)

A mixture of 2-chloro-*N*-(3-(1-(2-(2-methylquinolin-5-yloxy)ethyl)piperidin-4-ylmethyl)phenyl)acetamide (0.055 g, 0.00012 mol), diethylamine (0.01 g, 0.00013 mol), sodium iodide (0.018 g, 0.00012 mol) and *N,N*-diisopropylethylamine (0.048 g, 0.00037 mol) in isopropanol (5 mL) was stirred at reflux for 6h. The reaction mixture was evaporated *in vacuo,* and the residue partitioned between dichloromethane (20 mL) and water (3 x 20 mL). The organic layer was separated and added to SiO₂, and eluted from 0-15% methanol in ethyl acetate to give the **title compound** (0.013 g, 22%) as an oil.
Mass spectrum (API⁺): Found 489 (MH⁺). C₃₀H₄₀N₄O₂ requires 488.
¹H NMR (CDCl₃) δ: 1.09 (6H, t, J = 7 Hz), 1.58 (2H, m), 1.67 (1H, m), 1.74 (2H, m), 2.36 (2H, m), 2.56 (2H, d, J = 7 Hz), 2.65 (4H, q, J = 7 Hz), 2.73 (3H, s), 3.10 (2H, m), 3.14 (2H, s), 3.23 (2H, m), 4.41 (2H, m), 6.81 (1 H, d, J = 7 Hz), 6.88 (1 H, d, J = 8 Hz), 7.23 (2H, m), 7.33 (1 H, m), 7.46 (1 H, m), 7.55 (1 H, m), 7.61 (1 H, m), 8.40 (1 H, d, J = 8 Hz), 9.36 (1 H, bs).

### Example 47

### N-(3-(1-(2-(2-Methylquinolin-5-yloxy)ethyl)piperidin-4-ylmethyl)phenyl)-2-pyrrolidin-1-ylacetamide (E47)

The **title compound** was prepared using methods analogous to Example 46.
Mass spectrum (API⁺): Found 487 (MH⁺). C₃₀H₃₈N₄O₂ requires 486.
¹H NMR (CDCl₃) δ: 1.54 (2H, m), 1.65 (1H, m), 1.73 (2H, m), 1.86 (4H, m), 2.34 (2H, m), 2.55 (2H, d, J = 7 Hz), 2.71 (7H, m), 3.08 (2H, m), 3.20 (2H, m), 3.28 (2H, s), 4.39 (2H, m), 6.80 (1H, d, J = 8 Hz), 6.88 (1 H, d, J = 8 Hz), 7.24 (2H, m), 7.36 (1 H, m), 7.44 (1 H, m), 7.55 (1 H, m), 7.61 (1 H, m), 8.40 (1 H, d, J = 8 Hz), 9.07 (1 H, bs).

### Example 48

### 3-(1-(2-(2-Methylquinazolin-5-yloxy)ethyl)piperidin-4-ylidenemethyl)nitrobenzene (E48)

A mixture of 5-(2-methanesulfonyloxyethyl)-2-methylquinazoline (0.28 g, 1.0 mmol), 4-(3-nitrobenzylidene)piperidine hydrochloride (0.25 g, 1.0 mmol), and potassium carbonate (0.42 g, 3.0 mmol) in *N,N*-dimethylformamide (7 mL) was stirred at 100 °C for 2 h. The reaction mixture was cooled, partitioned between water (4 x 10 mL) and dichloromethane (10 mL). The organic layer was added to SiO₂ and eluting from 0-5% methanol in ethyl acetate gave the **title compound** (0.17 g, 42%) as a brown solid.
Mass spectrum (API⁺): Found 405 (MH⁺). C₂₃H₂₄N₄O₃ requires 404.
¹H NMR (CDCl₃) δ: 2.47 (2H, m), 2.53 (2H, m), 2.66 (2H, m), 2.78 (2H, m), 2.88 (3H, m), 3.01 (2H, m), 4.34 (2H, m), 6.33 (1H, s), 6.87 (1 H, d, J = 8 Hz), 7.49 (3H, m), 7.75 (1 H, t, J = 8 Hz), 8.04 (2H, m), 9.64 (1 H, s).

### Example 49

### N-(3-(1-(2-(2-Methylquinazolin-5-yloxy)ethyl)piperidin-4-ylmethyl)phenyl)methanesulfonamide (E49)

The **title compound** was prepared using analogous routes and intermediates to those used to prepare Example 3.
Mass spectrum (API⁺): Found 455 (MH⁺). C₂₄H₃₀N₄O₃S requires 454.
¹H NMR (CDCl₃) δ: 1.33 (2H, m), 1.55 (1H, m), 1.64 (2H, m), 2.16 (2H, m), 2.54 (2H, d, J = 7 Hz), 2.88 (3H, s), 2.94 (2H, m), 3.02 (5H, m), 4.30 (2H, m), 6.85 (1H, d, J = 8 Hz), 6.97 (1H, m), 7.01 (1H, s), 7.05 (1H, m), 7.25 (1 H, m), 7.49 (1H, d, J = 8 Hz), 7.74 (1H, t, J = 8 Hz), 9.63 (1H, s).

### Example 50

### N-Methyl-N-(3-{4-[2-(2-methylquinolin-5-yloxy)ethyl]piperazin-1-ylmethyl}phenyl)acetamide (E50)

The title compound was prepared from *N*-(3-{4-[2-(2-methylquinolin-5-yloxy)ethyl]piperazin-1-ylmethyl}phenyl)acetamide in a similar manner to Example 4.
Mass spectrum (API⁺): Found 433 (MH⁺). C₂₆H₃₂N₄O₂ requires 432.

### Example 51

### N-Ethyl-N-(3-{4-[2-(2-methylquinolin-5-yloxy)ethyl]piperazin-1-ylmethyl}phenyl)acetamide (E51)

The **title compound** was prepared from *N*-(3-{4-[2-(2-methylquinolin-5-yloxy)ethyl]piperazin-1-ylmethyl}phenyl)acetamide in a similar manner to Example 4.
Mass spectrum (API⁺): Found 447 (MH⁺). C₂₇H₃₄N₄O₂ requires 446.
¹H NMR (CDCl₃) δ: 1.11 (3H, t *J* 7), 1.82 (3H, s), 2.47-2.51 (4H, br), 2.70 (4H, br s), 2.73 (3H, s), 2.97 (2H, t *J* 6), 3.55 (2H, s), 3.74 (2H, q *J* 7), 4.29 (2H, t *J* 6), 6.79 (1H, dd *J* 7 and 1), 7.02-7.06 (1H, m), 7.16 (1H, s), 7.23-7.39 (3H, m), 7.51-7.62 (2H, m), 8.42 (1H, d *J* 9).

### Example 52

### 5-{2-[4-(4-Methoxy-3-nitrobenzylidene)piperidin-1-yl]ethoxy}-2-methylquinoline (E52)

The **title compound** was prepared using an analogous route and intermediates to that used to prepare Example 1.
Mass spectrum (API⁺): Found 434 (MH⁺). C₂₅H₂₇N₃O₄ requires 433.
¹H NMR (CDCl₃) δ: 2.43 (2H, t *J* 5), 2.51 (2H, t *J* 5), 2.64 (2H, t *J* 5), 2.73-2.76 (5H, m), 2.99 (2H, t *J* 6), 3.95 (3H, s), 4.30 (2H, t *J* 6), 6.80 (1H, d *J* 1), 7.02 (1H, t *J* 9), 7.26 (1H, t *J* 2), 7.34 (1H, dd *J* 9 and 2), 7.54-7.62 (2H, m), 7.67 (2H, t *J* 2), 8.44 (1H, d *J* 9).

### Example 53

### 5-{2-[4-(3-Amino-4-methoxybenzyl)piperidin-1-yl]ethoxy}-2-methylquinoline (E53)

The **title compound** was prepared from 5-{2-[4-(4-methoxy-3-nitrobenzylidene)piperidin-1-yl]ethoxy}-2-methylquinoline following the method of Example 2.
Mass spectrum (API⁺): Found 406 (MH⁺). C₂₅H₃₁N₃O₂ requires 405.
¹H NMR (CDCl₃) δ: 1.25-1.35 (2H, m), 1.46-1.49 (1H, m), 1.65-1.68 (2H, m), 2.09-2.15 (2H, m), 2.39 (2H, d *J* 7), 2.72 (3H, s), 2.91 (2H, t *J* 6), 3.01-3.04 (2H, m), 3.81 (3H, s), 4.26 (2H, t *J* 6), 6.47-6.51 (2H, m), 6.68-6.70 (1H, m), 6.77-6.79 (1H, m), 7.22-7.26 (1H, m), 7.52-7.60 (2H, m), 8.42 (1H, d *J* 9).

### Example 54

### N-(2-Methoxy-5-{1-[2-(2-methylquinolin-5-yloxy)ethyl]piperidin-4-ylmethyl}phenyl)methanesulfonamide (E54)

The **title compound** was prepared from 5-{2-[4-(3-amino-4-methoxybenzyl)piperidin-1-yl]ethoxy}-2-methylquinoline following the method of Example 3.
Mass spectrum (API⁺): Found 484 (MH⁺). C₂₆H₃₃N₃O₄S requires 483.
¹H NMR (CDCl₃) δ: 1.26-1.37 (2H, m), 1.49-1.53 (1H, m), 1.62-1.66 (2H, m), 2.10-2.16 (2H, m), 2.48 (2H, d *J* 7), 2.72 (3H, s), 2.87-2.94 (5H, m), 3.01-3.03 (2H, m), 3.84 (3H, s), 4.26 (2H, t *J* 6), 6.80 (2H, t *J* 9), 6.89-6.91 (1H, m), 7.23-7.27 (2H, m), 7.32 (1 H, d *J* 2), 7.52-7.60 (2H), 8.43 (1H, d *J* 9).

### Example 55

### 2-Methyl-5-{2-[4-(3-nitrophenoxy)piperidin-1-yl]ethoxy}quinoline (E55)

The **title compound** was prepared from 5-(2-bromoethoxy)-2-methylquinoline and 4-(3-nitrophenoxy)piperidine using the method of Example 1.
Mass spectrum (API⁺): Found 408 (MH⁺). C₂₃H₂₅N₃O₄ requires 407.
¹H NMR (250MHz, CDCl₃) δ: 1.84-1.95 (2H, m), 2.02-2.10 (2H, m), 2.53-2.62 (2H, m), 2.73 (3H, s), 2.89-2.97 (2H, m), 3.00 (2H, t), 4.30 (2H, t), 4.40-4.50 (2H, m), 6.82 (1H, dd), 7.20-7.29 (2H, m), 7.41 (1H, t), 7.53-7.64 (2H, m), 7.74 (1H, m), 7.78-7.82 (1H. m), 8.45 (1 H, d).

### Example 56

### N-(3-{1-[2-(2-Methylquinolin-5-yloxy)ethyl]piperidin-4-yloxy}phenyl)methanesulphonamide (E56)

The **title compound** was prepared from 5-{2-[4-(3-aminophenoxy)piperidin-1-yl]ethoxy}-2-methylquinoline using the method of Example 3.
Mass spectrum (API⁺): Found 456 (MH⁺). C₂₄H₂₉N₃O₄S requires 455.
¹H NMR (250MHz, CDCl₃) δ: 1.80-1.90 (2H, m), 1.97-2.06 (2H, m), 2.49-2.58 (2H, m), 2.73 (3H, s), 2.86-2.95 (2H, m), 2.97 (2H, t), 3.01 (3H, s), 4.28 (2H, t), 4.30-4.39 (1 H, m), 6.71-6.77 (2H, m), 6.80-6.86 (2H, m), 7.19-7.28 (2H, m), 7.52-7.63 (2H, m), 8.45 (1 H, d).

### Example 57

### N-(2-Chloro-5-{1-[2-(2-methylquinolin-5-yloxy)ethyl]piperidin-4-yloxy}phenyl)methanesulphonamide (E57)

The title compound was prepared from 5-{2-[4-(3-amino-4-chlorophenoxy)piperidin-1-yl]ethoxy}-2-methylquinoline using the method of Example 3.
Mass spectrum (API⁺): Found 490 (MH⁺). C₂₄H₂₈³⁵ClN₃O₄S requires 489.
¹H NMR (250MHz, CDCl₃) δ: 1.80-1.91 (2H, m), 1.98-2.08 (2H,m), 2.51-2.60 (2H, m), 2.73 (3H, s), 2.86-2.97 (2H, m), 2.99 (2H, t), 3.00 (3H, s), 4.29 (2H, t), 4.30-4.39 (1H, m), 6.69 (1H, dd), 6.81 (1H, d), 7.20-7.30 (3H, m), 7.52-7.63 (2H, m), 8.45 (1H, d).

### Example 58

### 5-(2-(4-(3-(cis-3,5-Dimethylpiperazin-1-yl)benzylidene)piperidin-1-y)ethoxy)-2-methylquinoline (E58)

A mixture of cesium carbonate (0.28 g, 0.86 mmol), palladium (II) acetate (0.013 g, 0.057 mmol), and BINAP (0.053 g, 0.086 mmol) in 1,4-dioxane (5 mL) was sonicated at 20 °C under argon for 0.5 h. To this mixture was added 3-(1-(2-(2-methylquinolin-5-yloxy)ethyl)piperidin-4-ylidenemethyl)bromobenzene (0.25 g, 0.57 mmol) and cis-2,6-dimethylpiperazine (0.2 g, 1.75 mmol) and the mixture stirred at reflux for 72 h. The mixture was cooled to 20 °C and filtered through celite and the filtrate was evaporated *in vacuo*. Chromatography of the residue on SiO₂ eluting from 0-15% methanol in ethyl acetate and 2% .880 ammonia in 15% methanol in ethyl acetate gave the **title compound** (0.12 g, 45%) as an oil.
Mass spectrum (API⁺): Found 471 (MH⁺). C₃₀H₃₈N₄O requires 470.
¹H NMR (CDCl₃)δ: 1.13 (6H, m), 2.29 (2H, m), 2.42 (2H, m), 2.57 (2H, m), 2.62 (2H, m), 2.73 (5H, m), 2.88 - 3.06 (5H, m), 3.50 (2H, m), 4.30 (2H, m), 6.28 (1 H, s), 6.70 (1 H, d, J = 7 Hz), 6.78 (3H, m), 7.22 (2H, m), 7.57 (2H, m), 8.44 (1H, d, J = 9 Hz).

### Example 59

### 2-Methyl-5-(2-(4-(3-(4-methylpiperazin-1-yl)benzylidene)piperidin-1-yl)ethoxy)quinoline (E59)

The **title compound** was prepared in a similar manner to Example 58.
Mass spectrum (API⁺): Found 457 (MH⁺). C₂₉H₃₆N₄O requires 456.
¹H NMR (CDCl₃) δ: 2.35 (3H, s), 2.42 (2H, m), 2.57 (6H, m), 2.62 (2H, m), 2.73 (5H, m), 2.97 (2H, m), 3.20 (4H, m), 4.30 (2H, m), 6.27 (1H, s), 6.72 (1H, d, J = 8 Hz), 6.79 (3H, m), 7.22 (2H, m), 7.57 (2H, m), 8.44 (1H, d, J = 9 Hz).

### Example 60

### 5-(2-(4-(3-(cis-3,5-Dimethylpiperazin-1-yl)benzyl)piperidin-1-yl)ethoxy)-2-methylquinoline (E60)

A mixture of 5-(2-(4-(3-(*cis*-3,5-dimethylpiperazin-1-yl)benzylidene)piperidin-1-yl)ethoxy)-2-methylquinoline (0.1 g, 0.21 mmol) and 10% palladium on charcoal (paste) (0.13g) in methanol (10 mL) was stirred at 20 °C under an atmosphere of hydrogen (1 atm) for 3 h. The reaction mixture was filtered through celite and the filtrate evaporated *in vacuo* to give the **title compound** (0.081 g, 82%) as an oil.
Mass spectrum (API⁺): Found 473 (MH⁺). C₃₀H₄₀N₄O requires 472.
¹H NMR (CDCl₃) δ: 1.14 (6H, m), 1.34 (2H, m), 1.57 (1H, m), 1.68 (2H, m), 2.13 (2H, m), 2.27 (2H, m), 2.50 (2H, d, J = 7 Hz), 2.72 (3H, s), 2.91 (4H, m), 3.04 (3H, m), 3.50 (2H, m), 4.27 (2H, m), 6.64 (1H, d, J = 7 Hz), 6.70 (1H, s), 6.77 (2H, m), 7.16 (1H, t, J = 8 Hz), 7.24 (1H, d, J = 9 Hz), 7.56 (2H, m), 8.43 (1H, d, J = 9 Hz).

### Example 61

### 2-Methyl-5-(2-(4-(3-(4-methylpiperazin-1-yl)benzyl)piperidin-1-yl)ethoxy)quinoline (E61)

The **title compound** was prepared from 2-methyl-5-(2-(4-(3-(4-methylpiperazin-1-yl)benzylidene)piperidin-1-yl)ethoxy)quinoline in a similar manner to Example 60.
Mass spectrum (API⁺): Found 459 (MH⁺). C₂₉H₃₈N₄O requires 458.
¹H NMR (CDCl₃) δ: 1.33 (2H, m), 1.53 (1H, m), 1.67 (2H, m), 2.13 (2H, m), 2.35 (3H, s), 2.49 (2H, d, J = 7 Hz), 2.57 (4H, m), 2.72 (3H, s), 2.92 (2H, m), 3.02 (2H, m), 3.20 (4H, m), 4.27 (2H, m), 6.65 (1 H, d, J = 7 Hz), 6.72 (1 H, s), 6.77 (2H, m), 7.16 (1 H, t, J = 8 Hz), 7.24 (1 H, d, J = 9 Hz), 7.56 (2H, m), 8.43 (1 H, d, J = 9 Hz).

### Example 62

### 2-Methyl-5-(2-(4-(3-((R)-3-methylpiperazin-1-yl)benzylidene)piperidin-1-yl)ethoxy)quinoline (E62)

The title compound was prepared in a similar manner to Example 58.
Mass spectrum (API⁺): Found 457 (MH⁺). C₂₉H₃₆N₄O require 456.
¹H NMR (CDCl₃) δ: 1.12 (3H, d, J = 6 Hz), 2.34 (1H, m), 2.42 (2H, m), 2.56 (2H, m), 2.62 (2H, m), 2.73 (7H, m), 2.97 (2H, m), 3.07 (3H, m), 3.50 (2H, m), 4.29 (2H, m), 6.28 (1H, s), 6.71 (1 H, d, J = 8 Hz), 6.78 (3H, m), 7.22 (2H, m), 7.57 (2H, m), 8.44 (1 H, d, J = 8 Hz).

### Example 63

### 2-Methyl-5-{2-[4-(3-morpholin-4-ylbenzylidene)piperidin-1-yl]ethoxy}quinoline (E63) The title compound was prepared in a similar manner to Example 58.

Mass spectrum (API⁺): Found 444 (MH⁺). C₂₈H₃₃N₃O₂ requires 443.
¹H NMR (CDCl₃) δ: 2.42 (2H, m), 2.56 (2H, m), 2.63 (2H, m), 2.73 (5H, m), 2.97 (2H, m), 3.15 (4H, m), 4.12 (4H, m), 4.30 (2H, m), 6.28 (1H, s), 6.77 (4H, m), 7.23 (2H, m), 7.57 (2H, m), 8.44 (1H, d, J = 8 Hz).

### Example 64

### 5-(2-{4-[3-((2S,5R)-2,5-Dimethylpiperazin-1-yl)benzylidene]piperidin-1-yl}ethoxy)-2-methylquinoline (E64)

The **title compound** was prepared in a similar manner to Example 58.

Mass spectrum (API⁺): Found 471 (MH⁺). C₃₀H₃₈N₄O requires 470.
¹H NMR (CDCl₃) δ: 0.91 (3H, m), 1.05 (3H, m), 2.41 (4H, m), 2.56 (2H, m), 2.64 (2H, m), 2.70 (5H, m), 2.95 (4H, m), 3.07 (3H, m), 4.30 (2H, m), 6.27 (1H, m), 6.81 (1H, m), 6.94 (2H, m), 7.25 (2H, m), 7.57 (2H, m), 8.45 (2H, d, J = 9 Hz).

### Example 65

### 2-Methyl-5-(2-(4-(3-((S)-3-methylpiperazin-1-yl)benzylidene)piperidin-1-yl)ethoxy)quinoline (E65)

The **title compound** was prepared in a similar manner to Example 58.
Mass spectrum (API⁺): Found 457 (MH⁺). C₂₉H₃₆N₄O requires 456.
¹H NMR (CDCl₃) δ: 1.12 (3H, m), 2.35 (1H, m), 2.42 (2H, m), 2.56 (2H, m), 2.62 (2H, m), 2.73 (7H, m), 2.97 (2H, m), 3.07 (3H, m), 3.50 (2H, m), 4.30 (2H, m), 6.28 (1H, s), 6.71 (1H, d, J = 8 Hz), 6.79 (3H, m), 7.23 (2H, m), 7.57 (2H, m), 8.44 (1H, d, J = 9 Hz).

### Example 66

### 2-Methyl-5-(2-(4-(3-((R)-3-methylpiperazin-1-yl)benzyl)piperidin-1-yl)ethoxy)quinoline (E66)

The **title compound** was prepared from 2-methyl-5-(2-(4-(3-((R)-3-methylpiperazin-1-yl)benzylidene)piperidin-1-yl)ethoxy)quinoline in a similar manner to Example 60.
Mass spectrum (API⁺): Found 459 (MH⁺). C₂₉H₃₈N₄O requires 458.
¹H NMR (CDCl₃) δ: 1 .13 (3H, d, J = 6 Hz), 1.33 (2H, m), 1.55 (1H, m), 1.67 (2H, m), 2.13 (2H, m), 2.33 (1 H, m), 2.50 (2H, m), 2.69 (2H, m), 2.72 (3H, s), 2.92 (2H, m), 2.96 - 3.09 (5H, m), 3.50 (2H, m), 4.27 (2H, m), 6.65 (1 H, d, J = 7 Hz), 6.71 (1 H, s), 6.77 (2H, m), 7.16 (1 H, t, J = 8 Hz), 7.24 (1 H, d, J = 9 Hz), 7.56 (2H, m), 8.43 (1 H, d, J = 9 Hz).

### Example 67

### 2-Methyl-5-{2-[4-(3-morpholin-4-ylbenzyl)piperidin-1-yl]ethoxy}quinoline (E67) The title compound was prepared from 2-methyl-5-{2-[4-(3-morpholin-4-ylbenzylidene)piperidin-1-yl]ethoxy}quinoline in a similar manner to Example 60.

Mass spectrum (API⁺): Found 446 (MH⁺). C₂₈H₃₅N₃O₂ requires 445. ¹H NMR (CDCl₃) δ: 1.33 (2H, m), 1.54 (1H, m), 1.67 (2H, m), 2.13 (2H, m), 2.50 (2H, d, J = 7 Hz), 2.72 (3H, s), 2.92 (2H, m), 3.03 (2H, m), 3.14 (4H, m), 3.85 (4H, m), 4.26 (2H, m), 6.68 (2H, m), 6.74 (1 H, dd, J = 8, 2 Hz), 6.78 (1 H, d, J = 7 Hz), 7.18 (1 H, t, J = 8 Hz), 7.23 (1 H, d, J =8 Hz), 7.56 (2H, m), 8.42 (1 H, d, J = 8 Hz).

### Example 68

### 5-(2-{4-[3-((2S,5R)-2,5-Dimethylpiperazin-1-yl)benzyl]piperidin-1-yl}ethoxy)-2-methylquinoline (E68)

The **title compound** was prepared from 5-(2-{4-[3-((2S,5R)-2,5-dimethylpiperazin-1-yl)benzylidene]piperidin-1-yl}ethoxy)-2-methylquinoline in a similar manner to Example 60. Mass spectrum (API⁺): Found 473 (MH⁺). C₃₀H₄₀N₄O requires 472.
¹H NMR (CDCl₃) δ: 0.90 (3H, m), 1.08 (3H, m), 1.34 (2H, m), 1.55 (1H, m), 1.66 (2H, m), 2.14 (2H, m), 2.50 (4H, m), 2.71 (5H, m), 2.98 (2H, m), 2.98 - 3.11 (5H,m), 4.27 (2H, m), 6.79 (1H, dd, J = 8, 1 Hz), 6.87 (2H, m), 6.95 (1H, m), 7.22 (2H, m), 7.56 (2H, m), 8.43 (1H, d, J= 8 Hz).

### Example 69

### 5-(2-{4-[3-((2R,6S)-2,6-Dimethylmorpholin-4-yl)benzyl]piperidin-1-yl}ethoxy)-2-methylquinoline (E69)

The title compound was prepared in a similar manner to Example 60.
Mass spectrum (API⁺): Found 474 (MH⁺). C₃₀H₃₉N₃O₂ requires 473.
¹H NMR (CDCl₃)δ: 1.26 (6H, m), 1.34 (2H, m), 1.54 (1H, m), 1.67 (2H, m), 2.14 (2H, m), 2.40 (2H, m), 2.50 (2H, d, J = 7 Hz), 2.72 (3H, s), 2.91 (2H, m), 3.04 (2H, m), 3.44 (2H, d, J = 11 Hz), 3.80 (2H, m), 4.27 (2H, m), 6.67 (2H, m), 6.74 (1 H, dd, J = 8, 2Hz), 6.79 (1 H, d, J = 7 Hz), 7.17 (1H, t, J = 8 Hz), 7.23 (1H, d, J = 9 Hz), 7.56 (2H, m), 8.42 (1H, d, J = 9 Hz).

### Example 70

### 2-Methyl-5-{2-[4-(3-piperidin-1-ylbenzyl)piperidin-1-yl]ethoxy}quinoline (E70)

The **title compound** was prepared from 2-methyl-5-{2-[4-(3-piperidin-1-ylbenzylidene)piperidin-1-yl]ethoxy}quinoline in a similar manner to Example 60.
Mass spectrum (API⁺): Found 444 (MH⁺). C₂₉H₃₇N₃O requires 443.
¹H NMR (CDCl₃)δ: 1.33 (2H, m), 1.57 (3H, m), 1.70 (6H, m), 2.13 (2H, m), 2.49 (2H, d, J = 7 Hz), 2.72 (3H, s), 2.92 (2H, m), 3.03 (2H, m), 3.14 (4H, m), 4.27 (2H, m), 6.61 (1H, d, J = 7 Hz), 6.76 (3H, m), 7.14 (1H, t, J = 8 Hz), 7.24 (1H, d, J = 9 Hz), 7.56 (2H, m), 8.43 (1H, d, J = 9 Hz).

### Example 71

### 2-Methyl-5-(2-{4-[3-((S)-3-methylpiperazin-1-yl)benzyl]piperidin-1-yl}ethoxy)quinoline (E71)

The **title compound** was prepared from 2-methyl-5-(2-(4-(3-((*S*)-3-methylpiperazin-1-yl)benzylidene)piperidin-1-yl)ethoxy)quinoline in a similar manner to Example 60.
Mass spectrum (API⁺): Found 459 (MH⁺). C₂₉H₃₈N₄O requires 458.
¹H NMR (CDCl₃)δ: 1.13 (3H, m), 1.34 (2H, m), 1.55 (1H, m), 1.67 (2H, m), 2.16 (2H, m), 2.34 (1 H, m), 2.50 (2H, d, J = 7 Hz), 2.72 (5H, m), 2.92 (2H, m), 2.98 - 3.09 (5H, m), 3.48 (2H, m), 4.27 (2H, m), 6.65 (1H, d, J = 7 Hz), 6.71 (1H, s), 6.77 (2H, m), 7.16 (1H, t, J = 8 Hz), 7.24 (1H, d, J = 9 Hz), 7.56 (2H, m), 8.43 (1H, d, J = 9 Hz).

### Example 72

### 2-Methyl-5-(2-{4-[3-(4-methyl[1,4]diazepan-1-yl)benzyl]piperidin-1-yl}ethoxy)quinoline (E72)

The title compound was prepared in a similar manner to Example 60.
Mass spectrum (API⁺): Found 473 (MH⁺). C₃₀H₄₀N₄O requires 472.
¹H NMR (CDCl₃) δ: 1.34 (2H, m), 1.55 (1H, m), 1.69 (2H, m), 2.14 (2H, m), 2.38 (3H, s), 2.48 (2H, d, J = 7 Hz), 2.57 (4H, m), 2.72 (5H, m), 2.93 (2H, m), 3.03 (2H, m), 3.47 (2H, m), 3.56 (2H, m), 4.27 (2H, m), 6.47 (2H, m), 6.53 (1H, m), 6.79 (1H, d, J = 7 Hz), 7.11 (1H, t, J = 8 Hz), 7.24 (1H, m), 7.56 (2H, m), 8.43 (1H, d, J = 9 Hz).

### Example 73

### 2-Methyl-5-(2-(4-(3-pyrrolidin-1-ylbenzyl)piperidin-1-yl)ethoxy)quinoline (E73)

The title compound was prepared in a similar manner to Example 60.
Mass spectrum (API⁺): Found 430 (MH⁺). C₂₈H₃₅N₃O requires 429.
¹H NMR (CDCl₃) δ: 1.36 (2H, m), 1.54 (1H, m), 1.68 (2H, m), 1.74 (4H, m), 1.98 (2H, m), 2.13 (2H, m), 2.54 (2H, d, J = 7 Hz), 2.72 (3H, s), 2.92 (2H, m), 3.03 (2H, m), 3.27 (2H, m), 4.27 (2H, m), 6.79 (1H, m), 7.14 (1H, m), 7.26 (4H, m), 7.56 (2H, m), 8.43 (1H, d, J = 9 Hz).

### Example 74

### 2-Methyl-5-{2-[4-(3-piperidin-1-ylbenzylidene)piperidin-1-yl]ethoxy}quinoline (E74)

The **title compound** was prepared in a similar manner to Example 58.
Mass spectrum (API⁺): Found 442 (MH⁺). C₂₉H₃₅N₃O requires 441.
¹H NMR (CDCl₃) δ: 1.57 (2H, m), 1.70 (4H, m), 2.42 (2H, m), 2.58 (2H, m), 2.62 (2H, m), 2.73 (5H, m), 2.97 (2H, m), 3.14 (4H ,m), 4.30 (2H, m), 6.27 (1H, s), 6.68 (1H, d, J = 8 Hz), 6.79 (3H, m), 7.18 (1H, t, J = 8 Hz), 7.25 (1 H, m), 7.57 (2H, m), 8.44 (1H, d, J = 9 Hz).

### Example 75

### 2-Methyl-5-(2-(4-(3-piperidin-1-ylbenzylidene)piperidin-1-yl)ethoxy)quinazoline (E75)

The **title compound** was prepared in a similar manner to Example 58 from 5-(2-(4-(3-bromobenzylidene)piperidin-1-yl)ethoxy)-2-methylquinazoline.
Mass spectrum (API⁺): Found 443 (MH⁺). C₂₈H₃₄N₄O requires 442.
¹H NMR (CDCl₃) δ: 1.57 (2H, m), 1.70 (4H, m), 2.42 (2H, m), 2.54 (2H, m), 2.58 (2H, m), 2.62 (2H, m), 2.88 (3H, s), 2.99 (2H, m), 3.14 (4H, m), 4.33 (2H, m), 6.28 (1H, s), 6.68 (1H, d, J = 7 Hz), 6.79 (2H, m), 6.87 (1H, d, J = 8 Hz), 7.19 (1H, m), 7.50 (1H, d, J = 9 Hz), 7.77 (1H, t, J = 8 Hz), 9.65 (1H, s).

### Example 76

### 2-Methyl-5-(2-(4-(3-piperidin-1-ylbenzyl)piperidin-1-yl)ethoxy)quinazoline (E76)

The **title compound** was prepared from 2-methyl-5-(2-(4-(3-piperidin-1-ylbenzylidene)piperidin-1-yl)ethoxy)quinazoline in a similar manner to Example 60.
Mass spectrum (API⁺): Found 445 (MH⁺). C₂₈H₃₆N₄O requires 444.
¹H NMR (CDCl₃) δ: 1.33 (2H, m), 1.57 (3H, m), 1.70 (6H, m), 2.14 (2H, m), 2.48 (2H, d, J = 7 Hz), 2.88 (3H, s), 2.93 (2H, m), 3.02 (2H, m), 3.14 (4H, m), 4.30 (2H, m), 6.61 (1 H, d, J = 7 Hz), 6.72 (1H, s), 6.76 (1H, dd, J = 8, 2 Hz), 6.85 (1H, d, J = 8 Hz), 7.14 (1H,t, J = 8 Hz), 7.48 (1 H, d, J = 8 Hz), 7.73 (1 H, t, J = 8 Hz), 9.63 (1 H, s).

### Example 77

### N-(3-{4-[2-(2-Methylquinolin-5-yloxy)ethyl]piperazin-1-ylmethyl}phenyl)-acetamide (E77)

A mixture of 2-methyl-5-(2-piperazin-1-ylethoxy)quinoline (0.04 g, 0.15 mmol) and 3-acetamidobenzaldehyde (0.024 g, 0.15 mmol) in 1,2-dichloroethane (5 mL) was treated with sodium triacetoxyborohydride (47 m g, 0.22 mmol) and stirred at 20°C under an atmosphere of argon for 24 h. The mixture was then treated with saturated aqueous NaHCO₃ (20 mL) and the organic layer separated and purified directly by chromatography on silica (ethyl acetate to 10% methanol / ethyl acetate), to afford the title compound (0.034 g, 55%) as a solid.
Mass spectrum (API⁺): Found 419 (MH⁺). C₂₅H₃₀N₄O₂ requires 418.
¹H NMR (CDCl₃) δ: 2.15 (3H, s), 2.41-2.50 (4H, m), 2.67 (4H, br), 2.72 (3H, s), 2.95 (2H, t *J* 6), 3.48 (2H, s), 4.27 (2H, t *J* 6), 6.78 (1 H, dd *J* 7 and 1), 7.05 (1 H, d *J* 8), 7.22-7.28 (2H, m), 7.43-7.61 (4H, m), 8.42 (1 H, d *J* 9).

The examples of Table 1 were prepared in a similar manner to Example E77.

**Table 1**

| | | | | |
|---|---|---|---|---|
| 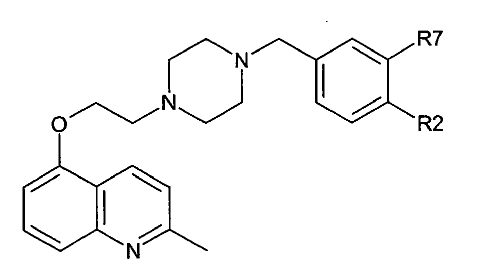 | | | | |

| **Example** | R7 | R2 | Mass Spectrum (API⁺) | |
|---|---|---|---|---|
| **E78** | NO₂ | OMe | Found 437 requires 436. (MH⁺). | C₂₄H₂₈N₄O₄ |
| **E79** | NO₂ | H | Found 407 requires 406. (MH⁺). | C₂₃H₂₆N₄O₃ |
| **E80** | NO₂ | F | Found 425 requires 424. (MH⁺). | C₂₃H₂₅FN₄O₃ |
| **E81** | NO₂ | Me | Found 421 requires 420. (MH⁺). | C₂₄H₂₈N₄O₃ |
| **E82** | NO₂ | Cl | Found 441 requires 440. (MH⁺). | C₂₃H₂₅ClN₄O₃ |
| **E83** | NHSO₂Me | H | Found 455 requires 454. (MH⁺). | C₂₄H₃₀N₄O₃S |
| **E84** | 4-methyl-1-piperazinyl | H | Found 460 requires 459. (MH⁺). | C₂₈H₃₇N₅O |
| **E85** | 1-imidazolinyl | H | Found 428 requires 427. (MH⁺). | C₂₆H₂₉N₅O |

### Example 86

### 2-Amino-4-{4-[2-(2-methylquinolin-5-yloxy)ethyl]piperazin-1-ylmethyl}phenol (E86)

5-{2-[4-(4-Methoxy-3-nitrobenzyl)piperazin-1-ylmethyl]ethoxy}-2-methylquinoline (0.169 g, 0.4 mmol) was dissolved in methanol (20 mL) and treated with 10% palladium on carbon (0.169 g) and ammonium formate (0.126 g, 2.0 mmol) and the resulting mixture stirred at ambient temperature for 2h before being filtered through Kieselguhr. The filtrate was evaporated to dryness under reduced pressure and the residue partitioned between ethyl acetate and saturated NaHCO₃ *aq*. The phases were separated and the organic phase washed with saturated brine, dried (MgSO₄) and evaporated to dryness under reduced pressure. Chromatography of the residue on SiO₂, eluting with 10% 0.880 NH₃/MeOH in dichloromethane (0-10% gradient), gave the title compound (0.099 g, 63%) as a brown gum.
¹H NMR (CDCl₃) δ: 2.52 and 2.70 (each 4H, 2br m), 2.72 (3H, s), 2.96 (2H, t), 3.38 (2H, s), 4.27 (2H, t), 6.48 (1H, dd), 6.55 (1H, d), 6.70 (1 H, d), 6.77 (1H, d), 7.23 (1H, d) 7.53 (1H, t), 7.61 (1 H, d), 8.43 (1H, d).

### Example 87

### N-(4-Fluoro-2-methoxy-5-{1-[2-(2-methylquinolin-5-yloxy)ethyl]piperidin-4-ylmethyl}phenyl)methanesulfonamide (E87)

The title compound was prepared using analogous routes and intermediates to those used to prepare Example 3.
Mass spectrum (API⁺): Found 502 (MH⁺). C₂₆H₃₂FN₃O₄S requires 501.
¹H NMR (CDCl₃) δ: 1.30-1.45 (2H, m), 1.50-1.70 (3H, m), 2.10-2.20 (2H, m), 2.53 (2H, d, J = 8 Hz), 2.73 (3H, s), 2.85-2.95 (5H, m), 3.00-3.05 (2H, m), 3.85 (3H, s), 4.26 (2H, t, J = 6 Hz), 6.51 (1H, br. s), 6.63 (1H, d, J = 11 Hz), 6.79 (1 H, d, J = 8 Hz), 7.23 (1 H, d, J = 8 Hz), 7.30 (1H, d, J = 8 Hz), 7.50-7.70 (2H, m), 8.43 (1 H, d, J = 8 Hz).

### Example 88

### N-(4-Fluoro-2-methoxy-5-{1-[2-(2-methylquinolin-5-yloxy)ethyl]piperidin-4-ylmethyl}phenyl)-2-propanesulfonamide (E88)

The title compound was prepared using analogous routes and intermediates to those used to prepare Example 3.
Mass spectrum (API⁺): Found 530 (MH⁺). C₂₈H₃₆FN₃O₄S requires 529.
¹H NMR (CDCl₃) δ: 1.35 (6H, d, J = 7 Hz), 1.48-1.58 (1H, m), 1.60-1.70 (4H, m), 2.10-2.20 (2H, m), 2.52 (2H, d, J = 8 Hz), 2.73 (3H, s), 2.92 (2H, t, J = 6 Hz), 2.95-3.05 (2H, m), 3.10-3.20 (1H, m), 3.85 (3H, s), 4.26 (2H, t, J = 6 Hz), 6.50 (1H, br. s), 6.60 (1H, d, J = 11 Hz), 6.79 (1H, d, J = 8 Hz), 7.26 (1H, d, J = 8 Hz), 7.36 (1 H, d, J = 8 Hz), 7.50-7.60 (2H, m), 8.43 (1H, d, J = 8 Hz).

### Example 89

### 1-(4-Fluoro-2-methoxy-5-{1-[2-(2-methylquinolin-5-yloxy)ethyl]piperidin-4-ylmethyl}phenyl)pyrrolidin-2-one (E89)

The **title compound** was prepared using analogous routes and intermediates to those used to prepare Example 5.
Mass spectrum (API⁺): Found 492 (MH⁺). C₂₉H₃₄FN₃O₃ requires 491.
¹H NMR (CDCl₃) δ: 1.30-1.48 (2H, m), 1.50-1.80 (3H, m), 2.10-2.25 (4H, m), 2.45-2.60 (4H, m), 2.73 (3H, s), 2.93 (2H, t, J = 6 Hz), 3.02-3.06 (2H, m), 3.71 (2H, t, J = 4 Hz), 3.79 (3H, s), 4.28 (2H, t, J = 6 Hz), 6.64 (1H, d, J = 12 Hz), 6.79 (1H, d, J = 8 Hz), 7.00 (1H, d, J = 8 Hz), 7.25 (1H, d, J = 10 Hz), 7.50-7.60 (2H, m), 8.43 (1H, d, J = 8 Hz).

### Example 90

### N-(3-Fluoro-2-methoxy-5-{1-[2-(2-methylquinolin-5-yloxy)ethyl]piperidin-4-ylmethyl}phenyl)-methanesulfonamide (E90)

The **title compound** was prepared using analogous routes and intermediates to those used to prepare Example 3.
Mass spectrum (API⁺): Found 502 (MH⁺). C₂₆H₃₂FN₃O₄S requires 501.
¹H NMR (CDCl₃) δ: 1.30-1.42 (2H, m), 1.45-1.60 (1H, m), 1.60-1.70 (2H, m), 2.17 (2H, t, J = 12 Hz), 2.48 (2H, d, J = 8 Hz), 2.72 (3H, s), 2.95 (2H, t, J = 6 Hz), 3.00 (3H, s), 3.02-3.09 (2H, m), 3.97 (3H, s), 4.28 (2H, t, J = 6 Hz), 6.66 (1H, d, J = 11 Hz), 6.79 (1H, d, J = 8 Hz), 7.10 (1H, s), 7.25 (1H, d, J = 8 Hz), 7.50-7.61 (2H, m), 8.43 (1H, d, J = 8 Hz). **Example 91**

### N-(3-Fluoro-2-methoxy-5-{1-[2-(2-methylquinolin-5-yloxy)ethyl]piperidin-4-ylmethyl}phenyl)-1-propanesulfonamide (E91)

The **title compound** was prepared using analogous routes and intermediates to those used to prepare Example 3.
Mass spectrum (API⁺): Found 530 (MH⁺). C₂₈H₃₆FN₃O₄S requires 529.
¹H NMR (CDCl₃) δ: 1.01 (3H, t, J = 8 Hz), 1.30-1.40 (2H, m), 1.48-1.58 (1 H, m), 1.60-1.70 (2H, m), 1.80-1.90 (2H, m), 2.10-2.20 (2H, m), 2.47 (2H, d, J = 7 Hz), 2.73 (3H, s), 2.93 (2H, t, J = 6 Hz), 3.00-3.10 (4H, m), 3.97 (3H, s), 4.28 (2H, t, J = 6 Hz), 6.66 (1H, d, J = 12 Hz), 6.80 (1H, d, J = 8 Hz), 6.85 (1H, br. s), 7.11 (1 H, s), 7.25 (1H, d, J = 8 Hz), 7.50-7.70 (2H, m), 8.43 (1H, d, J = 8 Hz).

### Example 92

### N-(3-Fluoro-2-methoxy-5-{1-[2-(2-methylquinolin-5-yloxy)ethyl]piperidin-4-ylmethyl}phenyl)-2-propanesulfonamide (E92)

The **title compound** was prepared using analogous routes and intermediates to those used to prepare Example 3.
Mass spectrum (API⁺): Found 530 (MH⁺). C₂₈H₃₆FN₃O₄S requires 529.
¹H NMR (CDCl₃) δ: 1.25-1.40 (2H, m), 1.38 (6H, d, J = 7 Hz), 1.45-1.60 (1H, m), 1.60-1.72 (2H, m), 2.10-2.20 (2H, m), 2.46 (2H, d, J = 8 Hz), 2.73 (3H, s), 2.93 (2H, t, J = 7 Hz), 3.00-3.10 (2H, m), 3.22-3.32 (1H, m), 3.97 (3H, s), 4.28 (2H, t, J = 6 Hz), 6.63 (1H, d, J = 12 Hz), 6.70-6.90 (2H, m), 7.15 (1H, s), 7.25 (1H, d, J = 8 Hz), 7.50-7.70 (2H, m), 8.43 (1H, d, J = 8 Hz).

### Example 93

### 5-(2-{4-[3-(1,1 -Dioxo-1l⁶-isothiazolidin-2-yl)-5-fluoro-4-methoxybenzyl]piperidin-1-yl}ethoxy)-2-methylquinoline (E93)

The **title compound** was prepared using analogous routes and intermediates to those used to prepare Example 5.
Mass spectrum (API⁺): Found 529 (MH⁺). C₂₈H₃₄FN₃O₄S requires 528.
¹H NMR (CDCl₃) δ: 1.30-1.42 (2H, m), 1.45-1.50 (1H, m), 1.63-1.72 (2H, m), 2.10-2.25 (2H, m), 2.42-2.60 (4H, m), 2.73 (3H, s), 2.96 (2H, t, J = 6 Hz), 3.02-3.12 (2H, m), 3.28 (2H, t, J = 7 Hz), 3.79 (2H, t, J = 8 Hz), 3.96 (3H, s), 4.30 (2H, t, J = 6 Hz), 6.80 (1 H, d, J = 7 Hz), 6.85 (1H, dd, J = 8, 2 Hz), 7.00 (1H, s), 7.25 (1H, d, J = 8 Hz), 7.50-7.70 (2H, m), 8.43 (1H, d, J = 8 Hz).

### Example 94

### 1-(3-Fluoro-2-methoxy-5-{1-[2-(2-methylquinolin-5-yloxy)ethyl]piperidin-4-ylmethyl}phenyl)pyrrolidin-2-one (E94)

The **title compound** was prepared using analogous routes and intermediates to those used to prepare Example 5.
Mass spectrum (API⁺): Found 492 (MH⁺). C₂₉H₃₄FN₃O₃ requires 491.
¹H NMR (CDCl₃) δ: 1.30-1.48 (2H, m), 1.50-1.60 (1H, m), 1.64-1.76 (2H, m), 2.10-2.28 (4H, m), 2.48 (2H, d, J = 7 Hz), 2.56 (2H, t, J = 8 Hz), 2.73 (3H, s), 2.98 (2H, t, J = 6 Hz), 3.02-3.12 (2H, m), 3.76 (2H, t, J = 8 Hz), 3.90 (3H, s), 4.31 (2H, t, J = 6 Hz), 6.70-6.90 (3H, m), 7.25 (1 H, d, J = 8 Hz), 7.50-7.66 (2H, m), 8.42 (1H, d, J = 8 Hz).

### Example 95

### N-(3-(1-(2-(2-Trifluoromethylquinolin-5-yloxy)ethyl)piperidin-4-ylmethyl)phenyl)methanesulfonamide (E95)

The **title compound** was prepared from 5-(2-bromoethoxy)-2-trifluoromethylquinoline using analogous routes and intermediates to those used to prepare Example 3.
Mass spectrum (API⁺): Found 508 (MH⁺). C₂₅H₂₈F₃N₃O₃S requires 507.
¹H NMR (CDCl₃) δ: 1.25-1.38 (2H, m), 1.47-1.60 (1H, m), 1.60-1.70 (2H, m), 2.10-2.20 (2H, m), 2.55 (2H, d), 2.94 (2H, t), 3.00 (3H, s), 3.02-3.08 (2H, m), 4.30 (2H, t), 6.92-6.99 (2H, m), 7.00-7.07 (2H, m), 7.20-7.30 (1 H, m), 7.66-7.73 (2H, m), 7.79 (1 H, d), 8.74 (1H, d). NH not discernible.

### Example 96

### 5-(2-(4-(3-(4-Methylpiperazin-1-yl)benzylidene)piperidin-1-yl)ethoxy)-2-trifluoromethylquinoline (E96)

The **title compound** was prepared from 5-(2-bromoethoxy)-2-trifluoromethylquinoline using analogous routes and intermediates to those used in Description 19 and Example 58.
Mass spectrum (API⁺): Found 511 (MH⁺). C₂₉H₃₃F₃N₄O requires 510.
¹H NMR (CDCl₃) δ: 2.34 (3H, s), 2.40-2.46 (2H, m), 2.52-2.59 (6H, m), 2.60-2.66 (2H, m), 2.7-2.76 (2H, m), 2.99 (2H, t), 3.18-3.23 (4H, m), 4.33 (2H, t), 6.28 (1 H, s), 6.71 (1 H, d), 6.73-6.80 (2H, m), 6.97 (1 H, d), 7.20 (1 H, t), 7.68-7.73 (2H, m), 7.80 (1 H, d), 8.75 (1 H, d).

### Example 97

### 5-(2-(4-(3-(4-Methylpiperazin-1-yl)benzyl)piperidin-1-yl)ethoxy)-2-trifluoromethylquinoline (E97)

The **title compound** was prepared from 5-(2-(4-(3-(4-methylpiperazin-1-yl)benzylidene)piperidin-1-yl)ethoxy)-2-trifluoromethylquinoline using an analogous method to Example 2.
Mass spectrum (API⁺): Found 513 (MH⁺). C₂₉H₃₅F₃N₄O requires 512.
¹H NMR (CDCl₃) δ: 1.22-1.30 (m, 2H), 1.49-1.60 (m, 1H), 1.68 (br d, 2H), 2.14 (t, 2H), 2.35 (s, 3H), 2.49 (d, 2H), 2.57 (t, 4H), 2.94 (t, 2H), 3.01 (br d, 2H), 3.20 (t, 4H), 4.30 (t, 2H), 6.65 (d, 1H), 6.72 (s, 1H), 6.75 (dd, 1H), 6.95 (d, 1H), 7.72 (t, 1H), 7,67-7.71 (m, 2H), 7.79 (d, 1H), 8.74 (d, 1H).

### Example 98

### N-(2-Methoxy-3-{1-[2-(2-methylquinolin-5-yloxy)ethyl]piperidin-4-ylmethyl}phenyl)propane-1-sulfonamide (E98)

The **title compound** was prepared using analogous routes and intermediates to those used to prepare Example 3.
Mass spectrum (API⁺): Found 512 (MH⁺). C₂₈H₃₇N₃O₄S requires 511.
¹H NMR (CDCl₃) δ: 1.01 (3H, t, *J* 7), 1.25-1.45 (2H, m), 1.55-1.70 (2H, m), 1.70-1.95 (3H, m), 2.10-2.23 (2H, m), 2.58 (2H, d, *J* 7), 2.72 (3H, s), 2.93 (2H, t, *J* 6), 3.00-3.16 (4H, m), 3.75 (3H, s), 4.27 (2H, t, *J* 6), 6.79 (1 H, d, *J* 7), 6.82 (1 H, br. s), 6.89 (1H, dd, *J* 7 and 2), 7.03 (1H, t, *J* 8), 7.24 (1H, d, *J* 8), 7.36 ( 1H, dd, *J* 8 and 2), 7.50-7.65 (2H, m), 8.42 (1H, d, *J* 8).

### Example 99

### N-(2-Methoxy-3-{1-[2-(2-methylquinolin-5-yloxy)ethyl]piperidin-4-ylmethyl}phenyl)propane-2-sulfonamide (E99)

The **title compound** was prepared using analogous routes and intermediates to those used to prepare Example 3.
Mass spectrum (API⁺): Found 512 (MH⁺). C₂₈H₃₇N₃O₄S requires 511.
¹H NMR (CDCl₃) δ: 1.40 (6H, d, *J* 7), 1.55-1.75 (5H, m), 2.10-2.25 (2H, m), 2.58 (2H, d, *J* 7), 2.73 (3H, s), 2.93 (2H, m), 3.01-3.08 (2H, m), 3.36 (1H, m), 3.75 (3H, s), 4.28 (2H, t, *J* 6), 6.74 (1H, br. s), 6.79 (1H, d, *J* 7), 6.87 (1H, dd, *J* 8 and 1), 7.01 (1H, t, *J* 8), 7.24 (1H, d, *J* 8), 7.36 (1H, dd, *J* 8 and 2), 7.50-7.65 (2H, m), 8.42 (1 H, d, *J* 8).

### Example 100

### N-(2-Methoxy-3-{1-[2-(2-methylquinolin-5-yloxy)ethyl]piperidin-4-ylmethyl}phenyl)benzenesulfonamide (E100)

The **title compound** was prepared using analogous routes and intermediates to those used to prepare Example 3.
Mass spectrum (API⁺): Found 546 (MH⁺). C₃₁H₃₅N₃O₄S requires 545.
¹H NMR (CDCl₃) δ: 1.25 (2H, m), 1.51 (3H, m), 2.01 (2H, m), 2.46 (2H, d, *J* 7), 2.72 (3H, s), 2.91 (2H, t, *J* 6), 3.00 (2H, m), 3.45 (3H, s), 4.26 (2H, t, *J* 6), 6.70-6.85 (2H, m), 6.97 (1H, t, *J* 8), 7.02 (1H, br. s), 7.25 (1H, d, *J* 8), 7.42 (3H, m), 7.45-7.60 (3H, m), 7.83 ( 2H, dd, *J* 8 and 1), 8.42 (1H, d, *J* 8).

### Example 101

### 5-(2-{4-[3-(1,1-Dioxo-1l⁶-isothiazolidin-2-yl)-2-methoxybenzyl]piperidin-1-yl}ethoxy)-2-methylquinoline (E101)

The **title compound** was prepared using analogous routes and intermediates to those used to prepare Example 5.
¹H NMR (CDCl₃) δ: 1.35 (2H, m), 1.55-1.75 (7H, m), 2.17 (2H, m), 2.54 (2H, d, *J* 7), 2.72 (3H, s), 2.94 (2H, t, *J* 6), 3.06 (2H, m), 3.73 (3H, s), 3.74 (2H, d, *J* 7), 4.29 (2H, t, *J* 6), 6.53 (1 H, d, *J* 8), 6.61 (1H, d, *J* 8), 6.79 (1 H, d, *J* 8), 6.84 (1 H, t, *J* 8), 7.24 (1 H, d, *J* 8), 7.50-7.72 (2H, m), 8.42 (1H, d, *J* 8).

### Example 102

### 1-Methyl-4-(3-{1-[2-(2-methylquinolin-5-yloxy)ethyl]piperidin-4-ylidenemethyl}phenyl)piperazin-2-one (E102)

The **title compound** was prepared from 5-{2-[4-(3-bromobenzylidene)piperidin-1-yl]ethoxy}-2 methylquinoline in a manner similar to Example 58.
Mass spectrum (API⁺): Found 471 (MH⁺). C₂₉H₃₄N₄O₂ requires 470.
¹H NMR (CDCl₃) δ: 2.43 (2H, m), 2.56 (2H, m)m 2.64 (2H, m), 2.74 (5H, m), 2.98 (6H, m), 3.46 (3H s), 3.87 (2H, s), 4.31 (2H, m), 6.27 (1 H, bs), 6.71-6.83 (4H, m), 7.24 (2H, m), 7.57 (2H, m), 8.4 (1 H, d J 9).

### Example 103

### 1-Methyl-4-(3-{1-[2-(2-methylquinolin-5-yloxy)ethyl]piperidin-4-ylmethyl}phenyl)piperazin-2-one (E103)

The **title compound** was prepared from 1-methyl-4-(3-{1-[2-(2-methylquinolin-5 yloxy)ethyl]piperidin-4-ylidenemethyl}phenyl)piperazin-2-one in a manner similar to Example 60.
Mass spectrum (API⁺): Found 473 (MH⁺). C₂₉H₃₆N₄O₂ requires 472.
¹H NMR (CDCl₃) δ: 1.39 (2H, m), 1.53 (1H, m), 1.67 (2H, m), 1.87 (2H, m), 2.51 (2H, d J 7), 2.7 (3H, s), 2.95-3.11 (8H, m), 3.46 (3H, s), 3.87 (2H, s), 4.29 (2H, m), 6.67 (1 H, m), 6.72 (1 H, m), 6.7 (1 H, m), 7.16-7.26 (2H, m), 7.50-7.63 (3H, m), 8.42 (1 H, d J 9).

### Example 104

### 2-Methyl-5-(2-{4-[3-(4-methylpiperazin-1-yl)benzylidene]piperidin-1-yl}ethoxy)quinazoline (E104)

The title compound was prepared from 5-{2-[4-(3-bromobenzylidene)piperidin-1-yl]ethoxy}-2 methylquinazoline in a manner similar to Example 58.
Mass spectrum (API⁺): Found 458 (MH⁺). C₂₈H₃₅N₅O requires 457.
¹H NMR (CDCl₃) δ: 2.35 (3H, s), 2.42 (2H, m), 2.58 (6H, m), 2.62 (2H, m), 2.74 (2H, m), 2.88 (3H s), 2.99 (2H, m), 3.21 (4H, m), 4.33 (2H, m), 6.28 (1 H, bs), 6.71 (1H, m), 6.76 (1H, m), 6.79 (1H m), 6.87 (1H, d *J* 8), 7.20 (1H, t *J* 8), 7.50 (1H, d *J* 8), 7.74 (1H, t *J* 8), 9.65 (1H, s).

### Example 105

### 2-Methyl-5-(2-{4-[3-(4-methylpiperazin-1-yl)benzyl]piperidin-1-yl}ethoxy)quinazoline (E105)

The **title compound** was prepared from 2-methyl-5-(2-{4-[3-(4-methylpiperazin-1-yl)benzylidene]piperidin-1-yl}ethoxy)quinazoline in a manner similar to Example 60.
Mass spectrum (API⁺): Found 460 (MH⁺). C₂₈H₃₇N₅O requires 459.
¹H NMR (CDCl₃) δ: 1.34 (2H, m), 1.54 (1H, m) 1.67 (2H, m), 2.14 (2H, m), 2.35 (3H, s), 2.49 (2H, *J* 7), 2.58 (4H, m), 2.88 (3H, s), 2.93 (2H, m), 3.02 (2H, m), 3.21 (4H, m), 4.30 (2H, m), 6.50 (1H m), 6.75 (2H, m), 6.85 (1H, d *J* 8), 7.16 (1H, m), 7.48 (1H, m), 7.74 (1H, m), 9.63 (1H, s).

### Example 106

### 4-Methyl-1-(3-{1-[2-(2-methylquinolin-5-yloxy)ethyl]piperidin-4-ylmethyl}phenyl)piperazin-2-one (E106)

A solution of *C*-[(2-chloroethyl)methylamino]-*N*-(3-{1-[2-(2-methylquinolin-5-yloxy)ethyl]piperidin-4-ylmethyl}phenyl)acetamide (130 mg, 0.26 mmol) in DMF (5 ml) was treated with sodium hydride (14 mg, 60% suspension in oil, 0.35 mmol) at ambient temperature. The resulting mixture was stirred, under argon, at ambient temperature for 2 h, and then partitioned between ethyl acetate (5 ml) and water (3 x 5 ml). The organic layer was separated and added to a 10 g silica column and eluted from 0-10% methanol in ethyl acetate and 2% 0.880 ammonia in 10% methanol in ethyl acetate to give the **title compound** (28 mg, 23%) as a yellow oil.
Mass spectrum (API⁺): Found 473 (MH⁺). C₂₉H₃₆N₄O₂ requires 472.
¹H NMR (CDCl₃) δ: 1.35 (2H, m), 1.55 (1 H, m), 1.68 (2H, m), 2.13 (2H, m), 2.40 (3H, s), 2.54 (2H, *J* 7), 2.72 (3H, s), 2.78 (2H, m), 2.88 (2H, m), 2.92 (2H, m), 3.03 (2H, m), 3.69 (2H, m), 4.27 (2H m), 6.79 (1 H, m), 7.04-7.12 (2H, m), 7.27 (3H, m), 7.56 (2H, m), 8.42 (1 H, d *J* 9).

### Example 107

### 4-Benzyl-1-(3-{1-[2-(2-methylquinolin-5-yloxy)ethyl]piperidin-4-ylmethyl)phenyl)piperazin-2-one

The **title compound** was prepared from *C*-[benzyl-(2-chloroethyl)amino]-*N*-(3-{1-[2-(2-methylquinolin-5-yloxy)ethyl]piperidin-4-ylmethyl}phenyl)acetamide in a manner similar to Example 106.
Mass spectrum (API⁺): Found 549 (MH⁺). C₃₅H₄₀N₄O₂ requires 548.
¹H NMR (CDCl₃) δ: 1.65 (2H, m), 1.77 (3H, m), 2.47 (2H, m), 2.58 (2H, m), 2.73 (3H, s), 2.80 (2H m), 3.22 (2H, m), 3.33 (2H, s), 3.37 (2H, m), 3.63 (2H, s), 3.67 (2H, m), 4.49 (2H, m), 6.82 (1H, d 8), 7.03 (1H, m), 7.08 (1H, m), 7.13 (1H, m), 7.25-7.36 (7H, m), 7.56 (1H, t *J* 8), 7.63 (1H, m), 8.3 (1H, d *J* 8).

### Example 108

### 2-Methyl-5-{2-[4-(3-pyrazol-1-ylbenzylidene)piperidin-1-yl]ethoxy}quinoline (E108)

The **title compound** was prepared from 4-(3-pyrazol-1-ylbenzylidene)piperidine and 5-(2 bromoethoxy)-2-methylquinoline in an analogous manner to Example 1.
Mass spectrum (API⁺): Found 424 (MH⁺). C₂₇H₂₈N₄O requires 424.
¹H NMR (CDCl₃) δ: 2.46 (2H, m), 2.58 (2H, m), 2.65 (2H, m), 2.73 (3H, s), 2.76 (2H, m), 2.99 (2H m), 4.30 (2H, m), 6.34 (1H, s), 6.46 (1H, m), 6.81 (1H, d *J* 7), 7.12 (1H, d *J* 8), 7.25 (1H, m), 7.3 (1H, t *J* 8), 7.56 (4H, m), 7.72 (1H, d *J* 1), 7.91 (1H, d *J* 4), 8.44 (1H, d *J* 9).

### Example 109

### 2-Methyl-5-{2-[4-(3-pyrazol-1-ylbenzyl)piperidin-1-yl]ethoxy}quinoline (E109)

The **title compound** was prepared from 2-methyl-5-{2-[4-(3-pyrazol-1-yl-benzylidene)piperidin-1 yl]ethoxy}quinoline in a manner similar to Example 60.
Mass spectrum (API⁺): Found 427 (MH⁺). C₂₇H₃₀N₄O requires 426.
¹H NMR (CDCl₃) δ: 1.38 (2H, m), 1.62 (1H, m), 1.69 (2H, m), 2.15 (2H, m), 2.61 (2H, d *J* 7), 2.7 (3H, s), 2.93 (2H, m), 3.04 (2H, m), 4.27 (2H, m), 6.46 (1H, m), 6.79 (1H, d *J* 7), 7.07 (1H, d *J* 8 7.24 (1H, d *J* 9), 7.34 (1H, t *J* 8), 7.47 (1H, m), 7.56 (3H, m), 7.72 (1 H, d *J* 1), 7.92 (1H, d *J* 2), 8.4 (1H, d *J* 9).

### Example 110

### N-(3-Chloro-5-{1-[2-(2-methylquinolin-5-yloxy)ethyl]piperidin-4-ylidenemethyl}phenyl)methanesulfonamide (E110)

The **title compound** was prepared from 3-chloro-5-{1-[2-(2-methylquinolin-5-yloxy)ethyl]piperidin-4-ylidenemethyl}aniline using analogous routes and intermediates to those used in the preparation of Example 3.
Mass spectrum (API⁺): Found 487 (MH⁺). C₂₅H₂₈ClN₃O₃S requires 486.
¹H NMR (400 MHz, CDCl₃) δ 2.42 (2H,t *J* 5.6), 2.51 (2H, t *J* 5.2), 2.65 (2H, t *J* 5.2), 2.73 (3H, s), 2.74 (2H, t *J* 6.4), 2.99 (2H, t *J* 5.6), 3.04 (3H, s), 4.30 (2H, t *J* 5.6), 6.19 (1H, s), 6.81 (1H, d *J* 6.4), 6.92 (1H, s), 7.01 (1H, s), 7.06 (1H, s), 7.26 (1H, *J* 4.8), 7.54-7.62 (2H, m), 8.44 (1H, d *J* 8.4).

### Example 111

### 5-(2-{4-[3-((3R,5S)-3,5-Dimethylpiperazin-1-yl)-4-fluorobenzylidene]piperidin-1-ylethoxy)-2-methylquinoline (E111)

The **title compound** was prepared using analogous routes and intermediates to those used in the preparation of Example 58.
Mass spectrum (API⁺): Found 489 (MH⁺). C₃₀H₃₇N₄FO requires 488.
¹H NMR (400 MHz, CDCl₃) δ 1.1 (6H, d *J* 6.4), 2.30 (2H, t *J* 10.8), 2.42 (2H, t *J* 5.2), 2.53 (2H, t *J* 5.2), 2.63 (2H, t *J* 5.2), 2.73 (3H, s) 2.74 (2H, t *J* 5.6), 2.98 (2H, t *J* 5.6), 3.10-3.12 (2H, m), 3.29-3.32 (2H, d *J* 12.4), 4.30 (2H, t *J* 5.6), 6.232 (1h, s), 6.73-6.76 (2H, m), 6.81 (1H, d *J* 7.2), 6.92-6.97 (1H, m), 7.25 (1H, d *J* 8.8), 7.54-7.62 (2H, m), 8.44 (1H, d *J* 8.8).

### Example 112

### 5-(2-{4-[4-Fluoro-3-((R)-3-methylpiperazin-1-yl)benzylidene]piperidin-1-yl}ethoxy)-2-methylquinoline (E112)

The **title compound** was prepared using analogous routes and intermediates to those used in the preparation of Example 58.
Mass spectrum (API⁺): Found 475 (MH⁺). C₂₉H₃₅N₄FO requires 474.
¹H NMR (400 MHz, CDCl₃) δ 1.10 (3H, d *J* 6.4), 2.37 (1 H, m), 2.41 (2H, t *J* 5.2), 2.53 (2H, t *J* 5.2), 2.63 (2H, t *J* 5.2), 2.73 (3H, s), 2.74 (2H, t *J* 5.6), 2.98 (2H, t *J* 5.6) 3.06 (4H, m), 3.31 (2H, d *J* 11.6), 4.30 (2H, t *J* 5.6), 6.23 (1H, s), 6.75 (2H, m), 6.81 (2H, d *J* 7.2), 6.92-6.97 (1H, m), 7.25 (1H, d *J* 8.4), 7.54-7.62 (2H, m), 8.44 (1H, d *J* 8.8)*.*

### Example 113

### 5-(2-{4-[4-Fluoro-3-((S)-3-methylpiperazin-1-yl)benzylidene]piperidin-1-yl}ethoxy)-2-methylquinoline (E113)

The **title compound** was prepared using analogous routes and intermediates to those used in the preparation of Example 58.
Mass spectrum (API⁺): Found 475 (MH⁺). C₂₉H₃₅N₄FO requires 474.
¹H NMR (400 MHz, CDCl₃) δ 1.09 (3H, d *J* 6.2), 2.41 (3H, m), 2.53 (2H, t *J* 5.4), 2.63 (2H, t *J* 5.4), 2.73 (3H, s), 2.75 (3H, m), 2.98 (2H, t *J* 5.7), 3.08-3.12 (3H, m), 3.31 (2H, d *J* 11.5), 4.30 (2H, t *J* 5.7), 6.23 (2H, s), 6.74-6.77 (2H, m), 6.81 (1 H, d *J* 6.6), 6.92-6.97 (1H, m), 7.25 (1H, t *J* 3.6), 7.53-7.61 (2H, m), 8.44 (1H, d *J* 7.7).

### Example 114

### 5-(2-{4-[4-Fluoro-3-(4-methylpiperazin-1-yl)benzylidene]piperidin-1-yl}ethoxy)-2-methylquinoline (E114)

The **title compound** was prepared using analogous routes and intermediates to those used in the preparation of Example 58.
Mass spectrum (API⁺): Found 475 (MH⁺). C₂₉H₃₅FN₄O requires 474.
¹H NMR (400 MHz, CDCl₃) *δ* 2.38 (3H, s), 2.42 (2H, t *J* 5.2), 2.53 (2H, t *J* 5.4), 2.64 (6H, t *J* 4.7), 2.73 (3H, s), 2.74 (2H, t *J* 5.5), 2.99 (2H, t *J* 5.7), 3.13 (4H, t 4.7), 4.30 (2H, t *J* 5.7), 6.23 (1H, s), 6.75-6.77 (2H, m), 6.81 (1H, d *J* 7.5), 6.92-6.98 (1H, m), 7.26 (1H, s), 7.53-7.62 (2H, m), 8.44 (1H, d *J* 8.6).

### Example 115

### 5-(2-{4-[3-((3R,5S)-3,5-Dimethylpiperazin-1-yl)-4-fluorobenzyl]piperidin-1-yl}ethoxy)-2-methylquinoline (E115)

The **title compound** was prepared from 5-(2-{4-[3-((3*R*,5*S*)-3,5-dimethylpiperazin-1-yl)-4-fluorobenzylidene]piperidin-1-ylethoxy)-2-methylquinoline using the method of Example 60.
Mass spectrum (API⁺): Found 491 (MH⁺). C₃₀H₃₉FN₄O requires 490.
¹H NMR (400 MHz, CDCl₃) δ 1.11 (6H, d *J* 6.4), 1.29-1.33 (2H, m), 1.50 (1H, m), 1.64 (2H, d *J* 12.4), 2.14 (2H, t *J* 10.0), 2.29 (2H, t *J* 10.8), 2.47 (2H, d *J* 6.8), 2.73 (3H, s), 2.93 (2H, t *J* 5.6), 3.03 (2H, d *J* 11.6), 3.09-3.14 (2H, m), 3.29 (2H, d *J* 10.4), 4.27 (2H, t *J* 5.6), 6.66 (2H, m), 6.79 (1H, d *J* 7.6), 6.88-6.93 (1H, m), 7.25 (1H, d), 7.52-7.60 (2H, m), 8.42 (1H, d *J* 8.4).

### Example 116

### 5-(2-{4-[4-Fluoro-3-(4-methylpiperazin-1-yl)benzyl]piperidin-1-yl}ethoxy)-2-methylquinoline (E116)

The **title compound** was prepared from 5-(2-{4-[4-fluoro-3-(4-methylpiperazin-1-yl)benzylidene]piperidin-1-yl}ethoxy)-2-methylquinoline using the method of Example 60.
Mass spectrum (API⁺): Found 477 (MH⁺). C₂₉H₃₇N₄OF requires 476.
¹H NMR (400 MHz, CDCl₃) δ 1.36 (2H, *J* 12.4), 1.49 (1H, m br), 1.65 (2H, d *J* 12.5), 2.16 (2H, t *J* 11.6), 2.36 (3H, s), 2.47 (2H, d *J* 7.0), 2.61 (4H, t *J* 4.4), 2.72 (3H, s), 2.94 (2H, t *J* 5.7), 3.05 (2H, d *J* 11.6), 3.12 (4H, t *J* 4.4), 4.28 (2H, t *J* 5.7), 6.69 (2H, m), 6.79 (2H, d *J* 7.4), 6.91 (1H, m), 7.57 (2H, m), 8.42 (1H, d *J* 8.6).

### Example 117

### 5-(2-{4-[4-Fluoro-3-(-4-methylpiperazin-1-yl)benzyl]piperidin-1-yl}ethoxy)-2 methylquinazoline (E117)

The **title compound** was prepared 1-(2-fluoro-5-(piperidin-4-ylmethyl)phenyl)-4-methylpiperazine and 5-[2-(methanesulfonyloxy)ethoxy]-2-methylquinazoline according the method of Example 48.
Mass spectrum (API⁺): Found 478 (MH⁺). C₂₈H₃₆N₅OF requires 477.
¹H NMR (400 MHz, CDCl₃) δ 1.36 (2H, t br *J* 11.6), 1.50 (1H, m br), 1.65 (2H, d *J* 12.4), 2.2 (2H, t *J* 11.2), 2.39 (3H, s), 2.47 (2H, d *J* 6.8), 2.65 (4H, br), 2.88 (3H, s), 2.97 (2H, t *J* 5.6), 3.05 (2H, d *J* 11.2), 3.15 (4H, t *J* 4.4), 4.33 (2H, t *J* 5.6), 6.68 (2H, m), 6.84-6.94 (2H, m), 7.49 (1H, d *J* 8.0), 7.74 (1 H, t *J* 8.4), 9.63 (1H, s).

### Example 118

### 5-(2-{4-[4-Fluoro-3-(4-methylpiperazin-1-yl)benzyl]piperidin-1-yl}ethoxy)-2-trifluoromethylquinoline (E118)

The **title compound** was prepared 1-(2-fluoro-5-(piperidin-4-ylmethyl)phenyl)-4-methylpiperazine and 5-(2-bromoethoxy)-2-(trifluoromethyl)quinoline according the method of Example 2.
Mass spectrum (API⁺): Found 531 (MH⁺). C₂₉H₃₄N₄OF₄ requires 530.
¹H NMR (400 MHz, CDCl₃) δ 1.26-1.39 (2H, m), 1.66 (2H, d *J* 12.8), 1.90 (1H, br), 2.17 (2H, t *J* 11.5), 2.38 (3H, s), 2.48 (2H, d *J* 6.92), 2.63 (4H, s br), 2.97 (2H, t *J* 5.6), 3.05 (2H, d *J* 11.3), 3.13 (4H, s br), 4.32 (2H, t *J* 5.6), 6.68 (2H, m), 6.88-6.97 (2H, m), 7.68-7.72 (2H, m), 7.79 (1 H, d *J* 8.6), *8.74* (1H, d *J* 8.8).

### Example 119

### 5-(2-{4-[4-Fluoro-3-((R)-3-methylpiperazin-1-yl)benzyl]piperidin-1-yl}ethoxy)-2-methylquinoline (E119)

The **title compound** was prepared from 5-(2-{4-[4-fluoro-3-((*R*)-3-methylpiperazin-1-yl)benzylidene]piperidin-1-yl}ethoxy)-2-methylquinoline using the method of Example 60.
Mass spectrum (API⁺): Found 477 (MH⁺). C₂₉H₃₇FN₄O requires 476.
¹H NMR (400 MHz, CDCl₃) δ: 1.1 (3H, d), 1.25-1.33 (4H, m), 1.49 (1H, m), 1.63 (2H, br d), 2.13 (2H, t), 2.35 (1H, t), 2.47 (2H, d), 2.69 (1H,m), 2.71 (3H, s), 2.92 (2H, t), 3.01-3.11 (4H, m), 3.31 (2H, br d), 4.27 (2H, t), 6.67 (2H, d), 6.78 (1 H, d), 6.90 (1H, m), 7.23 (1H, d), 7.52-7.60 (2H, m), 8.42 (1 H, d).

### Example 120

### N-(3-{4-Cyano-1-[2-(2-methylquinolin-5-yloxy)ethyl]piperidin-4-ylmethyl}phenyl)methanesulfonamide (E120)

The **title compound** was prepare from *tert*-butyl 4-cyano-4-(3-nitrobenzyl)piperidine-1-carboxylate using analogous routes and intermediates to those used to prepare Example 3.
Mass spectrum (API⁺): Found 479 (MH⁺). C₂₆H₃₀N₄O₃S requires 478.
¹H NMR (400 MHz, CDCl₃) *δ* 1.69 (2H, t *J* 13.2), 1.88 (2H, d *J* 11.6), 2.51 (2H, t *J* 12.4), 2.73 (3H, s), 2.85 (2H, s), 2.98 (2H, t *J* 5.6), 3.04 (3H, s), 3.07 (2H, t *J* 5.6), 4.26 (2H, t *J* 5.6), 6.50 (1H, s br), 6.79 (1H, d *J* 7.2), 7.08 (1H, d *J* 7.6), 7.14 (1H, d *J* 7.2)*,* 7.26 (1H, d *J* 6), 7.32 (1H, t *J* 7.6)*,* 7.58 (2H, m), 8.41 (1H, d *J* 8.8).

### Example 121

### 5-(2-{4-[3-(4-Methylpiperazin-1-yl)benzylidene] piperidin-1-yl}ethoxy)-2-cyanoquinoline (E121)

The **title compound** was prepared from 3-{1-[2-(2-cyanoquinolin-5-yloxy)ethyl]piperidin-4-ylidenemethyl}bromobenzene and 4-methylpiperazine using a similar procedure to Example 58.
Mass spectrum (API+): Found 468 (MH⁺). C₂₉H₃₃N₅O requires 467.
¹H NMR (CDCl₃) δ: 2.35 (3H, s), 2.38-2.44 (2H, m), 2.52-2.64 (8H, m), 2.70-2.77 (2H, m), 2.95-3.02 (2H, m), 3.17-3.22 (4H, m), 4.33 (2H, t), 6.28 (1 H, s), 6.71 (1 H, d), 6.75-6.80 (2H, m), 6.99 (1 H, dd), 7.20 (1 H, t), 7.67 (1 H, d), 7.70-7.78 (2H, m), 8.70 (1H, d).

### Example 122

### N-(3-{1-[2-(2-Methoxycarbonylquinolin-5-yloxy)ethyl]piperidin-4-ylmethyl}phenyl)methanesulfonamide (E122)

A solution of 5-(2-bromoethoxy)quinoline-2-carbonitrile (50 mg, 0.18 mmol), *N-*[3-(piperidin-4-ylmethyl)phenyl]methanesulphonamide hydrochloride (95 mg, 0.25 mmol) and diisopropylethylamine (100 mg, 0.75 mmol) in 2-propanol (10 ml) was heated under reflux for 48 h, then concentrated under vacuum. The residue was dissolved in a mixture of methanol (10 ml) and THF (5 ml), treated with 1 M sodium hydroxide (5 ml) and stirred at room temperature for 1 h, then concentrated under vacuum. The residue was treated with 10% sodium carbonate solution, extracted with ethyl acetate and the extract separated, dried (Na₂SO₄), concentrated under vacuum and then chromatographed on silca gel eluting with 0-5% methanol/DCM to afford the **title compound** as a yellow oil (30 mg, 36%).
Mass spectrum (API+): Found 498 (MH⁺). C₂₆H₃₁N₃O₅S requires 497.
¹H NMR (CDCl₃) δ: 1.27-1.40 (2H, m), 1.50-1.60 (1H, m), 1.66 (2H, br d), 2.14 (2H, br t), 2.54 (2H, d), 2.94 (2H, t), 3.00 (3H, s), 3.03 (2H, br d), 4.08 (3H, s), 4.30 (2H, t), 6.50 (1H, br, NH), 6.90-7.05 (4H, m), 7.25 (1H, t), 7.66 (1H, t), 7.87 (1H, d), 8.15 (1H, d), 8.69 (1H, d).

## Claims

1. A compound of formula (I) or a pharmaceutically acceptable salt thereof: 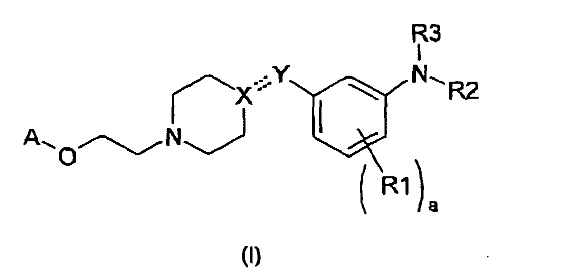 wherein
A is optionally substituted phenyl, naphthyl, indolyl, quinolinyl, quinazolinyl, indazolyl, isoquinolinyl or benzofuranyl;
X is carbon, Y is CH and 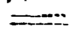 is a double bond; or X is CH, Y is CH₂ or oxygen and 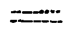 is a single bond; or X is nitrogen, Y is CH₂ and 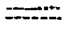 is a single bond;
R1 is halogen, hydroxy, cyano, C₁₋₆alkyl, haloC₁₋₆alkyl or C₁₋₆alkoxy;
a is 0, 1, 2, 3 or 4;
R2 and R3, together with the nitrogen atom to which they are attached, form a nitro group or an optionally substituted 3 to 7 membered heterocyclic group, or R2 and R3 are independently hydrogen, aroyl, C₁₋₆alkyl, C₁₋₆alkanoyl, fluoroC₁₋₆alkanoyl, C₁₋₆alkylsulfonyl, fluoroC₁₋₆alkylsulfonyl, carbamoyl, C₁₋₆alkylcarbamoyl, arylC₁₋₆alkyl or a group CO(CH₂)bNR4R5 wherein b is 1, 2, 3 or 4 and R4 and R5 are independently hydrogen or C₁₋₆alkyl, or R4 and R5, together with the nitrogen atom to they are attached, form part of an optionally substituted 3 to 7 membered heterocyclic group.

2. A compound as claimed in claim 1, wherein A is quinolinyl or quinazolinyl.

3. A compound as claimed in claim 2, wherein A is 5-(2-methyl)quinolinyl or 5-(2-methyl)quinazolinyl.

4. A compound as claimed in any of claims 1-3, wherein a is 0, 1 or 2.

5. A compound as claimed in any of claims 1-4, wherein one of R2 and R3 is hydrogen or C₁₋₆alkyl (particularly methyl, ethyl or propyl) and the other is C₁₋₆alkanoyl, C₁₋₆alkylsulfonyl, haloC₁₋₆alkanoyl or C₁₋₆alkylcarbamoyl, or R2 and R3 together with the nitrogen atom to which they are attached, form an optionally substituted piperidinyl or piperazinyl group or a nitro group.

6. A compound as claimed in claim 1, selected from the list consisting of:
*N-*(3-(1*-*(2-(2-Methylquinolin-5-yloxy)ethyl)piperidin-4-ylmethyl)phenyl)methanesulfonamide (E3);
*N-*(3-(1-(2-(2-Methylquinolin-5-yloxy)ethyl)piperidin-4-ylidenemethyl)phenyl)acetamide (E13);
*N*-(3-(1-(2-(2-Methylquinolin-5-yloxy)ethyl)piperidin-4-ylmethyl)phenyl)propane-2-sulfonamide (E25);
N-(3-(1-(2-(2-Methylquinolin-5-yloxy)ethyl)piperidin-4-ylmethyl)phenyl)propane-1-sulfonamide (E28);
*N*-(2-Fluoro-5-(1-(2-(2-methylquinolin-5-yloxy)ethyl)piperidin-4-ylmethyl)phenyl)methanesulfonamide (E31);
5-(2-(4-(3-(*cis*-3,5-Dimethylpiperazin-1-yl)benzylidene)piperidin-1-y)ethoxy)-2-methylquinoline (E58);
2-Methyl-5-(2-(4-(3-(4-methylpiperazin-1-yl)benzylidene)piperidin-1-yl)ethoxy)quinoline (E59);
5-(2-(4-(3-(*cis*-3,5-Dimethylpiperazin-1-yl)benzyl)piperidin-1-yl)ethoxy)-2-methylquinoline (E60);
2-Methyl-5-(2-(4-(3-(4-methylpiperazin-1-yl)benzyl)piperidin-1-yl)ethoxy)quinoline (E61);
2-Methyl-5-(2-(4-(3-((*R*)-3-methylpiperazin-1-yl)benzylidene)piperidin-1-yl)ethoxy)quinoline (E62);
2-Methyl-5-(2-(4-(3-((S)-3-methylpiperazin-1-yl)benzylidene)piperidin-1-yl)ethoxy)quinoline (E65);
2-Methyl-5-(2-(4-(3-((R)-3-methylpiperazin-1-yl)benzyl)piperidin-1-yl)ethoxy)quinoline (E66);
2-Methyl-5-{2-[4-(3-piperidin-1-ylbenzyl)piperidin-1-yl]ethoxy}quinoline (E70);
2-Methyl-5-(2-{4-[3-(4-methyl[1,4]diazepan-1-yl)benzyl]piperidin-1-yl}ethoxy)quinoline (E72);
2-Methyl-5-{2-[4-(3-piperidin-1-ylbenzylidene)piperidin-1-yl]ethoxy}quinoline (E74);
2-Methyl-5-{2-[4-(3-pyrazol-1-ylbenzylidene)piperidin-1-yl]ethoxy}quinoline (E108);
5-(2-(4-[4-Fluoro-3-(4-methylpiperazin-1-yl)benzylidene]piperidin-1-yl)ethoxy)-2-methylquinoline (E114);
5-(2-{4-[4-Fluoro-3-((*R*)-3-methylpiperazin-1-yl)benzyl]piperidin-1-yl}ethoxy)-2-methylquinoline (E119); and
5-(2-{4-[3-(4-Methylpiperazin-1-yl)benzylidene]piperidin-1-yl}ethoxy)-2-cyanoquinoline (E121).

7. A process for the preparation of a compound of formula (I) as defined in any of claims 1-6 or a pharmaceutically acceptable salt thereof, which process comprises:
(a) the coupling of a compound of formula (II): 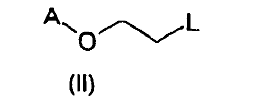 wherein A is as defined for formula (I) and L is a leaving group, and a compound of formula (III): 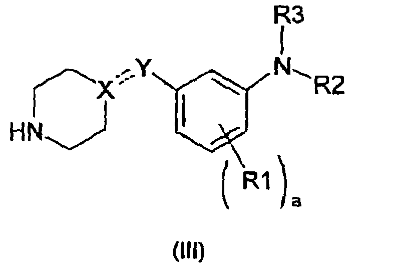 wherein a, R1, R2, R3, X, Y and 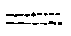 are as defined for formula (I); or
(b) for a compound wherein X is nitrogen, the coupling of a compound of formula (IV): 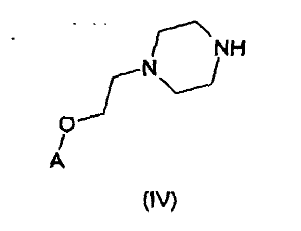 wherein A is as defined for formula (I), and a compound of formula (V): 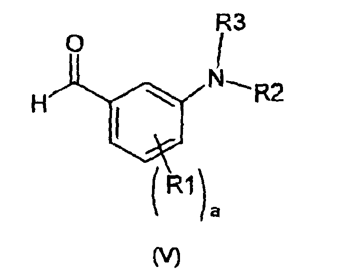 wherein a, R1, R2 and R3 are as defined for formula (1), or
(c) a Buchwald reaction between a compound of formula (VI): 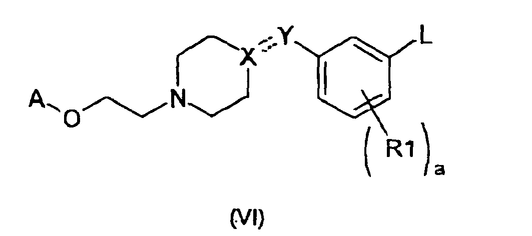 wherein L is a suitable leaving group and a, R1, X, Y and 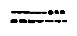 are as defined for formula (I), and a compound of formula (VII): 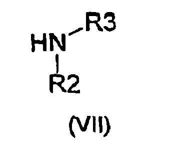 wherein R2 and R3 are as defined for formula (I);
and thereafter optionally for process (a), (b) or (c):
• removing any protecting groups and/or
• converting a compound of formula (I) into another compound of formula (I) and/or
• forming a pharmaceutically acceptable salt.

8. A pharmaceutical composition comprising a compound as defined in any of claims 1-6, and a pharmaceutically acceptable diluent, carrier and/or excipient.

9. A process for preparing a composition as defined in claim 8, the process comprising mixing a compound as defined in any of claims 1-6 with a pharmaceutically acceptable diluent, carrier and/or excipient.

10. A compound or a composition as defined in any of claims 1-6 or 8 for use in therapy.

11. A compound or a composition as defined in any of claims 1-6 or 8 for use in the treatment of a CNS disorder.

12. A compound or a composition as defined in any of claims 1-6 or 8 for use in the treatment of depression or anxiety.

13. Use of a compound or a composition as defined in any of claims 1-6 or 8 in the manufacture of a medicament for use in the treatment of a CNS disorder.

14. The use as claimed in claim 13, wherein the disorder is depression or anxiety.

## Patentansprüche

1. Verbindung der Formel (I) oder ein pharmazeutisch verträgliches Salz davon: 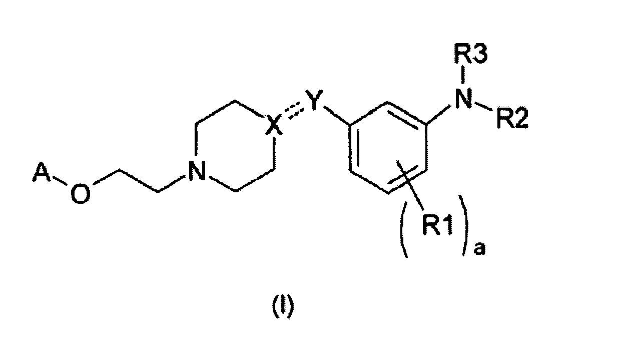 wobei
A gegebenenfalls substituiertes Phenyl, Naphthyl, Indolyl, Chinolinyl, Chinazolinyl, Indazolyl, Isochinolinyl oder Benzofuranyl ist;
X Kohlenstoff ist, Y CH ist und :::::::: eine Doppelbindung ist; oder X CH ist, Y CH₂ oder Sauerstoff ist und :::::::: eine Einfachbindung ist; oder X Stickstoff ist, Y CH₂ ist und :::::::: eine Einfachbindung ist;
R1 Halogen, Hydroxy, Cyano, C₁₋₆-Alkyl, Halogen-C₁₋₆-alkyl oder C₁₋₆-Alkoxy ist;
a 0, 1, 2, 3 oder 4 ist;
R2 und R3, zusammen mit dem Stickstoffatom, an das sie gebunden sind, eine Nitrogruppe oder einen gegebenenfalls substituierten 3- bis 7-gliedrigen heterocyclischen Rest bilden, oder R2 und R3 unabhängig voneinander Wasserstoff, Aroyl, C₁₋₆-Alkyl, C₁₋₆-Alkanoyl, Fluor-C₁₋₆-alkanoyl, C₁₋₆-Alkylsulfonyl, Fluor-C₁₋₆-alkylsulfonyl, Carbamoyl, C₁₋₆-Alkylcarbamoyl, Aryl-C₁₋₆-alkyl oder ein Rest CO(CH₂)_{b}NR4R5 sind, wobei b 1, 2, 3 oder 4 ist und R4 und R5 unabhängig voneinander Wasserstoff oder C₁₋₆-Alkyl sind, oder R4 und R5, zusammen mit dem Stickstoffatom, an das sie gebunden sind, einen Teil eines gegebenenfalls substituierten 3- bis 7-gliedrigen heterocyclischen Restes bilden.

2. Verbindung gemäß Anspruch 1, wobei A Chinolinyl oder Chinazolinyl ist.

3. Verbindung gemäß Anspruch 2, wobei A 5-(2-Methyl)chinolinyl oder 5-(2-Methyl)chinazolinyl ist.

4. Verbindung gemäß einem der Ansprüche 1 bis 3, wobei a 0, 1 oder 2 ist.

5. Verbindung gemäß einem der Ansprüche 1 bis 4, wobei eines von R2 und R3 Wasserstoff oder C₁₋₆-Alkyl (insbesondere Methyl, Ethyl oder Propyl) ist und das andere C₁₋₆-Alkanoyl, C₁₋₆-Alkylsulfonyl, Halogen-C₁₋₆-alkanoyl oder C₁₋₆-Alkylcarbamoyl ist, oder R2 und R3, zusammen mit dem Stickstoffatom, an das sie gebunden sind, einen gegebenenfalls substituierten Piperidinyl- oder Piperazinylrest oder eine Nitrogruppe bilden.

6. Verbindung gemäß Anspruch 1, ausgewählt aus:
N-(3-(1-(2-(2-Methylchinolin-5-yloxy)ethyl)piperidin-4-ylmethyl)phenyl)methansulfonamid (E3);
N-(3-(1-(2-(2-Methylchinolin-5-yloxy)ethyl)piperidin-4-ylidenmethyl)phenyl)acetamid (E13);
N-(3-(1-(2-(2-Methylchinolin-5-yloxy)ethyl)piperidin-4-ylmethyl)phenyl)propan-2-sulfonamid (E25);
N-(3-(1-(2-(2-Methylchinolin-5-yloxy)ethyl)piperidin-4-ylmethyl)phenyl)propan-1-sulfonamid (E28);
N-(2-Fluor-5-(1-(2-(2-methylchinolin-5-yloxy)ethyl)piperidin-4-ylmethyl)phenyl)-methansulfonamid (E31);
5-(2-(4-(3-(cis-3,5-Dimethylpiperazin-1-yl)benzyliden)piperidin-1-yl)ethoxy)-2-methylchinolin (E58);
2-Methyl-5-(2-(4-(3-(4-methylpiperazin-1-yl)benzyliden)piperidin-1-yl)ethoxy)chinolin (E59);
5-(2-(4-(3-(cis-3,5-Dimethylpiperazin-1-yl)benzyl)piperidin-1-yl)ethoxy)-2-methylchinolin (E60);
2-Methyl-5-(2-(4-(3-(4-methylpiperazin-1-yl)benzyl)piperidin-1-yl)ethoxy)chinolin (E61);
2-Methyl-5-(2-(4-(3-((R)-3-methylpiperazin-1-yl)benzyliden)piperidin-1-yl)ethoxy)-chinolin (E62);
2-Methyl-5-(2-(4-(3-((S)-3-methylpiperazin-1-yl)benzyliden)piperidin-1-yl)ethoxy)-chinolin (E65);
2-Methyl-5-(2-(4-(3-((R)-3-methylpiperazin-1-yl)benzyl)piperidin-1-yl)ethoxy)chinolin (E66);
2-Methyl-5-{2-[4-(3-piperidin-1-ylbenzyl)piperidin-1-yl]ethoxy}chinolin (E70);
2-Methyl-5-(2-{4-[3-(4-methyl-[1,4]-diazepan-1-yl)benzyl]piperidin-1-yl}ethoxy)-chinolin (E72);
2-Methyl-5-{2-[4-(3-piperidin-1-ylbenzyliden)piperidin-1-yl]ethoxy}chinolin (E74);
2-Methyl-5-{2-[4-(3-pyrazol-1-ylbenzyliden)piperidin-1-yl]ethoxy}chinolin (E108);
5-(2-{4-[4-Fluor-3-(4-methylpiperazin-1-yl)benzyliden]piperidin-1-yl}ethoxy)-2-methylchinolin (E114);
5-(2-{4-[4-Fluor-3-((R)-3-methylpiperazin-1-yl)benzyl]piperidin-1-yl}ethoxy)-2-methylchinolin (E119); und
5-(2-{4-[3-(4-Methylpiperazin-1-yl)benzyliden]piperidin-1-yl}ethoxy)-2-cyanochinolin (E121).

7. Verfahren zur Herstellung einer Verbindung der Formel (I) wie in einem der Ansprüche 1 bis 6 definiert, oder eines pharmazeutisch verträglichen Salzes davon, wobei das Verfahren umfasst:
(a) das Kuppeln einer Verbindung der Formel (II): 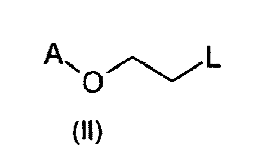 wobei A wie für die Formel (I) definiert ist und L eine Abgangsgruppe ist, und einer Verbindung der Formel (III): 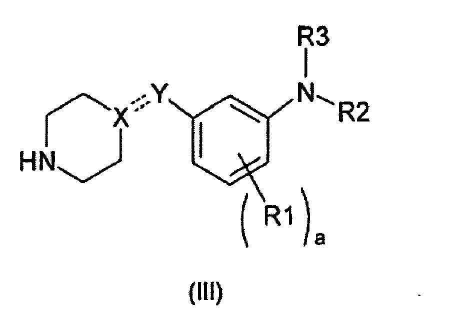 wobei a, R1, R2, R3, X, Y und :::::::: wie für die Formel (I) definiert sind; oder
(b) für eine Verbindung, wobei X Stickstoff ist, das Kuppeln einer Verbindung der Formel (IV): 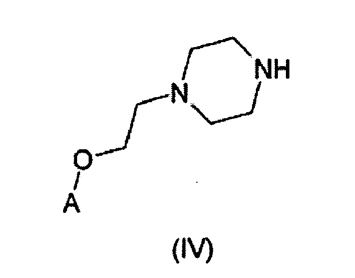 wobei A wie für die Formel (I) definiert ist, und einer Verbindung der Formel (V): 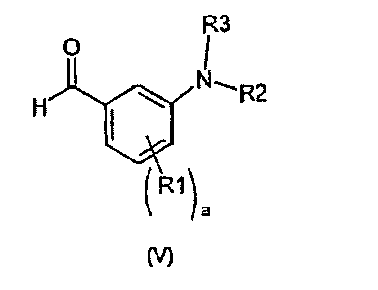 wobei a, R1, R2 und R3 wie für die Formel (I) definiert sind, oder
(c) eine Buchwald-Reaktion zwischen einer Verbindung der Formel (VI): 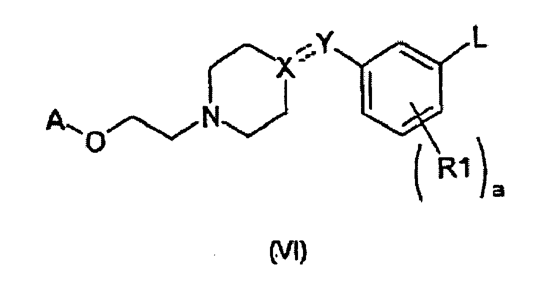 wobei L eine geeignete Abgangsgruppe ist und a, R1, X, Y und :::::::: wie für die Formel (I) definiert sind, und einer Verbindung der Formel (VII): 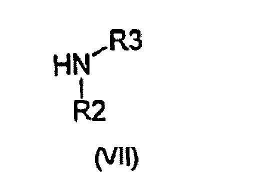 wobei R2 und R3 wie für die Formel (I) definiert sind;
und anschließend gegebenenfalls für das Verfahren (a), (b) oder (c):
• Entfernen jeglicher Schutzgruppen und/oder
• Umwandeln einer Verbindung der Formel (I) in eine andere Verbindung der Formel (I) und/oder
• Bilden eines pharmazeutisch verträglichen Salzes.

8. Arzneimittel, umfassend eine Verbindung wie in einem der Ansprüche 1 bis 6 definiert, und ein pharmazeutisch verträgliches Verdünnungsmittel, einen pharmazeutisch verträglichen Träger und/oder einen pharmazeutisch verträglichen Exzipienten.

9. Verfahren zur Herstellung einer Zusammensetzung wie in Anspruch 8 definiert, wobei das Verfahren das Mischen einer Verbindung wie in einem der Ansprüche 1 bis 6 definiert mit einem pharmazeutisch verträglichen Verdünnungsmittel, Träger und/oder Exzipienten umfasst.

10. Verbindung oder Zusammensetzung wie in einem der Ansprüche 1 bis 6 oder 8 definiert zur Verwendung in der Therapie.

11. Verbindung oder Zusammensetzung wie in einem der Ansprüche 1 bis 6 oder 8 definiert zur Verwendung bei der Behandlung einer Erkrankung des ZNS.

12. Verbindung oder Zusammensetzung wie in einem der Ansprüche 1 bis 6 oder 8 definiert zur Verwendung bei der Behandlung von Depression oder Angst.

13. Verwendung einer Verbindung oder Zusammensetzung wie in einem der Ansprüche 1 bis 6 oder 8 definiert zur Herstellung eines Medikaments zur Verwendung bei der Behandlung einer Erkrankung des ZNS.

14. Verwendung gemäß Anspruch 13, wobei die Erkrankung Depression oder Angst ist.

## Revendications

1. Composé de formule (I) ou un de ses sels pharmaceutiquement acceptables : 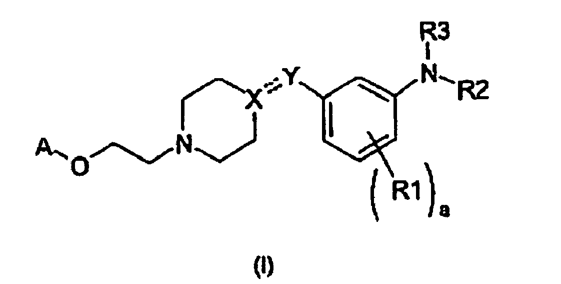 formule dans laquelle
A représente un groupe phényle, naphtyle, indolyle, quinolinyle, quinazolinyle, indazolyle, isoquinolinyle ou benzofurannyle, facultativement substitué ;
X représente un atome de carbone, Y représente un groupe CH et 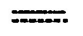 représente une double liaison ; ou X représente un groupe CH, Y représente un groupe CH₂ ou un atome d'oxygène et 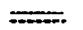 représente une liaison simple ; ou bien X représente un atome d'azote, Y représente un groupe CH₂ et 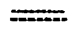 représente une liaison simple ;
R1 représente un groupe halogéno, hydroxy, cyano, alkyle en C₁ à C₆, halogénalkyle en C₁ à C₆ ou alkoxy en C₁ à C₆ ;
a est égal à 0, 1, 2, 3 ou 4 ;
R2 et R3, conjointement avec l'atome d'azote auquel ils sont fixés, forment un groupe nitro ou un groupe hétérocyclique tri- à heptagonal facultativement substitué, ou bien R2 et R3 représentent indépendamment un atome d'hydrogène, un groupe aroyle, alkyle en C₁ à C₆, alcanoyle en C₁ à C₆, fluoralcanoyle en C₁ à C₆, alkylsulfonyle en C₁ à C₆, fluoralkylsulfonyle en C₁ à C₆, carbamoyle, (alkyle en C₁ à C₆)carbamoyle, aryl(alkyle en C₁ à C₆) ou un groupe CO(CH₂)bNR4R5 dans lequel b est égal à 1, 2, 3 ou 4 et R4 et R5 représentent indépendamment un atome d'hydrogène ou un groupe alkyle en C₁ à C₆, ou bien R4 et R5, conjointement avec l'atome d'azote auquel ils sont fixés, font partie d'un groupe hétérocyclique tri- à heptagonal facultativement substitué.

2. Composé suivant la revendication 1, dans lequel A représente un groupe quinolinyle ou quinazolinyle.

3. Composé suivant la revendication 2, dans lequel A représente un groupe 5-(2-méthyl)quinolinyle ou 5-(2-méthyl)-quinazolinyle.

4. Composé suivant l'une quelconque des revendications 1 à 3, dans lequel a est égal à 0, 1 ou 2.

5. Composé suivant l'une quelconque des revendications 1 à 4, dans lequel un des groupes R2 et R3 représente un atome d'hydrogène ou un groupe alkyle en C₁ à C₆ (en particulier méthyle, éthyle ou propyle) et l'autre représente un groupe alcanoyle en C₁ à C₆, alkylsulfonyle en C₁ à C₆, halogénalcanoyle en C₁ à C₆ ou (alkyle en C₁ à C₆)carbamoyle, ou bien R2 et R3, conjointement avec l'atome d'azote auquel ils sont fixés, forment un groupe pipéridinyle ou pipérazinyle facultativement substitué ou un groupe nitro.

6. Composé suivant la revendication 1, choisi dans la liste consistant en :
*N*-(3-(1-(2-(2-méthylquinoléine-5-yloxy)éthyl)pipéridine-4-ylméthyl)phényl)méthanesulfonamide (E3) ;
*N*-(3-(1-(2-(2-méthylquinoléine-5-yloxy)éthyl)pipéridine-4-ylidèneméthyl)phényl)acétamide (E13) ;
*N*-(3-(1-(2-(2-méthylquinoléine-5-yloxy)éthyl)pipéridine-4-ylméthyl)phényl)propane-2-sulfonamide (E25) ;
*N*-(3-(1-(2-(2-méthylquinoléine-5-yloxy)éthyl)pipéridine-4-ylméthyl)phényl)propane-1-sulfonamide (E28) ;
*N*-(2-fluoro-5-(1-(2-(2-méthylquinoléine-5-yloxy)éthyl)-pipéridine-4-ylméthyl)phényl)méthanesulfonamide (E31) ;
5-(2-(4-(3-*cis*-3,5-diméthylpipérazine-1-yl)benzylidène)-pipéridine-1-yl)éthoxy)-2-méthylquinoléine (E58) ;
2-méthyl-5-(2-(4-(3-(4-méthylpipérazine-1-yl)benzylidène)-pipéridine-1-yl)éthoxy)quinoléine (E59) ;
5-(2-(4- (3-*cis*-3, 5-diméthylpipérazine-1-yl) benzyl)-pipéridine-1-yl)éthoxy)-2-méthylquinoléine (E60) ;
2-méthyl-5-(2-(4-(3-(4-méthylpipérazine-1-yl)benzyl)-pipéridine-1-yl)éthoxy)quinoléine (E61) ;
2-méthyl-5-(2-(4-(3-((*R*)-3-méthylpipérazine-1-yl)benzylidène)-pipéridine-1-yl)éthoxy)quinoléine (E62) ;
2-méthyl-5-(2-(4-(3-((*S*)-3-méthylpipérazine-1-yl)benzylidène)-pipéridine-1-yl)éthoxy)quinoléine (E65) ;
2-méthyl-5-(2-(4-(3-((*R*)-3-méthylpipérazine-1-yl) benzyl)-pipéridine-1-yl)éthoxy)quinoléine (E66) ;
2-méthyl-5-{2-[4-(3-pipéridine-1-ylbenzyl) pipéridine-1-yl]-éthoxy}quinoléine (E70) ;
2-méthyl-5-(2-{4-[3-(4-méthyl[1,4]diazépane-1-yl)-benzyl]pipéridine-1-yl}éthoxy)quinoléine (E72) ;
2-méthyl-5-{2- [4- (3-pipéridine-1-ylbenzylidène)pipéridine-1-yl]éthoxy}quinoléine (E74) ;
2-méthyl-5-{2-[4-(3-pyrazole-1-ylbenzylidène)pipéridine-1-yl]éthoxy}quinoléine (E108) ;
5-(2-{4-[4-fluoro-3-(4-méthylpipérazine-1-yl)benzylidène]-pipéridine-1-yl}éthoxy)-2-méthylquinoléine (E114) ;
5- (2-{4-[4-fluoro-3-((R)-3-méthylpipérazine-1-yl) benzyl]-pipéridine-1-yl}éthoxy)-2-méthylquinoléine (E119) ; et
5-(2-{4-[3-(4-méthylpipérazine-1-yl)benzylidène]pipéridine-1-yl}éthoxy)-2-cyanoquinoléine (E121).

7. Procédé pour la préparation d'un composé de formule (I) tel que défini dans l'une quelconque des revendications 1 à 6, ou d'un de ses sels pharmaceutiquement acceptables, procédé qui comprend :
(a) le couplage d'un composé de formule (II) : 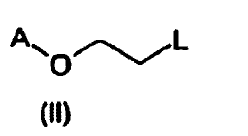 dans laquelle A est tel que défini pour la formule (I) et L représente un groupe partant, et d'un composé de formule (III) : 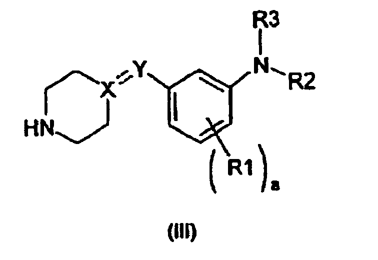 dans laquelle a, R1, R2, R3, X, Y et 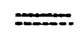 sont tels que définis pour la formule (I) ;
ou
(b) pour un composé dans lequel X représente un atome d'azote, le couplage d'un composé de formule (IV) : 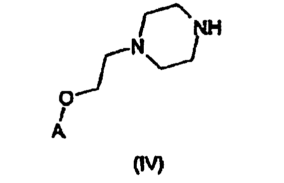 dans laquelle A est tel que défini pour la formule (I), et d'un composé de formule (V) : 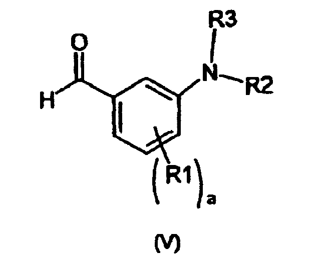 dans laquelle a, R1, R2 et R3 sont tels que définis pour la formule (I),
ou
(c) une réaction de Buchwald entre un composé de formule (VI) : 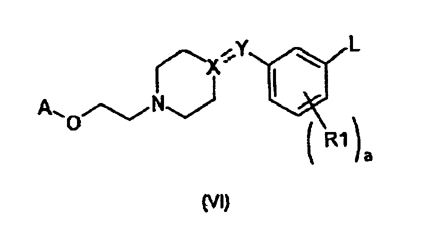 dans laquelle L représente un groupe partant convenable, et a, R1, X, Y et 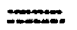 sont tels que définis pour la formule (I), et un composé de formule (VII) : 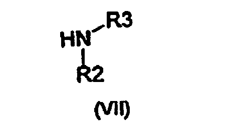 dans laquelle R2 et R3 sont tels que définis pour la formule (I) ;
et ensuite, facultativement, pour le procédé (a), (b) ou (c) :
• l'élimination de n'importe quels groupes protecteurs et/ou
• la conversion d'un composé de formule (I) en un autre composé de formule (I) et/ou
• la formation d'un sel pharmaceutiquement acceptable.

8. Composition pharmaceutique comprenant un composé tel que défini dans l'une quelconque des revendications 1 à 6, et un diluant, support et/ou excipient pharmaceutiquement acceptable.

9. Procédé pour la préparation d'une composition telle que définie dans la revendication 8, procédé comprenant le mélange d'un composé tel que défini dans l'une quelconque des revendications 1 à 6 avec un diluant, support et/ou excipient pharmaceutiquement acceptable.

10. Composé ou composition tel que défini dans l'une quelconque des revendications 1 à 6 ou 8, destiné à être utilisé en thérapie.

11. Composé ou composition tel que défini dans l'une quelconque des revendications 1 à 6 ou 8, destiné à être utilisé dans le traitement d'un trouble du SNC.

12. Composé ou composition tel que défini dans l'une quelconque des revendications 1 à 6 ou 8, destiné à être utilisé dans le traitement de la dépression ou de l'anxiété.

13. Composé ou composition tel que défini dans l'une quelconque des revendications 1 à 6 ou 8, dans la production d'un médicament destiné à être utilisé dans le traitement d'un trouble du SNC.

14. Utilisation suivant la revendication 13, dans laquelle le trouble est la dépression ou l'anxiété.
